# EUROPEAN PATENT APPLICATION

(11) **EP 2 218 442 A1**
(43) Date of publication of application: **18.08.2010**
(21) Application number: 10001901.7
(22) Date of filing: 08.11.2006
(51) Int. Cl.: A61K 9/00, A61P 27/02

(54) **Methods, compositions, and kits for the treatment of ophthalmic disorders**

(30) Priority: 09.11.2005 US 735989 P
(62) Divisional of application: 06827633.6
(71) Applicant: CombinatoRx, Inc., Cambridge MA 02142 (US)
(72) Inventor: Chappell, Todd, W., Boston, MA 02127 (US); Auspitz, Benjamin, A., Cambridge, MA 02139 (US); Jost-Price, Edward, Roydon, West Roxbury, MA 02132 (US); Nichols, M., James, Boston, MA 02116 (US); Grau, Daniel, S., Arlington, MA 02476 (US)
(74) Representative: Bösl, Raphael Konrad

(57) **Abstract**

The invention features methods, compositions and kits comprising a corticosteroid and a non-steroidal immunophilin-dependent immunosuppressant for treating an ophthalmic disorder in a patient, wherein at least one of said corticosteroid and said non-steroidal immunophilin-dependent immunosuppressant is present at a low concentration, in particular wherein said corticosteroid is prednisolone acetate and said non-steroidal immunophilin-dependent immunosuppressant is cyclosporine A.

## Description

### Background of the Invention

The invention relates to the treatment of immunoinflammatory disorders and ophthalmic disorders.

Immunoinflammatory disorders are characterized by the inappropriate activation of the body's immune defenses. Rather than targeting infectious invaders, the immune response targets and damages the body's own tissues or transplanted tissues. The tissue targeted by the immune system varies with the disorder. For example, in multiple sclerosis, the immune response is directed against the neuronal tissue, while in Crohn's disease the digestive tract is targeted. Immunoinflammatory disorders affect millions of individuals and include conditions such as asthma, allergic intraocular inflammatory diseases, arthritis, atopic dermatitis, atopic eczema, diabetes, hemolytic anaemia, inflammatory dermatoses, inflammatory bowel or gastrointestinal disorders (e.g., Crohn's disease and ulcerative colitis), multiple sclerosis, myasthenia gravis, pruritis/inflammation, psoriasis, rheumatoid arthritis, cirrhosis, and systemic lupus erythematosus.

Current treatment regimens for immunoinflammatory disorders typically rely on immunosuppressive agents. The effectiveness of these agents can vary and their use is often accompanied by adverse side effects. Thus, improved therapeutic agents and methods for the treatment of immunoinflammatory disorders are needed.

Musculoskeletal disorders such as arthritis are among the most frequent causes of physical disability among older adults. The three most common types of arthritis are osteoarthritis (OA), rheumatoid arthritis (R.A), and gout. Osteoarthritis is the most common joint disease, with radiological evidence of its existence found in 50% of the population.

OA affects the hands, lower back, neck, and weight-bearing joints such as the knees, hips, and foot joints. The yearly incidence of OA of the hand is about 50 new cases per 1,000 for persons under age 40, rising to 65 per 1,000 for ages 40-59 and 110 per 1,000 for ages 60 and greater.

OA has been characterized as a slowly evolving degenerative disease with a multifactorial etiology that may differ depending on the joint site. OA occurs when cartilage, the tissue that cushions the ends of the bones within the joints, begins to break down and wear away. In some cases, all of the cartilage may wear away, leaving bones that rub against each other. Arthroscopic studies of early disease have shown synovitis in approximately half of those joints with cartilage damage, suggesting a localized inflammatory reaction in patients with early OA. Furthermore, numerous studies have identified an association between C-reactive protein (CRP) and OA. CRP is an acute phase response protein whose production is stimulated by cytokines, particularly interleukin-6 (IL-6). The relationship between inflammatory processes and elevation in plasma CRP and pro-inflammatory cytokines is well known. CRP has also been related to the inflammatory activity of rheumatoid arthritis.

Symptoms of OA range from stiffness and intermittent mild pain to severe joint pain and impaired biomechanical function. Although there is no cure for most forms of OA, various therapies can help patients manage symptoms and improve their overall quality of life. Symptomatic treatment of OA traditionally involves administration of non-steroidal anti-inflammatory drugs (NSAIDs), local analgesic therapies, intra-articular corticosteroid injection, and surgery.

Treatment of OA with NSAIDs such as indomethacin, ketoprofen, ibuprofen, acetylsalicylic acid (ASA), and flurbiprofen can relieve pain by reducing local inflammation and attenuating levels of proinflammatory agents. However, long-term NSAID use is compromised by significant gastrointestinal (GI) toxicity. A large multi-center, prospective, observational study involving 1,921 patients with rheumatoid arthritis taking NSAms reported that 81% of patients who were hospitalized with serious GI complications had no prior GI problems. This makes it difficult for clinicians to identify patients at risk for GI side-effects. In the United States, it has been conservatively estimated that there are 107,000 annual hospitalizations for NSAID-related GI complications and 16,500 annual NSAID-related deaths among patients with RA or OA. This mortality figure is almost as high as the number of deaths due to asthma, cervical cancer and malignant melanoma combined.

Steroids are known powerful anti-inflammatory agents that have been used in treating OA. However, chronic administration of anti-inflammatory doses of steroids is also limited by well-known toxicities. For example, prolonged use of steroids has been associated with osteoporosis, high blood pressure, neurological complications, suboptimal immune response, and ocular disturbances, limiting their utility in therapeutic situations. A therapeutic agent that, for example, retained the potent anti-inflammatory effects of steroids, or the therapeutic effects of another class of drugs, while limiting the associated toxicities, would be of great benefit to patients with OA or other musculoskeletal disorders.

Periodontal disease refers to diseases of tissues that surround and support teeth, including the gingiva, cementum, periodontal ligament, alveolar process bone, and dental supporting bone. The most common forms of periodontal disease are periodontitis and gingivitis.

Periodontal disease involves the inflammation, destruction and degeneration of periodontal tissues that surround and support mammalian teeth. These periodontal tissues include the crevicular epithelium, junctional epithelium, external marginal epithelium, gingiva, alveolar bone, periodontal ligament, and cementum. The loss of supporting bone in periodontitis is the latest stage of this progressive disorder and is the major cause of tooth loss in adults.

Periodontal disease is typically classified as gingivitis and periodontitis according to the progress of disease. "Gingivitis" refers to a condition in which inflammation is localized within the gingiva and no lesion occurs in the bone and periodontal ligament, and a pocket is relative pocket. "Periodontitis" refers to a condition in which the inflammation of gingiva reaches the periodontal ligament and alveolar bone, the pocket becomes a periodontal pocket, and the attachment level (the position of attachment) is on the root apex side downward from the cementum-enamel junction. The inflammation prolongs and proceeds toward deep parts with a deepening periodontal pocket.

The relationship between inflammatory processes and elevation in plasma C-reactive protein (CRP) and proinflammatory cytokines is well known, and this relationship is observed in periodontitis. In atherosclerotic heart disease where elevated plasma CRP levels are a known risk factor, a correlation with the incidence of periodontitis has also been reported. As such, agents that can modify levels of inflammation in periodontitis would be expected to be active across such inflammatory diseases. Indeed, treatment of periodontitis with NSAIDs such as indomethacin, ketoprofen, ibuprofen, ASA and flurbiprofen, can reduce local inflammation and attenuate levels of pro-inflammatory agents. However, there have been no reports on the use of corticosteroids to treat periodontitis, perhaps because chronic administration of antiinflammatory doses of steroids is limited by well known toxicities. An agent that could provide the anti-inflammatory effect of steroids without the associated toxicity is desirable for the treatment of periodontal disease. Such an agent would also be useful for reducing the level of serum CRP and, consequently, for treating diseases and conditions associated with an elevated serum CRP level.

### Summary of the Invention

The invention features compositions, methods, and kits for the treatment of immunoinflammatory disorders and ophthalmic disorders.

In one aspect, the invention features a composition that includes a drug pair selected from the pairs listed on Table 1A and Table 3. Desirably, one or both drugs are present in amounts that together are sufficient to treat an immunoinflammatory disorder, ophthalmic disorder, or musculoskeletal disorder, or pain, tenderness, impairment in mobility, soft tissue swelling, or bony swelling associated therewith in a patient in need thereof. Exemplary drug pairs are: antihistamine and phosphodiesterase inhibitor; antihistamine and SSRI; antihistamine and tricyclic compound; antinfective and anticoccidial compound; corticosteroid and antihistamine; corticosteroid and phosphodiesterase inhibitor; corticosteroid and prostaglandin; NsIDI and alpha-2 adrenoceptor agonist; NsIDI and antihistamine; NsIDI and NMDA antagonist/antidyskinetic; NsIDI and prostaglandin; NsIDI and sympathomimetic; prostaglandin and phosphodiesterase inhibitor; prostaglandin and tetra-substituted pyrimidopyrimidine; sympathomimetic and NMDA antagonist/antidyskinetic; sympathomimetic and prostaglandin; sympathomimetic and tetra-substituted pyrimidopyrimidine; sympathomimetic and tricyclic compound; tetra-substituted pyrimidopyrimidine and phosphodiesterase inhibitor; tetra-substituted pyrimidopyrimidine and SSRI; tetra-substituted pyrimidopyrimidine and tricyclic compound; and tricyclic compound and calcium channel blocker.

One or both drugs in said pair may be present in the composition in a low dosage or in a high dosage. In certain embodiments, the composition is formulated for topical or systemic administration. In one embodiment, the composition is formulated for ophthalmic admistration.

In another aspect, the invention features a method for treating a patient diagnosed with an immunoinflammatory disorder by administering to the patient a drug pair selected from the pairs listed on Table 1A or Table 3. Desirably, the first and second drug of said drug pair are administered simultaneously or within 14 days of each other and in amounts that together are sufficient to treat the patient. Optionally, the patient may also be administered one or more additional drugs (e.g., an NSAID, corticosteroid, COX-2 inhibitor, biologic, DMARD, small molecule immunomodulator, xanthine, anticholinergic compound, beta receptor agonist, bronchodilator, NsIDI, vitamin D analog, psoralen; retinoid, or 5-amino salicylic acid).

In a related aspect, the invention features a method of modulating an immune response (e.g., by decreasing proinflammatory cytokine secretion or production, or by modulating adhesion, gene expression, chemokine secretion, presentation of MHC complex, presentation of costimulation signals, or cell surface expression of other mediators) in a patient by administering to the patient a drug pair selected from the pairs listed on Table 1A simultaneously or within 14 days of each other in amounts sufficient to modulate the immune response in the patient.

In another aspect, the invention features a method for treating a patient diagnosed with an ophthalmic disorder by administering to the patient a drug pair selected from the pairs listed on Table 1A or Table 3. Desirably, the first and second drug of said drug pair are administered simultaneously or within 14 days of each other in amounts that together are sufficient to treat the patient. Optionally, the patient may also be administered one or more additional drugs (e:g., an anti-VEGF compound, corticosteroid, NSAID, antiallergic agent, antihistamine, glaucoma-treating agent, antibiotic, antiviral agent, or antimycotic agent) or receive photodynamic therapy.

In any of the foregoing methods one or both drugs in said pair are administered in a low dosage or in a high dosage. The drugs may be administered within 10 days of each other, within five days of each other, within twenty-four hours of each other, or simultaneously.

In a related aspect, the invention features a method for treating an immunoinflammatory disorder, an ophthalmic disorder, a musculoskeletal disorder or pain associated therewith, a periodontal disease, or a disease or condition associated with an increased serum CRP level in a patient in need thereof by concomitantly administering to the patient a drug pair selected from the pairs listed on Table 1A in amounts that together are more effective in treating the immunoinflammatory disorder than the administration of either drug alone.

In still another related aspect, the invention features a method for treating an immunoinflammatory disorder, an ophthalmic disorder, a musculoskeletal disorder or pain associated therewith, a periodontal disease, or a disease or condition associated with an increased serum CRP level in a patient in need thereof by administering a drug pair selected from the pairs listed on Table 1A to the patient; wherein: (i) the two drugs are concomitantly administered and (ii) the respective amounts of the two drugs administered to the patient are more effective in treating the immunoinflammatory disorder compared to the administration of the either drug alone.

In another aspect, the invention features a composition that includes (i) drug listed on Table 1A or Table 3; and (ii) a second compound selected from the group consisting of an anti-VEGF compound, corticosteroid, NSAID, antiallergic agent, antihistamine, glaucoma-treating agent, antibiotic, antiviral agent, antimycotic agent, COX-2 inhibitor, biologic, DMARD, small molecule immunomodulator, xanthine, anticholinergic compound, beta receptor agonist, bronchodilator, NsIDI, vitamin D analog, psoralen, retinoid, or 5-amino salicylic acid.

In another aspect, the invention features a kit having (i) a composition that includes a drug pair selected from the pairs listed on Table 1A or Table 3; and (ii) instructions for administering the composition to a patient diagnosed with an immunoinflammatory disorder, an ophthalmic disorder, a musculoskeletal disorder or pain associated therewith, a periodontal disease, or a disease or condition associated with an increased serum CRP level.

In a related aspect, the invention features a kit having (i) a first drug from a drug pair selected from the pairs listed on Table 1A or Table 3; (ii) a second drug from the same drug pair; and (iii) instructions for administering said the two drugs to a patient diagnosed an immunoinflammatory disorder, an ophthalmic disorder, a musculoskeletal disorder or pain associated therewith, a periodontal disease, or a disease or condition associated with an increased serum CRP level.

In another related aspect, the invention features a kit having (i) a first drug from a drug pair selected from the pairs listed on Table 1A or Table 3; and (ii) instructions for administering this drug and a second drug from the same drug pair to a patient diagnosed with an immunoinflammatory disorder, an ophthalmic disorder, a musculoskeletal disorder or pain associated therewith, a periodontal disease, or a disease or condition associated with an increased serum CRP level..

In yet another related aspect, the invention features a kit having (i) a first drug listed on Table 1A or Table 3; (ii) a second drug selected from an anti-VEGF compound, corticosteroid, NSAID, antiallergic agent, antihistamine, glaucoma-treating agent, antibiotic, antiviral agent, antimycotic agent, COX-2 inhibitor, biologic, DMARD, small molecule immunomodulator, xanthine, anticholinergic compound, beta receptor agonist, bronchodilator, NsIDI, vitamin D analog, psoralen, retinoid, or 5-amino salicylic acid; and (iii) instructions for administering said first drug and said second drug to a patient diagnosed with an immunoinflammatory disorder, an ophthalmic disorder, a musculoskeletal disorder or pain associated therewith, a periodontal disease, or a disease or condition associated with an increased serum CRP level.

In another related aspect, the invention features a kit having (i) a first drug listed on Table 1A or Table 3; and (ii) instructions for administering this first drug and a second drug selected from an anti-VEGF compound, corticosteroid, NSAID, antiallergic agent, antihistamine, glaucoma-treating agent, antibiotic, antiviral agent, antimycotic agent, COX-2 inhibitor, biologic, DMARD, small molecule immunomodulator, xanthine, anticholinergic compound, beta receptor agonist, bronchodilator, NsIDI, vitamin D analog, psoralen, retinoid, or 5-amino salicylic acid to a patient diagnosed with an immunoinflammatory disorder or an ophthalmic disorder.

The invention also features methods, compositions, and kits for treating a musculoskeletal disorder, or pain, tenderness, impairment in mobility, soft tissue swelling, or bony swelling associated with a musculoskeletal disorder, in a patient by administering to the patient in need thereof a drug pair selected from the pairs listed on Table 1A, optionally in combination with any of a number of companion compounds, including a corticosteroid, a non-steroidal anti-inflammatory drug (NSAID) (e.g., naproxen sodium, diclofenac sodium, diclofenac potassium, aspirin, sulindac, diflunisal, piroxicam, indomethacin, ibuprofen, nabumetone, choline magnesium trisalicylate, sodium salicylate, salicylsalicylic acid, fenoprofen, flurbiprofen, ketoprofen, meclofenamate sodium, meloxicam, oxaprozin, sulindac, or tolmetin), a COX-2 inhibitor (e.g., rofecoxib, celecoxib, valdecoxib, or lumiracoxib), a biologic (e.g., adelimumab, etanercept, or infliximab), a small molecule immunomodulator (e.g., VX 702, SCIO 469, doramapimod, RO 30201195, SCIO 323, DPC 333, pranalcasan, mycophenolate, or merimepodib), a disease-modifying anti-rheumatic drug (DMARD) (e.g., methotrexate or leflunomide), a xanthine (e.g., theophylline), a non-steroidal immunophilin-dependent immunosuppressant (NsIDI) (e.g., cyclosporine, tacrolimus, ascomycin, pimecrolimus, rapamycin, or everolimus), a vitamin D analog (e.g., calcipotriene or calcipotriol), a psoralen (e.g., methoxsalen), a retinoid (e.g., acitretin or tazoretene), 5-amino salicylic acid (e.g., mesalamine, sulfasalazine, balsalazide disodium, or olsalazine sodium), hydroxychloroquine sulfate, penicillamine, or an analog of any thereof, as described herein.

Accordingly, the invention features, in one instance, a method for treating pain, tenderness, impairment in mobility, soft tissue swelling, or bony swelling associated with a musculoskeletal disorder, e.g., osteoarthritis, by administering to a patient diagnosed with or at risk of developing such pain, tenderness, impairment in mobility, soft tissue swelling, or bony swelling a drug pair selected from the pairs listed on Table 1A, such that the two drugs are administered simultaneously or within fourteen days, ten days, five days, or even 24 hours of each other in amounts sufficient to treat the patient. Desirably, the patient experiences a reduction in pain, tenderness, impairment in mobility, soft tissue swelling, or bony swelling subsequent to treatment, e.g., within fifty days of treatment. The reduction in pain, tenderness, impairment in mobility, soft tissue swelling, or bony swelling can be measured using any method known in the art, e.g., a 10 cm visual analog scale, a Likert scale, the Lequesne index, or the WOMAC index. For example, an AUSCAN index that utilizes a 10 cm visual analog scale may be used.

The invention also features a method for treating a musculoskeletal disorder, e.g., osteoarthritis, by administering to a patient diagnosed with or at risk of developing such a disorder a drug pair selected from the pairs listed on Table 1A, such that the two drugs are administered simultaneously or within fourteen days, ten days, five days, or even 24 hours of each other in amounts sufficient to treat the patient.

In any of the foregoing instances, the two drugs may be administered in the same or different pharmaceutical formulations. Compounds used in the methods of the invention may be formulated for, e.g., topical or systemic administration, and may be formulated in high, moderate, or low dosages.

In addition, a third drug, e.g., a corticosteroid, an NSAID, a COX-2 inhibitor, a biologic, a small molecule immunomodulator, a DMARD, a xanthine, an NsIDI, a vitamin D analog, a psoralen, a retinoid, 5-amino salicylic acid, hydroxychloroquine sulfate, or penicillamine may be administered to the patient such that the first drug, the second drug, and the third drug are administered simultaneously or within fourteen days, ten days, five days, or even 24 hours of each other in amounts sufficient to treat the patient.

In other embodiments, the drugs of the drug combination of Table 1A or Table 3 are the only two active ingredients (although excipients will generally also be present).

In another aspect, the invention features a method of treating an ophthalmic disorder in a patient by administering to the patient a corticosteroid and a non-steroidal immunophilin-dependent immunosuppressant (NsIDI). In this aspect of the invention, the corticosteroid and/or the NsIDI can be administered at a low concentration. Desirably, the concentration of the NsIDI does not cause eye irritation, such as burning, and the compositions of the invention are administered in an amount sufficient to alleviate the symptoms of the ophthalmic disorder. Also desirably; the concentration of the corticosteroid does not cause steroid toxicity.

In another aspect, the invention features a method of treating an ophthalmic disorder in a patient by administering to the patient a substance selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents in combination with a corticosteroid and/or an NsIDI. In this aspect of the invention, the corticosteroid and/or the NsIDI can be administered at a low concentration.

In another aspect, the invention features a composition (e.g., a solution, gel, ointment, suspension, emulsion, or solid insert) including a corticosteroid and a NsIDI. In this aspect of the invention, the corticosteroid and/or the NsIDI can be administered at a low concentration.

In another aspect, the invention features a composition (e.g., a solution, gel, ointment, suspension, emulsion, or solid insert) including a substance selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents in combination with a corticosteroid and/or an NsIDI. In this aspect of the invention, the corticosteroid and/or the NsIDI can be administered at a low concentration.

The invention also features a kit that includes (i) a corticosteroid; and (ii) instructions for administering a corticosteroid and an NsIDI to a patient having or at risk of having an ophthalmic disorder.

The invention also features a kit that includes (i) an NsIDI; and (ii) instructions for administering a corticosteroid and an NsIDI to a patient having or at risk of having an ophthalmic disorder.

The invention also features a kit that includes (i) a composition containing a corticosteroid and an NsIDI; and (ii) instructions for administering the composition to a patient having or at risk of having a metabolic disorder.

The invention also features a kit that includes (i) a corticosteroid; (ii) an NsIDI; and (iii) instructions for administering a corticosteroid and an NsIDI to a patient having or at risk of having an ophthalmic disorder.

Any of the foregoing kits can also include instructions for administering a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents.

Any of the foregoing kits can also include a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents. In these kits, the NsIDI and/or corticosteroid can optionally be formulated in a single composition with a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents.

The invention also features a kit that includes (i) a corticosteroid; and (ii) instructions for administering a corticosteroid and a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents, to a patient having or at risk of having an ophthalmic disorder.

The invention also features a kit that includes (i) an NsIDI; and (ii) instructions for administering an NsIDI and a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents, to a patient having or at risk of having an ophthalmic disorder.

The invention also features a kit that includes (i) a composition containing a corticosteroid and a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents; and (ii) instructions for administering the composition to a patient having or at risk of having a metabolic disorder.

The invention also features a kit that includes (i) a composition containing an NsIDI and a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents; and (ii) instructions for administering the composition to a patient having or at risk of having a metabolic disorder.

The invention also features a kit that includes (i) a corticosteroid; (ii) a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents (iii) instructions for administering a corticosteroid and a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents, to a patient having or at risk of having an ophthalmic disorder.

The invention also features a kit that includes (i) a NsIDI; (ii) a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents (iii) instructions for administering a NsIDI and a compound selected from: dipivefrin, anti-VEGF therapies, photodynamic therapy, NSAIDs, antiallergic agents, antihistamines, glaucoma-treating agents, artificial tears, antibiotics, antiviral agents, and antimycotic agents, to a patient having or at risk of having an ophthalmic disorder.

In any of the forgoing aspects of the invention, the corticosteroid can be selected from SEGRAs (selective glucocorticosteroid receptor agonists), fluocinolone acetonide, fluorometholone, dexamethasone, hydrocortisone, loteprednol, medrysone, methylprednisolone, prednisolone, rimexolone, or triamcinolone.

In any of the forgoing aspects of the invention, the NsIDI can be selected from cyclosporine A, ABT-281, ISAtx247, tacrolimus, ascomycin, pimecrolimus, rapamycin, or everolimus.

In any of the foregoing aspects of the invention, the concentration of the corticosteroid can be equivalent to a concentration of prednisolone of between 0.01% and 0.1% (e.g., 0.1%, 0.09%, 0.08%, 0.07%, 0.06%, 0.05%, or 0.01%) and the concentration of the non-steroidal immunophilin-dependent immunosuppressant can be equivalent to a concentration of cyclosporine A between 0.001% and 0.049% (e.g., 0.04%, 0.03%, 0.02%, 0.01%, 0.009%, 0.008%, 0.007%, 0.006%, 0.005%, or 0.001%).

In one embodiment, the corticosteroid is prednisolone and the concentration of prednisolone is between 0.01% and 0.12% (e.g., 0.12%, 0.1%, 0.09%, 0.08%, 0.07%, 0.06%, 0.05%, and 0.01%).

In another embodiment, the corticosteroid is clocortolone pivalate and the concentration of clocortolone pivalate is between 0.01% and 0.1% (e.g., 0.1%, 0.09%, 0.08%, 0.07%, 0.06%, 0.05%, and 0.01%).

In another embodiment, the corticosteroid is hydrocortisone and the concentration of hydrocortisone is between 0.01% and 1.0% (e.g., 1.0%, 0.9%, 0.8%, 0.7%, 0.6%, 0.5%, 0.1%, 0.09%, 0.08%, 0.07%, 0.06%, 0.05%, and 0.01%).

In another embodiment, the corticosteroid is dexamethasone and the concentration of dexamethasone is between 0.01% and 0.1% (e.g., 0.1%, 0.09%, 0.08%, 0.07%, 0.06%, 0.05%, and 0.01%).

In another embodiment, the corticosteroid is fluorometholone and the concentration of fluorometholone is between 0.01% and 0.1% (e.g., 0.1%, 0.09%, 0.08%, 0.07%, 0.06%, 0.05%, and 0.01%).

In another embodiment, the corticosteroid is loteprednol etabonate and the concentration of loteprednol etabonate is between 0.01% and 0.2% (e.g., 0.2%, 0.1%, 0.09%, 0.08%, 0.07%, 0.06%, 0.05%, and 0.01%).

In another embodiment, the corticosteroid is medrysone and the concentration ofmedrysone is between 0.01% and 1.0% (e.g., 1.0%, 0.9%, 0.8%, 0.7%, 0.6%, 0.5%, 0.1%, 0.09%, 0.08%, 0.07%, 0.06%, 0.05%, and 0.01%).

In another embodiment, the corticosteroid is rimexolone and the concentration of rimexolone is between 0.01% and 1.0% (e.g., 1.0%, 0.9%, 0.8%, 0.7%, 0.6%, 0.5%, 0.1%, 0.09%, 0.08%, 0.07%, 0.06%, 0.05%, and 0.01%).

In any of the foregoing embodiments, the NsIDI can be cyclosporine A and the concentration of cyclosporine A is between 0.001% and 0.049% (e.g., 0.04%, 0.03%, 0.02%, 0.01%, 0.009%, 0.008%, 0.007%, 0.006%, 0.005%, and 0.001%).

In certain embodiments of the compositions, kits, and methods of the invention, the only pharmacologically active agents in the composition or kit, or used in the method, are those recited. In this embodiment, pharmacologically inactive excipients may also be present in the composition or kit, or used in the practice of the method.

The invention features the treatment of an ophthalmic disorder, for example age related macular degeneration, alkaline erosive keratoconjunctivitis, allergic conjunctivitis, allergic keratitis, anterior uveitis, Behcet's disease, blepharitis, blood-aqueous barrier disruption, chorioiditis, chronic uveitis, conjunctivitis, contact lens-induced keratoconjunctivitis, corneal abrasion, corneal trauma, corneal ulcer, crystalline retinopathy, cystoid macular edema, dacryocystitis, diabetic keratophathy, diabetic macular edema, diabetic retinopathy, dry eye disease, dry age-related macular degeneration, eosinophilic granuloma, episcleritis, exudative macular edema, Fuchs' Dystrophy, giant cell arteritis, giant papillary conjunctivitis, glaucoma, glaucoma surgery failure, graft rejection, herpes zoster, inflammation after cataract surgery, iridocorneal endothelial syndrome, iritis, keratoconjunctiva sicca, keratoconjunctival inflammatory disease, keratoconus, lattice dystrophy, map-dot-fingerprint dystrophy, necrotic keratitis, neovascular diseases involving the retina, uveal tract or cornea such as neovascular glaucoma, corneal neovascularization, neovascularization resulting following a combined vitrectomy and lensectomy, neovascularization of the optic nerve, and neovascularization due to penetration of the eye or contusive ocular injury, neuroparalytic keratitis, non-infectious uveitisocular herpes, ocular lymphoma, ocular rosacea, ophthalmic infections, ophthalmic pemphigoid, optic neuritis, panuveitis, papillitis, pars planitis, persistent macular edema, phacoanaphylaxis, posterior uveitis, post-operative inflammation, proliferative diabetic retinopathy, proliferative sickle cell retinopathy, proliferative vitreoretinopathy, retinal artery occlusion, retinal detachment, retinal vein occlusion, retinitis pigmentosa, retinopathy of prematurity, rubeosis iritis, scleritis, Stevens-Johnson syndrome, sympathetic ophthalmia, temporal arteritis, thyroid associated ophthalmopathy, uveitis, vernal conjunctivitis, vitamin A insufficiency-induced keratomalacia, vitreitis, or wet age-related macular degeneration.

Compounds useful in the invention include those described herein in any of their pharmaceutically acceptable forms, including isomers such as diastereomers and enantiomers, salts, esters, solvates, and polymorphs thereof, as well as racemic mixtures and pure isomers of the compounds described herein.

By "corticosteroid" is meant any naturally occurring or synthetic compound characterized by a hydrogenated cyclopentanoperhydrophenanthrene ring system and having immunosuppressive and/or anti-inflammatory activity. Naturally occurring corticosteroids are generally produced by the adrenal cortex. Synthetic corticosteroids may be halogenated. Examples corticosteroids are provided herein.

By "non-steroidal immunophilin-dependent immunosuppressant" or "NsIDI" is meant any non-steroidal agent that decreases proinflammatory cytokine production or secretion, binds an immunophilin, or causes a down regulation of the proinflammatory reaction. NsIDIs include calcineurin inhibitors, such as cyclosporine A, ABT-281, ISAtx247, tacrolimus, ascomycin, pimecrolimus, as well as other agents (peptides, peptide fragments, chemically modified peptides, or peptide mimetics) that inhibit the phosphatase activity of calcineurin. NsIDIs also include rapamycin (sirolimus) and everolimus, which bind to an FK506-binding protein, FKBP-12, and block antigen-induced proliferation of white blood cells and cytokine secretion.

By "small molecule immunomodulator" is meant a non-steroidal, non-NsIDI compound that decreases proinflammatory cytokine production or secretion, causes a down regulation of the proinflammatory reaction, or otherwise modulates the immune system in an immunophilin-independent manner. Exemplary small molecule immunomodulators are p38 MAP kinase inhibitors such as VX 702 (Vertex Pharmaceuticals), SCIO 469 (Scios), doramapimod (Boehringer Ingelheim), RO 30201195 (Roche), and SCIO 323 (Scios), TACE inhibitors such as DPC 333 (Bristol Myers Squibb), ICE inhibitors such as pranalcasan (Vertex Pharmaceuticals), and IMPDH inhibitors such as mycophenolate (Roche) and merimepodib (Vertex Pharmaceuticals).

By "tricyclic compound" is meant a compound having one the formulas (I), (II), (III), (IV), or (V): 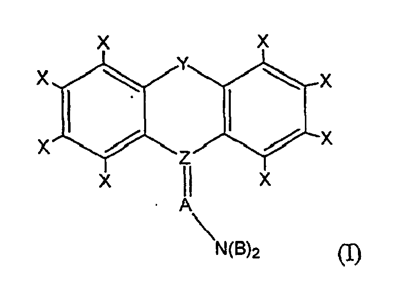 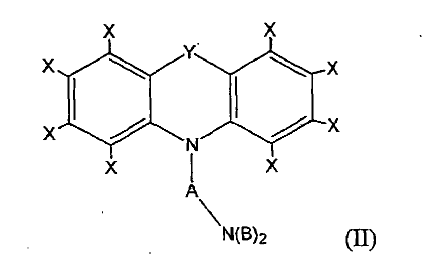 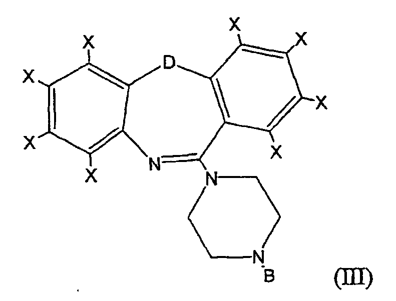 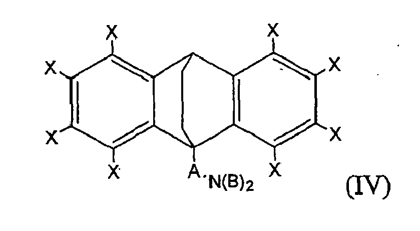 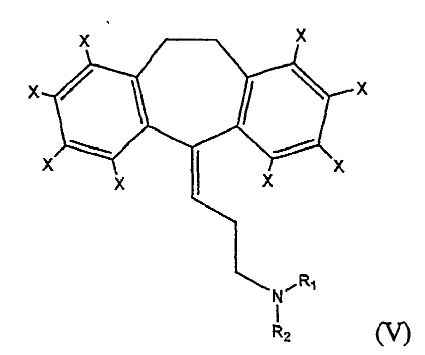 wherein each X is, independently, H, Cl, F, Br, I, CH₃, CF₃, OH, OCH₃, CH₂CH₃, or OCH₂CH₃;Y is CH₂, O, NH, S(O)₀₋₂, (CH₂)₂, (CH)₂, CH₂O, CH₂NH, CHN, or CH₂S; Z is C or S; A is a branched or unbranched, saturated or monounsaturated hydrocarbon chain having between 3 and 6 carbons, inclusive; each B is, independently, H, Cl, F, Br, I, CX₃, CH₂CH₃, OCX₃, or OCX₂CX₃; and D is CH₂, O, NH, or S(O)₀₋₂. In preferred embodiments, each X is, independently, H, Cl, or F; Y is (CH₂)₂, Z is C; A is (CH₂)₃; and each B is, independently, H, Cl, or F. Other tricyclic compounds are described below. Tricyclic compounds include tricyclic antidepressants such as amoxapine, 8-hydroxyamoxapine, 7-hydroxyamoxapine, loxapine (e.g., loxapine succinate, loxapine hydrochloride), 8-hydroxyloxapine, amitriptyline, clomipramine, doxepin, imipramine, trimipramine, desipramine, nortriptyline, and protriptyline, although compounds need not have antidepressant activities to be considered tricyclic compounds of the invention.

By "tetra-substituted pyrimidopyrimidine" is meant a compound having the formula (V): 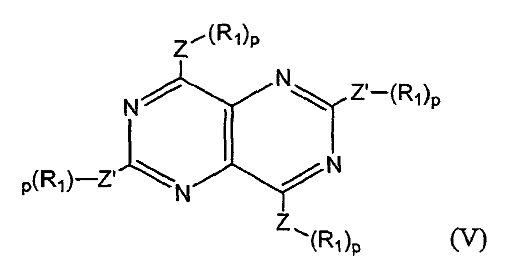 wherein each Z and each Z' is, independently, N, O, C, 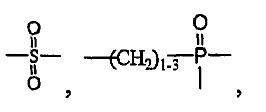 or

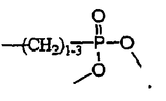

When Z or Z' is O or 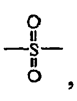 then p=1, when Z or Z' is N, 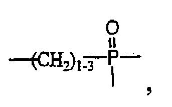 or 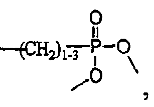 then p=2, and when Z or Z' is C, then p=3. In formula (V), each R₁ is, independently, X, OH, N-alkyl (wherein the alkyl group has 1 to 20, more preferably 1-5, carbon atoms); a branched or unbranched alkyl group having 1 to 20, more preferably 1-5, carbon atoms; or a heterocycle, preferably as defined in formula (V). Alternatively, when p>1, two R₁ groups from a common Z or Z' atom, in combination with each other, may represent -(CY₂)ₖ- in which k is an integer between 4 and 6, inclusive. Each X is, independently, Y, CY₃, C(CY₃)₃, CY₂CY₃, (CY₂)₁₋₅OY, substituted or unsubstituted cycloalkane of the structure CₙY₂ₙ₋₁, wherein n= 3-7, inclusive. Each Y is, independently, H, F, Cl, Br, or I. In one embodiment, each Z is the same moiety, each Z' is the same moiety, and Z and Z' are different moieties.

By "prostaglandin" is meant a member of the lipid class of biochemicals that belongs to a subclass of lipids known as the eicosanoids, because of their structural similarities to the C-20 polyunsaturated fatty acids, the eicosaenoic acids. Prostaglandins include alprostidil, dinoprostone, limaprost, misoprostil, prostaglandin E2, prostaglandin A1, prostaglandin A2, prostaglandin B1, prostaglandin B2, prostaglandin D2, prostaglandin F1α, prostaglandin F2α, prostaglandin I1, prostaglandin-ici 74205, prostaglandin F2β, 6-keto-prostaglandin F1α, prostaglandin E1 ethyl ester, prostaglandin E1 methyl ester, prostaglandin F2 methyl ester, arbaprostil, ornoprostil, 13,14-dihydroprostaglandin F2α, and prostaglandin J.

By "bufexamac" is meant a compound having the structure: 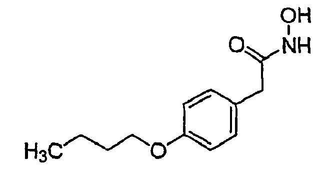 or a pharmaceutically acceptable salt or prodrug thereof.

By "bufexamac analog" is meant a compound having the formula (VI): 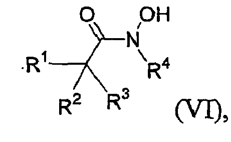 or a pharmaceutically acceptable salt or prodrug thereof, wherein R¹ is 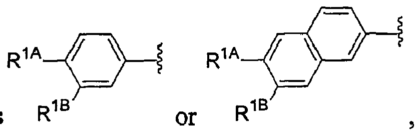 wherein R^{1A} is and R^{1B} is H, halo, CF₃, optionally substituted C₁₋₆ alkyl, optionally substituted C₂₋₆ alkenyl, optionally substituted C₂₋₆ alkynyl, optionally substituted C₃₋₈ cycloalkyl, optionally substituted C₁₋₆ alkoxy, or optionally substituted C₁₋₆ thioalkoxy; each of R² and R³ is, independently, H, C₁₋₄ alkyl, or CF₃; and R⁴ is optionally substituted C₁₋₆ alkyl or optionally substituted C₃₋₈ cycloalkyl.

By a "low dosage" or "low concentration" is meant at least 5% less (e.g., at least 10%, 20%, 50%, 80%, 90%, or even 95%) than the lowest standard recommended dosage or lowest standard recommended concentration of a particular compound formulated for a given route of administration for treatment of any human disease or condition. For example, a low dosage of corticosteroid formulated for administration by inhalation will differ from a low dosage of corticosteroid formulated for oral administration.

By a "high dosage" is meant at least 5% (e.g., at least 10%, 20%, 50%, 100%, 200%, or even 300%) more than the highest standard recommended dosage of a particular compound for treatment of any human disease or condition.

By a "moderate dosage" is meant the dosage between the low dosage and the high dosage.

By a "dosage equivalent to a prednisolone dosage" is meant a dosage of a corticosteroid that, in combination with a given dosage of a second drug produces the same anti-inflammatory effect in a patient as a dosage of prednisolone in combination with that dosage.

By "selective serotonin reuptake inhibitor" or "SSRI" is meant any member of the class of compounds that (i) inhibit the uptake of serotonin by neurons of the central nervous system, (ii) have an inhibition constant (Ki) of 10 nM or less, and (iii) a selectivity for serotonin over norepinephrine (i.e., the ratio of Ki(norepinephrine) over Ki(serotonin)) of greater than 100. Typically, SSRIs are administered in dosages of greater than 10 mg per day when used as antidepressants. Exemplary SSRIs for use in the invention are fluoxetine, fluvoxamine, paroxetine, sertraline, citalopram, and venlafaxine.

By "treating" is meant administering or prescribing a pharmaceutical composition for the treatment or prevention of an immunoinflammatory disease.

By "patient" is meant any animal (e.g., a human). Other animals that can be treated using the methods, compositions, and kits of the invention include horses, dogs, cats, pigs, goats, rabbits, hamsters, monkeys, guinea pigs, rats, mice, lizards, snakes, sheep, cattle, fish, and birds.

By "steroid toxicity" is meant a substantial detrimental increase in intraocular pressure resulting from steroid administration.

By "an amount sufficient" is meant the amount of a compound, in a combination of the invention, required to treat or prevent an immunoinflammatory disease in a clinically relevant manner. A sufficient amount of an active compound used to practice the present invention for therapeutic treatment of conditions caused by or contributing to an immunoinflammatory disease varies depending upon the manner of administration, the age, body weight, and general health of the patient. Ultimately, the prescribers will decide the appropriate amount and dosage regimen.

By "more effective" is meant that a method, composition, or kit exhibits greater efficacy, is less toxic, safer, more convenient, better tolerated, or less expensive, or provides more treatment satisfaction than another method, composition, or kit with which it is being compared. Efficacy may be measured by a skilled practitioner using any standard method that is appropriate for a given indication.

The term "immunoinflammatory disorder" encompasses a variety of conditions, including autoimmune diseases, proliferative skin diseases, and inflammatory dermatoses. Immunoinflammatory disorders result in the destruction of healthy tissue by an inflammatory process, dysregulation of the immune system, and unwanted proliferation of cells. Examples of immunoinflammatory disorders are acne vulgaris; acute respiratory distress syndrome; Addison's disease; adrenocortical insufficiency; adrenogenital ayndrome; allergic conjunctivitis; allergic rhinitis; allergic intraocular inflammatory diseases, ANCA-associated small-vessel vasculitis; angioedema; ankylosing spondylitis; aphthous stomatitis; arthritis, asthma; atherosclerosis; atopic dermatitis; autoimmune disease; autoimmune hemolytic anemia; autoimmune hepatitis; Behcet's disease; Bell's palsy; berylliosis; bronchial asthma; bullous herpetiformis dermatitis; bullous pemphigoid; carditis; celiac disease; cerebral ischaemia; chronic obstructive pulmonary disease; cirrhosis; Cogan's syndrome; contact dermatitis; COPD; Crohn's disease; Cushing's syndrome; dermatomyositis; diabetes mellitus; discord lupus erythematosus; eosinophilic fasciitis; epicondylitis; erythema nodosum; exfoliative dermatitis; fibromyalgia; focal glomerulosclerosis; giant cell arteritis; gout; gouty arthritis; graft-versus-host disease; hand dermatitis; Henoch-Schonlein purpura; herpes gestationis; hirsutism; hypersensitivity drug reactions; idiopathic ceratoscleritis; idiopathic pulmonary fibrosis; idiopathic thrombocytopenic purpura; inflammatory bowel or gastrointestinal disorders, inflammatory dermatoses; juvenile rheumatoid arthritis; laryngeal edema; lichen planus; Loeffler's syndrome; lupus nephritis; lupus vulgaris; lymphomatous tracheobronchitis; macular edema; multiple sclerosis; musculoskeletal and connective tissue disorder; myasthenia gravis; myositis; obstructive pulmonary disease; ocular inflammation; organ transplant rejection; osteoarthritis; pancreatitis; pemphigoid gestationis; pemphigus vulgaris; polyarteritis nodosa; polymyalgia rheumatica; primary adrenocortical insufficiency; primary billiary cirrhosis; pruritus scroti; pruritis/inflammation, psoriasis; psoriatic arthritis; Reiter's disease; relapsing polychondritis; rheumatic carditis; rheumatic fever; rheumatoid arthritis; rosacea caused by sarcoidosis; rosacea caused by scleroderma; rosacea caused by Sweet's syndrome; rosacea caused by systemic lupus erythematosus; rosacea caused by urticaria; rosacea caused by zoster-associated pain; sarcoidosis; scleroderma; segmental glomerulosclerosis; septic shock syndrome; serum sickness; shoulder tendinitis or bursitis; Sjogren's syndrome; Still's disease; stroke-induced brain cell death; Sweet's disease; systemic dermatomyositis; systemic lupus erythematosus; systemic sclerosis; Takayasu's arteritis; temporal arteritis; thyroiditis; toxic epidermal necrolysis; tuberculosis; type-1 diabetes; ulcerative colitis; uveitis; vasculitis; and Wegener's granulomatosis.

"Non-dermal inflammatory disorders", include rheumatoid arthritis, inflammatory bowel disease, asthma, and chronic obstructive pulmonary disease.

By "dermal inflammatory disorders" or "inflammatory dermatoses" is meant an inflammatory disorder selected from psoriasis, guttate psoriasis, inverse psoriasis, pustular psoriasis, erythrodermic psoriasis, acute febrile neutrophilic dermatosis, eczema, asteatotic eczema, dyshidrotic eczema, vesicular palmoplantar eczema, acne vulgaris, atopic dermatitis, contact dermatitis, allergic contact dermatitis, dermatomyositis, exfoliative dermatitis, hand eczema, pompholyx, rosacea, rosacea caused by sarcoidosis, rosacea caused by scleroderma, rosacea caused by Sweet's syndrome, rosacea caused by systemic lupus erythematosus, rosacea caused by urticaria, rosacea caused by zoster-associated pain, Sweet's disease, neutrophilic hidradenitis, sterile pustulosis, drug eruptions, seborrheic dermatitis, pityriasis rosea, cutaneous kikuchi disease, pruritic urticarial papules and plaques of pregnancy, Stevens-Johnson Syndrome and toxic epidermal necrolysis, tatoo reactions, Wells Syndrome (eosinophilic cellulitis), reactive arthritis (Reiter's Syndrome), bowel-associated dermatosis-arthritis syndrome, rheumatoid neutrophilic dermatosis, neutrophilic eccrine hidradenitis, neutrophilic dermatosis of the dorsal hands, balanitis circumscripta plasmacellularis, balanoposthitis, Behcet's disease, erythema annulare centrifugum, erythema dyschromicum perstans, erythema multiforme, granuloma annulare, hand dermatitis, lichen nitidus, lichen planus, lichen sclerosus et atrophicus, lichen simplex chronicus, lichen spinulosus, nummular dermatitis, pyoderma gangrenosum, sarcoidosis, subcorneal pustular dermatosis, urticaria, and transient acantholytic dermatosis.

By "proliferative skin disease" is meant a benign or malignant disease that is characterized by accelerated cell division in the epidermis or dermis. Examples of proliferative skin diseases are psoriasis, atopic dermatitis, nonspecific dermatitis, primary irritant contact dermatitis, allergic contact dermatitis, actinic keratosis, basal and squamous cell carcinomas of the skin, lamellar ichthyosis, epidermolytic hyperkeratosis, premalignant keratosis, acne, and seborrheic dermatitis.

By "musculoskeletal disorder" is meant an immune system-related disorder of the muscles, ligaments, bones, joints, cartilage, or other connective tissue. Among the most commonly-occurring musculoskeletal disorders are various forms of arthritis, e.g., osteoarthritis, rheumatoid arthritis, juvenile rheumatoid arthritis, and gout. Other musculoskeletal disorders include acquired hyperostosis syndrome, acromegaly, ankylosing spondylitis, Behcet's disease, bone diseases, bursitis, cartilage diseases, chronic fatigue syndrome, compartment syndromes, congenital hypothyroidism, congenital myopathies, dentigerous cyst, dermatomyositis, diffuse idiopathic skeletal hyperostosis, Dupuytren's contracture, eosinophilia-myalgia syndrome, fasciitis, Felty's syndrome, fibromyalgia, hallux valgus, infectious arthritis, joint diseases, Kabuki make-up syndrome, Legg-Perthes disease, lupus, Lyme disease, Melas syndrome, metabolic bone diseases, mitochondrial myopathies, mixed connective tissue disease, muscular diseases, muscular dystrophies, musculoskeletal abnormalities, musculoskeletal diseases, myositis, myositis ossificans, necrotizing fasciitis, neurogenic arthropathy, osteitis deformans, osteochondritis, osteomalacia, osteomyelitis, osteonecrosis, osteoporosis, Paget's disease, Pierre Robin syndrome, polymyalgia rheumatica, polymyositis, postpoliomyelitis syndrome, pseudogout, psoriatric arthritis, reactive arthritis, Reiter disease, relapsing polychondritis, renal osteodystrophy, rhabdomyolysis, rheumatic diseases, rheumatic fever, scleroderma, Sever's disease (calceneal apophysitis), Sjögren's syndrome, spinal diseases, spinal stenosis, Still's disease, synovitis, temporomandibular joint disorders, tendinopathy, tennis elbow, tenosynovitis, Tietze's syndrome, and Wegener's granulomatosis.

"Ophthalmic disorder" refers to physiologic abnormalities of the eye. They may involve the retina, the vitreous humor, lens, cornea, sclera or other portions of the eye, or physiologic abnormalities which adversely affect the eye, such as inadequate tear production.

Ophthalmic disorders that can be treated using the compositions, methods, and kits of the invention include age related macular degeneration, alkaline erosive keratoconjunctivitis, allergic conjunctivitis, allergic keratitis, anterior uveitis, Behcet's disease, blepharitis, blood-aqueous barrier disruption, chorioiditis, chronic uveitis, conjunctivitis, contact lens-induced keratoconjunctivitis, corneal abrasion, corneal trauma, corneal ulcer, crystalline retinopathy, cystoid macular edema, dacryocystitis, diabetic keratophathy, diabetic macular edema, diabetic retinopathy, dry eye disease, dry age-related macular degeneration, eosinophilic granuloma, episcleritis, exudative macular edema, Fuchs' Dystrophy, giant cell arteritis, giant papillary conjunctivitis, glaucoma, glaucoma surgery failure, graft rejection, herpes zoster, inflammation after cataract surgery, iridocorneal endothelial syndrome, iritis, keratoconjunctiva sicca, keratoconjunctival inflammatory disease, keratoconus, lattice dystrophy, map-dot-fingerprint dystrophy, necrotic keratitis, neovascular diseases involving the retina, uveal tract or cornea such as neovascular glaucoma, corneal neovascularization, neovascularization resulting following a combined vitrectomy and lensectomy, neovascularization of the optic nerve, and neovascularization due to penetration of the eye or contusive ocular injury, neuroparalytic keratitis, non-infectious uveitisocular herpes, ocular lymphoma, ocular rosacea, ophthalmic infections, ophthalmic pemphigoid, optic neuritis, panuveitis, papillitis, pars planitis, persistent macular edema, phacoanaphylaxis, posterior uveitis, post-operative inflammation, proliferative diabetic retinopathy, proliferative sickle cell retinopathy, proliferative vitreoretinopathy, retinal artery occlusion, retinal detachment, retinal vein occlusion, retinitis pigmentosa, retinopathy of prematurity, rubeosis iritis, scleritis, Stevens-Johnson syndrome, sympathetic ophthalmia, temporal arteritis, thyroid associated ophthalmopathy, uveitis, vernal conjunctivitis, vitamin A insufficiency-induced keratomalacia, vitreitis, or wet age-related macular degeneration.

As will be appreciated by one skilled in the art, a particular disease, disorder, or condition may be characterized as being both a proliferative skin disease and an inflammatory dermatosis. An example of such a disease is psoriasis.

As also will be appreciated, a particular disease may be both an immunoinflammatory disorder and an ophthalmic disorder. One such example is Behcet's disease.

The term "periodontal disease" encompasses a variety of conditions, including gingivitis and periodontitis, as well as diseases of tissues that surround and support teeth, including the gingiva, cementum, periodontal ligament, alveolar process bone, and dental supporting bone.

By "a disease or condition associated with an increased serum CRP level" is meant any disease or disorder in which the level of serum CRP may be elevated compared to normal controls. Typically a serum CRP level of >3 mg/L is considered elevated. Such diseases and conditions associated with an increased serum CRP level include cardiovascular diease (e.g., coronary artery disease, peripheral artery disease); hypertension; colon cancer; lymphoma; sarcoma; and pancreatitis.

By "sustained release" or "controlled release" is meant that the therapeutically active component is released from the formulation at a controlled rate such that therapeutically beneficial blood levels (but below toxic levels) of the component are maintained over an extended period of time ranging from e.g., about 12 to about 24 hours, thus, providing, for example, a 12 hour or a 24 hour dosage form.

In the generic descriptions of compounds of this invention, the number of atoms of a particular type in a substituent group is generally given as a range, e.g., an alkyl group containing from 1 to 4 carbon atoms or C₁₋₄ alkyl. Reference to such a range is intended to include specific references to groups having each of the integer number of atoms within the specified range. For example, an alkyl group from 1 to 4 carbon atoms includes each of C₁, C₂, C₃, and C₄. A C₁₋₁₂ heteroalkyl, for example, includes from 1 to 12 carbon atoms in addition to one or more heteroatoms. Other numbers of atoms and other types of atoms may be indicated in a similar manner.

As used herein, the terms "alkyl" and the prefix "alk-" are inclusive of both straight chain and branched chain groups and of cyclic groups, i.e., cycloalkyl. Cyclic groups can be monocyclic or polycyclic and preferably have from 3 to 6 ring carbon atoms, inclusive. Exemplary cyclic groups include cyclopropyl, cyclobutyl, cyclopentyl, and cyclohexyl groups.

By "C₁₋₄ alkyl" is meant a branched or unbranched hydrocarbon group having from 1 to 4 carbon atoms. A C₁₋₄ alkyl group may be substituted or unsubstituted. Exemplary substituents include alkoxy, aryloxy, sulfhydryl, alkylthio, arylthio, halide, hydroxyl, fluoroalkyl, perfluoralkyl, amino, aminoalkyl, disubstituted amino, quaternary amino, hydroxyalkyl, carboxyalkyl, and carboxyl groups. C₁₋₄ alkyls include, without limitation, methyl, ethyl, n-propyl, isopropyl, cyclopropyl, cyclopropylmethyl, n-butyl, iso-butyl, sec-butyl, tert-butyl, and cyclobutyl.

By "halogen" is meant bromine, chlorine, iodine, or fluorine.

By "alkoxy" is meant a chemical substituent of the formula -OR, wherein R is selected from C₁₋₇ alkyl, C₂₋₇ alkenyl, C₂₋₇ alkynyl, C₂₋₆ heterocyclyl, C₆₋₁₂ aryl, C₇₋₁₄ alkaryl, C₃₋₁₀ alkheterocyclyl, or C₁₋₇ heteroalkyl.

The term "pharmaceutically acceptable salt" represents those salts which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. The salts can be prepared in situ during the final isolation and purification of the compounds of the invention, or separately by reacting the free base function with a suitable organic acid. Representative acid addition salts include acetate, adipate, alginate, ascorbate, aspartate, benzenesulfonate, benzoate, bisulfate, borate, butyrate, camphorate, camphersulfonate, citrate, cyclopentanepropionate, digluconate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, isethionate, lactobionate, lactate, laurate, lauryl sulfate, malate, maleate, malonate, mesylate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium, potassium, calcium, magnesium, and the like, as well as nontoxic ammonium, quaternary ammonium, and amine cations, including, but not limited to ammonium, tetramethylammonium, tetraethylammonium, methylamine, dimethylamine, trimethylamine, triethylamine, ethylamine, and the like.

Compounds useful in the invention include those described herein in any of their pharmaceutically acceptable forms, including isomers such as diastereomers and enantiomers, salts, esters, amides, thioesters, solvates, and polymorphs thereof, as well as racemic mixtures and pure isomers of the compounds described herein.

### Brief Description of the Drawings

Figs. 1A-1MM are graphs showing suppression of PMA/ionomycin-induced TNFα secretion in cells trearted with a drug pair selected from the pairs listed on Table 1A or Table 3.
Figs. 2A-2LL are graphs showing suppression of IFNγ, IL-2, and TNFα in cells treated with combinations of an NsIDI and a corticosteroid.

Other features and advantages of the invention will be apparent from the following detailed description.

### Detailed Description

The invention features methods, compositions, and kits for the treatment of immunoinflammatory disorders. In one embodiment of the invention, treatment of an immunoinflammatory disorder is performed by administering two drugs simultaneously or within 14 days of each other to a patient in need of such treatment.

The invention also features methods, compositions, and kits for the treatment of ophthalmic disorders. In one embodiment, a patient with an ophthalmic disorder is treated by administering two drugs simultaneously.

Cyclosporine A, a non-steroidal immunophilin-dependent immunosuppressant (NsIDI), is approved for treating several ophthalmic conditions. Cyclosporine A causes eye irritation and other undesired side effects when administered to patients at the lowest approved concentration. Lower concentrations of cyclosporine A do not cause these undesired side effects but are not sufficient to alleviate the symptoms of the ophthalmic disorders.

Both corticosteroids and NsIDIs suppress cytokine production in cell culture models of immune function (Table 1B). We have discovered that combinations of certain NsIDIs with certain corticosteroids suppress cytokine production in a synergistic manner.

Based upon these data, we propose that when combined with a corticosteroid, low concentrations of cyclosporine A are sufficient to alleviate the symptoms of ophthalmic disorders while not causing undesired side effects.

The invention is based on our discovery that certain combinations of drugs inhibit cytokine production and/or secretion in vitro. The combinations are listed on Table 1A and Table 1B.

**Table 1A**

| **Drug 1** | **Drug 2** | **Drug 1** | **Drug 2** |
|---|---|---|---|
| Prednisolone | Ibudilast | Ibudilast | Desloratadine |
| Prednisolone | RO-20-1724 | Dipivefrin | Dipyridamole |
| Prednisolone | Epinastine | Dipivefrin | Limaprost |
| Prednisolone | Prostaglandin | Dipivefrin | Prostaglandin |
| Prednisolone | Bufexamac | Dipivefrin | Bufexamac |
| Cyclosporine A | Brimonidine | Dipivefrin | Amoxapine |
| Cyclosporine A | Epinastine | Dipivefrin | Amantadine |
| Cyclosporine A | Dipivefrin | Prostaglandin E | Ibudilast |
| Cyclosporine A | Prostaglandin E | Misoprostol | Ibudilast |
| Cyclosporine A | Amantadine | Misoprostol | RO-20-1724 |
| Cyclosporine A | Apraclonidine | Prostaglandin E | Dipyridamole |
| Cyclosporines A | Loratadine | Prostaglandin E | Rolipram |
| Tacrolimus | Amantadine | Prostaglandin E | RO-20-1724 |
| Dipyridamole | Amoxapine | Limaprost | RO-20-1724 |
| Dipyridamole | Sertraline | Limaprost | Ibudilast |
| Loratadine | Nortriptyline | Bithionol | Nicarbazin |
| Loratadine | Paroxetine | Dipyridamole | Rolipram |
| Amoxapine | Bufexamac | Triamcinolone | Ibudilast |
| Amoxapine | Loratadine | Triamcinolone | Bufexamac |
| Amoxapine | Nitrendipine | | |

**Table 1B**

| **Drug 1** | **Drug 2** | **Drug 1** | **Drug 2** |
|---|---|---|---|
| Dexamethasone | Cyclosporine A | Dexamethasone | Tacrolimus |
| Fluorometholone | Cyclosporine A | Fluorometholone | Tacrolimus |
| Hydrocortisone | Cyclosporine A | Hydrocortisone | Tacrolimus |
| Loteprednol Etabonate | Cyclosporine A | Medrysone | Tacrolimus |
| Medrysone | Cyclosporine A | Methylprednisolone | Tacrolimus |
| Methylprednisolone | Cyclosporine A | Prednisolone | Tacrolimus |
| Prednisolone | Cyclosporine A | Prednisolone Acetate | Tacrolimus |
| Prednisolone Acetate | Cyclosporine A | Triamcinolone | Tacrolimus |
| Rimexolone | Cyclosporine A | | |
| Triamcinolone | Cyclosporine A | | |

In the methods, compositions, and kits of the invention, drugs that are the same mechanistic, structural, or therapeutic class may be used in lieu of one or more of the the drugs listed on Table 1A or Table 1B. Exemplary classes are provided on Table 2.

**Table 2**

| **Class** | **Drug** |
|---|---|
| Alpha-2 adrenoceptor agonist | Brimonidine |
| | Apraclonidine |
| Anticoccidial compound | Nicarbazin |
| Antihistamine | Loratadine |
| | Epinastine |
| Antiinfective | Bithionol |
| Antiinflammatory | Bufexamac |
| Calcium channel blocker | Nitrendipine |
| Corticosteroid | Prednisolone |
| | Dexamethasone |
| | Fluorometholone |
| | Hydrocortisone |
| | Loteprednol Etabonate |
| | Medrysone Methylprednisolone |
| | Prednisolone Acetate |
| | Rimexolone |
| | Triamcinolone |
| Electron donor-acceptor molecular complex | Nicarbazin |
| NMDA antagonist/antidyskinetic | Amantadine |
| NsIDI | Cyclosporine A |
| | Tacrolimus |
| Phosphodiesterase inhibitor | Dipyridamole |
| | Ibudilast |
| | RO-20-1724 |
| | Rolipram |
| Prostaglandin | Prostaglandin E |
| | Misoprostol |
| | Limaprost |
| SSRI | Sertraline |
| | Paroxetine |
| Sympathomimetic | Dipivefrin |
| Tetra-substituted pyrimidopyrimidine | Dipyridamole |
| Tricyclic compound | Amoxapine |
| | Nortriptyline |

Thus, more broadly, the drug pairs of Table 3 may be used in the methods, compositions, and kits of the invention.

**Table 3**

| **Drug 1** | **Drug 2** |
|---|---|
| Antihistamine | Phosphodiesterase inhibitor |
| Antihistamine | SSRI |
| Antihistamine | Tricyclic compound |
| Antiinfective | Anticoccidial compound |
| Corticosteroid | Antihistamine |
| Corticosteroid | Phosphodiesterase inhibitor |
| Corticosteroid | Prostaglandin |
| NsIDI | Alpha-2 adrenoceptor agonist |
| NsIDI | Antihistamine |
| NsIDI | NMDA antagonist/antidyskinetic |
| NsIDI | Prostaglandin |
| NsIDI | Sympathomimetic |
| Prostaglandin | Phosphodiesterase inhibitor |
| Prostaglandin | Tetra-substituted pyrimidopyrimidine |
| Sympathomimetic | NMDA antagonist/antidyskinetic |
| Sympathomimetic | Prostaglandin |
| Sympathomimetic | Tetra-substituted pyrimidopyrimidine |
| Sympathomimetic | Tricyclic compound |
| Tetra-substituted pyrimidopyrimidine | Phosphodiesterase inhibitor |
| Tetra-substituted pyrimidopyrimidine | SSRI |
| Tetra-substituted pyrimidopyrimidine | Tricyclic compound |
| Tricyclic compound | Calcium channel blocker |

The classes are discussed in more detail below.

### Corticosteroids

In certain embodiments, a corticosteroid may be employed in a method, composition, or kit of the invention. Suitable corticosteroids include those from the class of selective glucocorticosteroid receptor agonists (SEGRAs), 11-alpha, 17-alpha,21-trihydroxypregn-4-ene-3,20-dione; 11-beta,16-alpha,17,21-tetrahydroxypregn-4-ene-3,20-dione; 11-beta, 16-alpha, 17,21-tetrahydroxypregn-1,4-diene-3,20-dione; 11-beta,17-alpha,21-trihydroxy-6-alpha-methylpregn-4-ene-3,20-dione; 11-dehydrocorticosterone; 11-deoxycortisol; 11-hydroxy-1,4-androstadiene-3,17-dione; 11-ketotestosterone; 14-hydroxyandrost-4-ene-3,6,17-trione; 15,17-dihydroxyprogesterone; 16-methylhydrocortisone; 17,21-dmydroxy-16-alpha-methylpregna-1,4,9(11)-triene-3,20-dione; 17-alpha-hydroxypregn-4-ene-3,20-dione; 17-alpha-hydroxypregnenolone; 17-hydroxy-16-beta-methyl-5-beta-pregn-9(11)-ene-3,20-dione; 17-hydroxy-4,6,8(14)-pregnatriene-3,20-dione; 17-hydroxypregna-4,9(11)-diene-3,20-dione; 18-hydroxycorticosterone; 18-hydroxycortisone; 18-oxocortisol; 21-acetoxypregnenolone; 21-deoxyaldosterone; 21-deoxycortisone; 2-deoxyecdysone; 2-methylcortisone; 3-dehydroecdysone; 4-pregnene-17-alpha,20-beta, 21-triol-3,11-dione; 6,17,20-trihydroxypregn-4-ene-3-one; 6-alpha-hydroxycortisol; 6-alpha-fluoroprednisolone, 6-alpha-methylprednisolone, 6-alpha-methylprednisolone 21-acetate, 6-alpha-methylprednisolone 21-hemisuccinate sodium salt, 6-beta-hydroxycortisol, 6-alpha, 9-alpha-difluoroprednisolone 21-acetate 17-butyrate, 6-hydroxycorticosterone; 6-hydroxydexamethasone; 6-hydroxyprednisolone; 9-fluorocortisone; alclomethasone dipropionate; aldosterone; algestone; alphaderm; amadinone; amcinonide; anagestone; androstenedione; anecortave acetate; beclomethasone; beclomethasone dipropionate; betamethasone 17-valerate; betamethasone sodium acetate; betamethasone sodium phosphate; betamethasone valerate; bolasterone; budesonide; calusterone; chlormadinone; chloroprednisone; chloroprednisone acetate; cholesterol; ciclesonide; clobetasol; clobetasol propionate; clobetasone; clocortolone; clocortolone pivalate; clogestone; cloprednol; corticosterone; cortisol; cortisol acetate; cortisol butyrate; cortisol cypionate; cortisol octanoate; cortisol sodium phosphate; cortisol sodium succinate; cortisol valerate; cortisone; cortisone acetate; cortivazol; cortodoxone; daturaolone; deflazacort, 21-deoxycortisol, dehydroepiandrosterone; delmadinone; deoxycorticosterone; deprodone; descinolone; desonide; desoximethasone; dexafen; dexamethasone; dexamethasone 21-acetate; dexamethasone acetate; dexamethasone sodium phosphate; dichlorisone; diflorasone; diflorasone diacetate; diflucortolone; difluprednate; dihydroelatericin a; domoprednate; doxibetasol; ecdysone; ecdysterone; emoxolone; endrysone; enoxolone; fluazacort; flucinolone; flucloronide; fludrocortisone; fludrocortisone acetate; flugestone; flumethasone; flumethasone pivalate; flumoxonide; flunisolide; fluocinolone; fluocinolone acetonide; fluocinonide; fluocortin butyl; 9-fluorocortisone; fluocortolone; fluorohydroxyandrostenedione; fluorometholone; fluorometholone acetate; fluoxymesterone; fluperolone acetate; fluprednidene; fluprednisolone; flurandrenolide; fluticasone; fluticasone propionate; formebolone; formestane; formocortal; gestonorone; glyderinine; halcinonide; halobetasol propionate; halometasone; halopredone; haloprogesterone; hydrocortamate; hydrocortiosone cypionate; hydrocortisone; hydrocortisone 21-butyrate; hydrocortisone aceponate; hydrocortisone acetate; hydrocortisone buteprate; hydrocortisone butyrate; hydrocortisone cypionate; hydrocortisone hemisuccinate; hydrocortisone probutate; hydrocortisone sodium phosphate; hydrocortisone sodium succinate; hydrocortisone valerate; hydroxyprogesterone; inokosterone; isoflupredone; isoflupredone acetate; isoprednidene; loteprednol etabonate; meclorisone; mecortolon; medrogestone; medroxyprogesterone; medrysone; megestrol; megestrol acetate; melengestrol; meprednisone; methandrostenolone; methylprednisolone; methylprednisolone aceponate; methylprednisolone acetate; methylprednisolone hemisuccinate; methylprednisolone sodium succinate; methyltestosterone; metribolone; mometasone; mometasone furoate; mometasone furoate monohydrate; nisone; nomegestrol; norgestomet; norvinisterone; oxymesterone; paramethasone; paramethasone acetate; ponasterone; prednicarbate; prednisolamate; prednisolone; prednisolone 21-diethylaminoacetate; prednisolone 21-hemisuccinate; prednisolone acetate; prednisolone farnesylate; prednisolone hemisuccinate; prednisolone-21(beta-D-glucuronide); prednisolone metasulphobenzoate; prednisolone sodium phosphate; prednisolone steaglate; prednisolone tebutate; prednisolone tetrahydrophthalate; prednisone; prednival; prednylidene; pregnenolone; procinonide; tralonide; progesterone; promegestone; rhapontisterone; rimexolone; roxibolone; rubrosterone; stizophyllin; tixocortol; topterone; triamcinolone; triamcinolone acetonide; triamcinolone acetonide 21-palmitate; triamcinolone benetonide; triamcinolone diacetate; triamcinolone hexacetonide; trimegestone; turkesterone; and wortmannin.

Standard recommended dosages for various steroid/disease combinations are provided in Table 4A and Table 4B, below.

**Table 4A-Standard Recommended Corticosteroid Dosages**

| Indication | Route | Drug | Dose | Schedule |
|---|---|---|---|---|
| Corticosteroid-responsive dermatoses | Topical | Clobetasol proprionate | 0.05% Lotion | Twice daily |
| | Topical | Betamethsone valerate | 0.1% Cream, Lotion | Twice daily |
| | Topical | Hydrocortisone acetate | 1% Gel | 2-4 times daily |
| | | | | |
| Psoriasis | oral | prednisolone | 7.5-60 mg | per day or divided b.i.d. |
| | oral | prednisone | 7.5-60 mg | per day or divided b.i.d |
| | | | | |
| Asthma | inhaled | beclomethasone dipropionate | 42 µg/puff) | 4-8 puffs b.i.d. |
| | inhaled | budesonide | (200 µg/inhalation) | 1-2 inhalations b.i.d. |
| | inhaled | flunisolide | (250 µg/puff) | 2-4 puffs b.i.d. |
| | inhaled | fluticasone propionate | (44, 110 or 220 µg/puff) | 2-4 puffs b.i.d. |
| | inhaled | triamcinolone acetonide | (100 µg/puff) | 2-4 puffs b.i.d. |
| | | | | |
| COPD | oral | prednisone | 30-40 mg | per day |
| | | | | |
| Crohn's disease | oral | budesonide | 9 mg | per day |
| | | | | |
| Ulcerative colitis | oral | prednisone | 40-60 mg | per day |
| | oral | hydrocortisone | 300 mg (IV) | per day |
| | oral | methylprednisolone | 40-60 mg | per day |
| | | | | |
| Rheumatoid arthritis | oral | prednisone | 10 mg | per day |

**Table 4B-Standard Recommended Corticosteroid Dosages for Ophthalmic Administration**

| **Ophthalmic corticosteroid** | **Lowest approved concentration for ophthalmic administration** | **Lowest standard recommended dosage** |
|---|---|---|
| Clocortolone Pivalate | 0.1 % | N/A |
| Hydrocortisone | 1.0 % | 0.5 µg / 3 times daily |
| Dexamethasone | 0.1 % | 0.05 µg / 4-6 times daily |
| Fluorometholone | 0.1 % | 0.05 µg / 2-11 times daily |
| Loteprednol Etabonate | 0.2 % | 0.1 µg / 4 times daily |
| Medrysone | 1.0 % | 0.5 µg / up to every 4 hours |
| Prednisolone Acetate | 0.12 % | 0.06 µg / 2-4 times daily |
| Rimexolone | 1.0 % | 0.5 µg / 4 times daily |

| | | |
|---|---|---|
| (N/A = Not Available) | | |

Other standard recommended dosages for corticosteroids are provided, e.g., in the Merck Manual of Diagnosis & Therapy (17th Ed. MH Beers et al., Merck & Co.) and Physicians' Desk Reference 2003 (57^{th} Ed. Medical Economics Staff et al., Medical Economics Co., 2002). In one embodiment, the dosage of corticosteroid administered is a dosage equivalent to a prednisolone dosage, as defined herein. For example, a low dosage of a corticosteroid may be considered as the dosage equivalent to a low dosage of prednisolone.

For ophthalmic administration, a corticosteroid can be administered at a concentration between 0.01% and 5% (e.g., 5.0%, 4.0%, 3.0%, 2.0%, 1.0%, 0.9%, 0.8%, 0.7%, 0.6%, 0.5%, 0.1%, 0.09%, 0.08%, 0.07%, 0.06%, 0.05%, and 0.01%).

Low concentrations of corticosteroids of the invention are 95% or less of the lowest approved concentration. For example, low concentration of corticosteroids of the invention can be 90%, 85%, 80%, 70%, 60%, 50%, 25%, 10%, 5%, 2%, 1%, 0.5% or 0.1% of the lowest approved concentration.

For ophthalmic administration for example, a low concentration of clocortolone pivalate is between 0.01% and 0.1% (e.g., 0.1%, 0.09%, 0.08%, 0.07%, 0.06%, 0.05%, and 0.01%), a low concentration of hydrocortisone is between 0.01% and 1.0% (e.g., 1.0%, 0.9%, 0.8%, 0.7%, 0.6%, 0.5%, 0.1%, 0.09%, 0.08%, 0.07%, 0.06%, 0.05%, and 0.01%), a low concentration of dexamethasone is between 0.01% and 0.1% (e.g., 0.1%, 0.09%, 0.08%, 0.07%, 0.06%, 0.05%, and 0.01%), a low concentration of fluorometholone is between 0.01% and 0.1% (e.g., 0.1%, 0.09%, 0.08%, 0.07%, 0.06%, 0.05%, and 0.01%), a low concentration of loteprednol etabonate is between 0.01% and 0.2% (e.g., 0.2%, 0.1%, 0.09%, 0.08%, 0.07%, 0.06%, 0.05%, and 0.01%), a low concentration of medrysone is between 0.01% and 1.0% (e.g., 1.0%, 0.9%, 0.8%, 0.7%, 0.6%, 0.5%, 0.1%, 0.09%, 0.08%, 0.07%, 0.06%, 0.05%, and 0.01%), a low concentration of rimexolone is between 0.01% and 1.0% (e.g., 1.0%, 0.9%, 0.8%, 0.7%, 0.6%, 0.5%, 0.1%, 0.09%, 0.08%, 0.07%, 0.06%, 0.05%, and 0.01%), and a low concentration ofprednisolone is between 0.01% and 0.12% (e.g., 0.12%, 0.1%, 0.09%, 0.08%, 0.07%, 0.06%, 0.05%, and 0.01%).

### Steroid receptor modulators

Steroid receptor modulators (e.g., antagonists and agonists) may be used as a substitute for or in addition to a corticosteroid in the methods, compositions, and kits of the invention. Glucocorticoid receptor modulators that may used in the methods, compositions, and kits of the invention include compounds described in U.S. Patent Nos. 6,380,207, 6,380,223, 6,448,405, 6,506,766, and 6,570,020, U.S. Patent Application Publication Nos. 2003/0176478, 2003/0171585, 200310120081, 200310073703, 2002/015631, 2002/4147336, 2002/0107235, 2002/0103217, and 2001/0041802, and PCT Publication No. WO00/66522, each of which is hereby incorporated by reference. Other steroid receptor modulators may also be used in the methods, compositions, and kits of the invention are described in U.S. Patent Nos. 6,093,821, 6,121,450, 5,994,544, 5,696,133, 5,696,127, 5,693,647, 5,693,646, 5,688,810, 5,688,808, and 5,696,130, each of which is hereby incorporated by reference.

### Other compounds

Other compounds that may be used in addition to the drug combination of Table 1A or Table 3 in the methods, compositions, and kits of the invention A-348441 (Karo Bio), adrenal cortex extract (GlaxoSmithKline), alsactide (Aventis), amebucort (Schering AG), amelometasone (Taisho), ATSA (Pfizer), bitolterol (Elan), CBP-2011 (InKine Pharmaceutical), cebaracetam (Novartis) CGP-13774 (Kissei), ciclesonide (Altana), ciclometasone (Aventis), clobetasone butyrate (GlaxoSmithKline), cloprednol (Hoffinann-La Roche), collismycin A (Kirin), cucurbitacin E (NIH), deflazacort (Aventis), deprodone propionate (SSP), dexamethasone acefurate (Schering-Plough), dexamethasone linoleate (GlaxoSmithKline), dexamethasone valerate (Abbott), difluprednate (Pfizer), domoprednate (Hoffinann-La Roche), ebiratide (Aventis), etiprednol dicloacetate (IVAX), fluazacort (Vicuron), flumoxonide (Hoffinann-La Roche), fluocortin butyl (Schering AG), fluocortolone monohydrate (Schering AG), GR-250495X (GlaxoSmithKline), halometasone (Novartis), halopredone (Dainippon), HYC-141 (Fidia), icomethasone enbutate (Hovione), itrocinonide (AstraZeneca), L-6485 (Vicuron), Lipocort (Draxis Health), locicortone (Aventis), meclorisone (Schering-Plough), naflocort (Bristol-Myers Squibb), NCX-1015 (NicOx), NCX-1020 (NicOx), NCX-1022 (NicOx), nicocortonide (Yamanouchi), NIK-236 (Nikken Chemicals), NS-126 (SSP), Org-2766 (Akzo Nobel), Org-6632 (Akzo Nobel), P16CM, propylmesterolone (Schering AG), RGH-1113 (Gedeon Richter), rofleponide (AstraZeneca), rofleponide palmitate (AstraZeneca), RPR-106541 (Aventis), RU-26559 (Aventis), Sch-19457 (Schering-Plough), T25 (Matrix Therapeutics), TBI-PAB (Sigma-Tau), ticabesone propionate (Hoffmann-La Roche), tifluadom (Solvay), timobesone (Hoffmann-La Roche), TSC-5 (Takeda), ZK-73634 (Schering AG), antibiotics (minocycline, penicillin, cephalosporin, tetracycline, oxytetracycline, chlortetracycline, metronidazole, chloramphenicol, streptomycin, neomycin, sulfonamides, phenolic compounds, quarternary ammonium compounds, doxycycline), antiseptics (e.g., chlorhexidine), tranexamic acid, allantoin, epsilon-aminocaproic acid, lysozyme, dihydrocholesterol, beta-glycyrrhetinic acid, platelet aggregation inhibitors (e.g., abciximab, aspirin, cilostazol, clopidogrel, dipyridamole, eptifibatide, ticlopidine, or tirofiban), anticoagulants (e.g., dalteparin, danaparoid, enoxaparin, heparin, tinzaparin, or warfarin), antipyretics (e.g., acetaminophen), ticlopidine, clopidogrel, angiotensin converting enzyme inhibitors, beta blockers, pentoxifylline, cilostazol, estrogen replacement therapy, and lipid-lowering agents (e.g., cholestyramine, colestipol, nicotinic acid, gemfibrozil, probucol, ezetimibe, or statins such as atorvastatin, rosuvastatin, lovastatin simvastatin, pravastatin, cerivastatin, and fluvastatin). These agents may be administered concommittantly or within 14 days of the method of the invention. If desired, one or more of the foregoing agents is coformulated with one or more agents of the invention to form a single composition.

### Non-steroidal immunophilin-dependent immunosuppressants

In one embodiment, the invention features methods, compositions, and kits employing a non-steroidal immunophilin-dependent immunosuppressant (NsIDI).

In healthy individuals the immune system uses cellular effectors, such as B-cells and T-cells, to target infectious microbes and abnormal cell types while leaving normal cells intact. In individuals with an autoimmune disorder or a transplanted organ, activated T-cells damage healthy tissues. Calcineurin inhibitors (e.g., cyclosporines, tacrolimus, pimecrolimus, ABT-281, ISAtx247), and rapamycin target many types of immunoregulatory cells, including T-cells, and suppress the immune response in organ transplantation and autoimmune disorders.

In one embodiment, the NsIDI is cyclosporine A, and is administered in an amount between 0.05 and 50 milligrams per kilogram per day (e.g., orally in an amount between 0.1 and 12 milligrams per kilogram per day). In another embodiment, the NsIDI is cyclosporine and is administered as a 0.05% ophthalmic emulsion twice per day. In another embodiment, the NsIDI is tacrolimus and is administered in an amount between 0.0001-20 milligrams per kilogram per day (e.g., orally in an amount between 0.01-0.2 milligrams per kilogram per day). In another embodiment, the NsIDI is tacrolimus and is administered as a 0.02% ophthalmic suspension. In another embodiment, the NsIDI is rapamycin and is administered in an amount between 0.1-502 milligrams per day (e.g., at a single loading dose of 6 mg/day, followed by a 2 mg/day maintenance dose). In another embodiment, the NsIDI is everolimus, administered at a dosage of 0.75-8 mg/day. In still other embodiments, the NsIDI is pimecrolimus, administered in an amount between 0.1 and 200 milligrams per day (e.g., as a 1% cream/twice a day to treat atopic dermatitis or 60 mg a day for the treatment of psoriasis), or the NsIDI is a calcineurin-binding peptide administered in an amount and frequency sufficient to treat the patient. Two or more NsIDIs can be administered contemporaneously.

### Cyclosporines

The cyclosporines are fungal metabolites that comprise a class of cyclic oligopeptides that act as immunosuppressants. Cyclosporine A is a hydrophobic cyclic polypeptide consisting of eleven amino acids. It binds and forms a complex with the intracellular receptor cyclophilin. The cyclosporine/cyclophilin complex binds to and inhibits calcineurin, a Ca²⁺-calmodulin-dependent serine-threonine-specific protein phosphatase. Calcineurin mediates signal transduction events required for T-cell activation (reviewed in Schreiber et al., Cell 70:365-365, 1991). Cyclosporines and their functional and structural analogs suppress the T cell-dependent immune response by inhibiting antigen-triggered signal transduction. This inhibition decreases the expression of proinflammatory cytokines, such as IL-2.

Many different cyclosporines (e.g., cyclosporine A, B, C, D, E, F, G, H, and I) are produced by fungi. Cyclosporine A is a commercially available under the trade name NEURAL from Novartis. Cyclosporine A structural and functional analogs include cyclosporines having one or more fluorinated amino acids (described, e.g., in U.S. Patent No. 5,227,467); cyclosporines having modified amino acids (described, e.g., in U.S. Patent Nos. 5,122,511 and 4,798,823); and deuterated cyclosporines, such as ISAtx247 (described in U.S. Patent Application Publication No. 2002/0132763 A1). Additional cyclosporine analogs are described in U.S. Patent Nos. 6,136,357,4,384,996, 5,284,826, and 5,709,797. Cyclosporine analogs include, but are not limited to, D-Sar (α-SMe)³ Val²-DH-Cs (209-825), Allo-Tbr-2-Cs, Norvaline-2-Cs, D-Ala(3-acetylamino)-8-Cs, Tbr-2-Cs, and D-MeSer-3-Cs, D-Ser(O-CH₂CH₂-OH)-8-Cs, and D-Ser-8-Cs, which are described in Cruz et al. (Antimicrob. Agents Chemother. 44:143-149,2000).

Cyclosporines are highly hydrophobic and readily precipitate in the presence of water (e.g. on contact with body fluids). Methods of providing cyclosporine formulations with improved bioavailability are described in U.S. Patent Nos. 4,388,307, 6,468,968, 5,051,402, 5,342,625, 5,977,066, and 6,022,852. Cyclosporine microemulsion compositions are described in U.S. Patent Nos. 5,866,159, 5,916,589, 5,962,014, 5,962,017, 6,007,840, and 6,024,978.

Cyclosporines can be administered either intravenously or orally, but oral administration is preferred. To overcome the hydrophobicity of cyclosporine A, an intravenous cyclosporine A may be provided in an ethanol-polyoxyethylated castor oil vehicle that must be diluted prior to administration. Cyclosporine A may be provided, e.g., as a microemulsion in a 25 mg or 100 mg tablets, in a 100 mg/ml oral solution (NEORAL), or in a 0.05% ophthalmic emulsion.

Typically, patient dosage of an oral cyclosporine varies according to the patient's condition, but some standard recommended dosages are provided herein. Patients undergoing organ transplant typically receive an initial dose of oral cyclosporine A in amounts between 12 and 15 mg/kg/day. Dosage is then gradually decreased by 5% per week until a 7-12 mg/kg/day maintenance dose is reached. For intravenous administration 2-6 mg/kg/day is preferred for most patients. For patients diagnosed as having Crohn's disease or ulcerative colitis, dosage amounts from 6-8 mg/kg/day are generally given. For patients diagnosed as having systemic lupus erythematosus, dosage amounts from 2.2-6.0 mg/kg/day are generally given. For psoriasis or rheumatoid arthritis, dosage amounts from 0.5-4 mg/kg/day are typical. A suggested dosing schedule is shown in Table 5. Other useful dosages include 0.5-5 mg/kg/day, 5-10 mg/kg/day, 10-15 mg/kg/day, 15-20 mg/kg/day, or 20-25 mg/kg/day.

**Table 5**

| **Compound** | **Atopic Dermatitis** | **Psoriasis** | **RA** | **Crohn's** | **UC** | **Transplant** | **SLE** |
|---|---|---|---|---|---|---|---|
| **CsA (NEORAL)** | N/A | 0.5-4 mg/kg/day | 0.5-4 mg/kg/day | 6-8 mg/kg/day (oral-fistulizing) | 6-8 mg/kg/day (oral) | ∼7-12 mg/kg/day | 2.2-6.0 mg/kg/day |
| **Tacrolimus** | 0.03-0.1% cream/twice day (30 and 60 gram tubes) | 0.05-1.15 mg/kg/day (oral) | 1-3 mg/day (oral) | 0.1-0.2 mg/kg/day (oral) | 0.1-02 mg/kg/day (oral) | 0.1-0.2 mg/kg/day (oral) | N/A |
| **Pimecrolimus** | 1% cream/twice day (15, 30, 100 gram tubes) | 40-60 mg/day (oral) | 40-60 mg/day (oral) | 80-160 mg/day (oral) | 160-240 mg/day (oral) | 40-120 mg/day (oral) | 40-120 mg/day (oral) |

| | | | | | | | |
|---|---|---|---|---|---|---|---|
| CsA=cyclosporine A RA=rheumatoid arthritis UC=ulcerative colitis SLE=systemic lupus erythamatosus | | | | | | | |

The lowest approved ophthalmic concentration of cyclosporine A is 0.05%. Low concentrations of cyclosporine A are 0.04%, or more preferably 0.03%, 0.02%, 0.01%, 0.008%, 0.005%, or 0.001%. The lowest standard recommended ophthalmic dosage of cyclosporine A is 0.2µg twice daily.

### Tacrolimus

Tacrolimus (FK506) is an immunosuppressive agent that targets T cell intracellular signal transduction pathways. Tacrolimus binds to an intracellular protein FK506 binding protein (FKBP-12) that is not structurally related to cyclophilin. The FKBP/FK506 complex binds to calcineurin and inhibits calcineurin's phosphatase activity. This inhibition prevents the dephosphorylation and nuclear translocation of nuclear factor of activated T cells (NFAT), a nuclear component that initiates gene transcription required for proinflammatory cytokine (e.g., IL-2, gamma interferon) production and T cell activation. Thus, tacrolimus inhibits T cell activation.

Tacrolimus is a macrolide antibiotic that is produced by Streptomyces tsukubaensis. It suppresses the immune system and prolongs the survival of transplanted organs. It is currently available in oral and injectable formulations. Tacrolimus capsules contain 0.5 mg, 1 mg, or 5 mg of anhydrous tacrolimus within a gelatin capsule shell. The injectable formulation contains 5 mg anhydrous tacrolimus in castor oil and alcohol that is diluted with 0.9% sodium chloride or 5% dextrose prior to injection. While oral administration is preferred, patients unable to take oral capsules may receive injectable tacrolimus. The initial dose should be administered no sooner than six hours after transplant by continuous intravenous infusion.

Tacrolimus and tacrolimus analogs are described by Tanaka et al., (J. Am. Chem. Soc., 109:5031, 1987) and in U.S. Patent Nos. 4,894,366, 4,929,611, and 4,956,352. FK506-related compounds, including FR-900520, FR-900523, and FR-900525, are described in U.S. Patent No. 5,254,562; O-aryl, O-alkyl, O-alkenyl, and O-alkynylmacrolides are described in U.S. Patent Nos. 5,250,678, 532,248, 5,693,648; amino O-aryl macrolides are described in U.S. Patent No. 5,262,533; alkylidene macrolides are described in U.S. Patent No. 5,284,840; N-heteroaryl, N-alkylheteroaryl, N-alkenylheteroaryl, and N-alkynylheteroaryl macrolides are described in U.S. Patent No. 5,208,241; aminomacrolides and derivatives thereof are described in U.S. Patent No. 5,208,228; fluoromacrolides are described in U.S. Patent No. 5,189,042; amino O-alkyl, O-alkenyl, and O-alkynylmacrolides are described in U.S. Patent No. 5,162,334; and halomacrolides are described in U.S. Patent No. 5,143,918.

While suggested dosages will vary with a patient's condition, standard recommended dosages are provided below. Typically patients diagnosed as having Crohn's disease or ulcerative colitis are administered 0.1-0.2 mg/kg/day oral tacrolimus. Patients having a transplanted organ typically receive doses of 0.1-0.2 mg/kg/day of oral tacrolimus. Patients being treated for rheumatoid arthritis typically receive 1-3 mg/day oral tacrolimus. For the treatment of psoriasis, 0.01-0.15 mg/kg/day of oral tacrolimus is administered to a patient. Atopic dermatitis can be treated twice a day by applying a cream having 0.03-0.1% tacrolimus to the affected area. Patients receiving oral tacrolimus capsules typically receive the first dose no sooner than six hours after transplant, or eight to twelve hours after intravenous tacrolimus infusion was discontinued. Other suggested tacrolimus dosages include 0.005-0.01 mg/kg/day, 0.01-0.03 mg/kg/day, 0.03-0.05 mg/kg/day, 0.05-0.07 mg/kg/day, 0.07-0.10 mg/kg/day, 0.10-0.25 mg/kg/day, or 0.25-0.5 mg/kg/day.

Tacrolimus is extensively metabolized by the mixed-function oxidase system, in particular, by the cytochrome P-450 system. The primary mechanism of metabolism is demethylation and hydroxylation. While various tacrolimus metabolites are likely to exhibit immunosuppressive biological activity, the 13-demethyl metabolite is reported to have the same activity as tacrolimus.

### Pimecrolimus

Pimecrolimus is the 33-epi-chloro derivative of the macrolactam ascomyin. Pimecrolimus structural and functional analogs are described in U.S. Patent No. 6,384,073. Pimecrolimus is particularly useful for the treatment of atopic dermatitis. Pimecrolimus is currently available as a 1% cream. Suggested dosing schedule for pimecrolimus is shown at Table 5. While individual dosing will vary with the patient's condition, some standard recommended dosages are provided below. Oral pimecrolimus can be given for the treatment of psoriasis or rheumatoid arthritis in amounts of 40-60 mg/day. For the treatment of Crohn's disease or ulcerative colitis amounts of 80-160 mg/day pimecrolimus can be given. Patients having an organ transplant can be administered 160-240 mg/day of pimecrolimus. Patients diagnosed as having systemic lupus erythamatosus can be administered 40-120 mg/day of pimecrolimus. Other useful dosages of pimecrolimus include 0.5-5 mg/day, 5-10 mg/day, 10-30 mg/day, 40-80 mg/day, 80-120 mg/day, or even 120-200 mg/day.

### Rapamycin

Rapamycin is a cyclic lactone produced by Streptomyces hygroscopicus. Rapamycin is an immunosuppressive agent that inhibits T cell activation and proliferation. Like cyclosporines and tacrolimus, rapamycin forms a complex with the immunophilin FKBP-12, but the rapamycin-FKBP-12 complex does not inhibit calcineurin phosphatase activity. The rapamycin immunophilin complex binds to and inhibits the mammalian kinase target of rapamycin (mTOR). mTOR is a kinase that is required for cell-cycle progression. Inhibition of mTOR kinase activity blocks T cell activation and proinflammatory cytokine secretion.

Rapamycin structural and functional analogs include mono- and diacylated rapamycin derivatives (U.S. Patent No. 4,316,885); rapamycin water-soluble prodrugs (U.S. Patent No. 4,650,803); carboxylic acid esters (PCT Publication No. WO 92/05179); carbamates (U.S. Patent No. 5,118,678); amide esters (U.S. Patent No. 5,118,678); biotin esters (U.S. Patent No. 5,504,091); fluorinated esters (U.S. Patent No. 5,100,883); acetals (U.S. Patent No. 5,151,413); silyl ethers (U.S. Patent No. 5,120,842); bicyclic derivatives (U.S. Patent No. 5,120,725); rapamycin dimers (U.S. Patent No. 5,120,727); O-aryl, O-alkyl, O-alkyenyl and O-alkynyl derivatives (U.S. Patent No. 5,258,389); and deuterated rapamycin (U.S. Patent No. 6,503,921). Additional rapamycin analogs are described in U.S. Patent Nos. 5,202,332 and 5,169,851.

Rapamycin is currently available for oral administration in liquid and tablet formulations. RAPAMUNE liquid contains 1 mg/mL rapamycin that is diluted in water or orange juice prior to administration. Tablets containing 1 or 2 mg of rapamycin are also available. Rapamycin is preferably given once daily as soon as possible after transplantation. It is absorbed rapidly and completely after oral administration. Typically, patient dosage of rapamycin varies according to the patient's condition, but some standard recommended dosages are provided below. The initial loading dose for rapamycin is 6 mg. Subsequent maintenance doses of 0.5-2 mg/day are typical. Alternatively, a loading dose, of 3 mg, 5 mg, 10 mg, 15 mg, 20 mg, or 25 mg can be used with a 1 mg, 3 mg, 5 mg, 7 mg, or 10 mg per day maintenance dose. In patients weighing less than 40 kg, rapamycin dosages are typically adjusted based on body surface area; generally a 3 mg/m²/day loading dose and a 1 mg/m²/day maintenance dose is used.

### Peptide moieties

Peptides, peptide mimetics, peptide fragments, either natural, synthetic or chemically modified, that impair the calcineurin-mediated dephosphorylation and nuclear translocation of NFAT are suitable for use in practicing the invention. Examples of peptides that act as calcineurin inhibitors by inhibiting the NFAT activation and the NFAT transcription factor are described, e.g., by Aramaburu et al., Science 285:2129-2133, 1999) and Aramburu et al., Mol. Cell 1:627-637, 1998). As a class of calcineurin inhibitors, these agents are useful in the methods, compositions, and kits of the invention.

### Phosphodiesterase inhibitors

In certain embodiments, a phosphodiesterase inhibitor may be employed in the methods, compositions, and kits of the invention. Suitable phosphodiesterase inhibitors include inhibitors of the type III phosphodiesterases (cAMP-specific-cGMP inhibitable form), the type IV phosphodiesterases (high affinity-high specificity cAMP form) and the type V phosphodiesterases (the cGMP specific form). Examples of type III phosphodiesterase inhibitors include bipyridines such as mihinone and amrinone, imidazolones such as piroximone and enoximone, dihydropyridazinones such as imazodan, 5-methylimazodan, indolidan and ICI118233 (6-(p-(3-methylureido)phenyl)-3(2H)-pyridazinone), quinolinone compounds such as cilostamide, cilostazol and vesnarinone, and other compounds such as bemoradan, anergrelide, siguazodan, trequensin, pimobendan, SKF-94120 (5-(4-acetamidophenyl)pyrazin-2-(1H)-one), SKF-95654, lixazinone and isomazole. Examples of type IV phosphodiesterase inhibitors include rolipram and rolipram derivatives such as RO-20-1724 (4-(3-butyloxy-4-methoxyphenyl)-imidazolidinone), nitraquazone and nitraquazone derivatives such as CP-77059 (1-(carbomethoxyphenyl)-3-benzylpyrido[2,3d] pyrimidine-2,4(1H,3H)dione), and RS-25344-00 (1-(3-nitrophenyl)-3-(4-pyridylmethyl)-1,2,3,4-tetrahydro pyrido(2,3-d) pyrimidine-2,4-dione)), xanthine derivatives such as denbufylline and ICI63197, and miscellaneous other compounds such as EMD54622 (5-[1-(3,4-dimethoxybenzoyl)-4,4-dimethyl-1,2,3,4-tetrahydrochinolin-6-yl] -6-methyl-3,6-dihydro-1,3,4-thiadiazin-2-one), LAS-31025 (1-propyl-3-(4-chlorophenyl) xanthine; also referred to as arofylline) and etazolate. Examples of type V phosphodiesterase inhibitors include zaprinast, MY5445 (N-(3-chlorophenyl)-4-phenyl-1-phthalazinamine), dipyridamole, and sildenafil. Sildenafil (5-[2-ethoxy-5-(4-methyl-1-piperazinylsulfonyl)-phenyl]-1-methyl-3-n-propy 1-1,6-dihydro-7H-pyrazolo[4,3-d]pyrimidin-7-one) and other suitable type V phosphodiesterase inhibitors are disclosed in PCT Publication Nos. WO 94/28902 and WO 96/16644 (e.g., 5-(2-ethoxy-5-morpholinoacetyl-phenyl)-1-methyl-3-n-propyl-1,6-dihydro-7H- pyrazolo[4,3-d]pyrimidin-7-one, 5-(5-morpholino-acetyl-2-n-propoxyphenyl)-1-methyl-3-n-propyl-1,6-dihydro- 7H-pyrazolo[4,3-d]pyrimidin-7-one, 5-[2-ethoxy-5-(4-methyl-1-piperazinyisulfonyl)-phenyl]-1-methyl-3-n-propyl -1,6-dihydro-7H-pyrazolo[4,3-d]pyrimidin-7-one, 5-[2-allyloxy-5-(4-methyl-1-piperazinylsulfonyl)-phenyl]-1-methyl-3-n-prop yl-1,6-dihydro-7H-pyrazolo[4,3-d]pyrimidin-7-one, 5-[2-ethoxy-5-[4-(2-propyl)-1-piperazinylsulfonyl)-phenyl]-1-methyl-3-n-propyl-1,6-dihydro-7H-pyrazolo[4,3-d]pyrimidin-7-one, 5-[2-ethoxy-5-[4-(2-hydroxyethyl)-1-piperazinylsulfonyl)phenyl]-1-methyl-3 -n-propyl-1,6-dihydro-7H-pyrazolo[4,3-d]pyrimidin-7-one, 5-[5-[4-(2-hydroxyethyl)-1-piperazinylsulfonyl]-2-n-propoxyphenyl]-1-methyl-3-n-propyl-1,6-dihydro-7H-pyrazolo[4,3-d]pyrimidin-7-one, 5-[2-ethoxy-5-(4-methyl-1-piperazinylcarbonyl)phenyl]-1-methyl-3-n-propyl- 1,6-dihydro-7H-pyrazolo[4,3-d]pyrimidin-7-one, 5-[2-ethoxy-5-(1-methyl-2-imidazolyl)phenyl]-1-methyl-3-n-propyl-1,6-dihydro-7H-pyrazolo[4,3-d]pyrimidin-7-one, 1,3-dimethyl-5-benzylpyrazolo[4,3-d]pyrimidine-7-one, 2-(2-propoxyphenyl)-6-purinone, 6-(2-propoxyphenyl)-1,2-dihydro-2-oxypyridine-3-carboxamide, 2-(2-propoxyphenyl)-pyrido[2,3-d]pyrimid-4(3H)-one, 7-methylthio-4-oxo-2-(2-propoxyphenyl)-3,4-dihydro-pyrimido[4,5-d]pyrimidine, 6-hydroxy-2(2-propoxyphenyl)-pyrimidine-4-carboxamide, 1-ethyl-3-methylimidazo[1,5a]quinoxalin-4(5H)-one, 4-phenylmethylamino-6-chloro-2-(1-imidazoloyl)quinazoline, 5-ethyl-8-[3-(N-cyclohexyl-N-methyl-carbamoyl)-propyloxy]-4,5-dihydro-4-oxo-pyrido[3,2-e]-pyrrolo[1,2-a]pyrazine, 5'-methyl-3'-(phenybnethyl)-spiro[cyclopentane-1,7'(8'H)-(3'H)-imidazo[2,1-b]purin]4'(5H)-one, 1-[6-chloro-4-(3,4-methylenedioxybenzyl)-aminoquinazolin-2-yl)piperidine-4 -carboxylic acid, (6R, 9S)-2-(4-trifluoromethyl-phenyl)methyl-5-methyl-3,4,5,6a,7,8,9,9a-octahydr ocyclopent[4,5]-imidazo[2,1-b]-purin-4-one, 1-t-butyl-3-phenylmethyl-6-(4-pyridyl)pyrazolo[3,4-d]-pyrimid-4-one, 1-cyclopentyl-3-methyl-6-(4-pyridyl)-4,5-dihydro-1H-pyrazolo[3,4-d]pyrimid-4-one, 2-butyl-1-(2-chlorobenzyl)6-ethoxy-carbonylbenzimidaole, and 2-(4-carboxypiperidino)-4-(3,4-methylene-dioxybenzyl)amino-6-nitroquinazol ine, and 2-phenyl-8-ethoxycycloheptimidazole. Still other type V phosphodiesterase inhibitors useful in conjunction with the present invention are IC-351 (ICOS), also referred to as tadalafil, 4-bromo-5-(pyriaylmethylamino)-6-[3-(4-chlorophenyl)-propoxy]-3(2H)pyridaz inone, 1-[4-[(1,3-benzodioxol-5-ylmethyl)amiono]-6-chloro-2-quinazolinyl]-4-piper idine-carboxylic acid, monosodium salt, (+)-cis-5,6a,7,9,9,9a-hexahydro-2-[4-(trifluoromethyl)-phenylmethyl-5-meth yl-cyclopent-4,5]imidazo[2,1-b]purin-4(3H)one, furazlocillin, cis-2-hexyl-5-methyl-3,4,5,6a,7,8,9,9a-octahydrocyclopent[4,5]imidazo[2,1-b]purin-4-one, 3-acetyl-1-(2-chlorobenzyl)-2-propylindole-6-carboxylate, 4-bromo-5-(3-pyridylmethylamino)-6-(3-(4-chlorophenyl)-propoxy)-3-(2H)pyridazinone, 1-methyl-5-(5-morpholinoacetyl-2-n-propoxyphenyl)-3-n-propyl-1,6-dihydro-7 H-pyrazolo(4,3-d)pyrimidin-7-one, 1-[4-[(1,3-benzodioxol-5-ylmethyl)amino]-6-chloro-2-quinazolinyl]-4-piperi dinecarboxylic acid, monosodium salt, Pharmaprojects No. 4516 (Glaxo Wellcome) Pharmaprojects No. 5051 (Bayer), Pharmaprojects No. 5064 (Kyowa Hakko; see WO 96/26940) Pharmaprojects No. 5069 (Schering Plough), and Sch-51866. Other phosphodiesterase inhibitors are disclosed in U.S. Patent No. 6,469,016.

Other phosphodiesterase inhibitors that may be used in the present invention include filaminast, piclamilast, Org 20241, MCI-154, roffumilast, toborinone, posicar, pyrazolopyrimidinones (such as those disclosed in WO 98/49166), motapizone, pimobendan, zardaverine, siguazodan, CI 930, EMD 53998, imazodan, saterinone, loprinone hydrochloride, 3-pyridinecarbonitrile derivatives, albifylline, torbafylline, doxofylline, theophylline, pentoxofylline, nanterinone, cilostazol, cilostamide, MS 857, piroximone, milrinone, amrinone, tolafentrine, papaverine, E4021, thienopyrimidine derivatives (such as those disclosed in PCT Publication No. WO 98/17668), triflusal, tetrahydropiperazino[1,2-b]beta-carboline-1,4-dione derivatives (such as those disclosed in U.S. Patent No. 5,859,006 and PCT Publication Nos. WO 97/03985 and WO 97/03675), carboline derivatives, (such as those disclosed in PCT Publication No. WO 97/43287), 2-pyrazolin-5-one derivatives (such as those disclosed in U.S. Patent No. 5,869,516), fused pyridazine derivatives (such as those disclosed in U.S. Patent No. 5,849,741), quinazoline derivatives (such as those disclosed in U.S. Patent No. 5,614,627), anthranilic acid derivatives (such as those disclosed in U.S. Patent No. 5,714,993), and imidazoquinazoline derivatives (such as those disclosed in PCT Publication No. WO 96/26940). Also included are phosphodiesterase inhibitors disclosed in U.S. Patent Nos. 3,850,941, 4,097,483, 4,193,926, 4,578,392, 4,925,849, 4,994,453, and 5,296,490, and PCT Publication Nos. WO 99/21562, and WO 99/30697.

### Alpha-2-adrenergic agonists

Alpha-2-adrenergic agonists can be used in the methods, compositions, and kits of the invention. An exemplary alpha-2-adrenergic agonist is brimonidine (5-bromo-N-(4,5-dihydro-1H-imidazil-2-yl)-6-quinoxalinamine), described in U.S. Patent No. 3,890,319. Other alpha-2-adrenergic agonists that can be used in the methods, compositions, and kits of the invention include apraclonidine, clonidine, dexmedetomidine, guanabenz, guanfacine, medetomidine, methyldopa, oxymetazoline, tizanidine, and (±)-(R,S)-5,6-diisobutyryloxy-2-methylaminotetralin. Other alpha-2-adrenergic agonists are described in U.S. Patent Nos. 2,868,818; 3,158,648; 3,202,660; 3,632,645; 3,843,668; 3,890,319; 4,029,792; 4,486,432; 4,517,199; 4,576,954; 4,910,214; 5,021,410, 5,037,829; 5,077,292, 5,091,528; 5,112,822; 5,130,441; 5,180,721; 5,198,442; 5,204,347; 5,215,991; 5,231,096; 5,237,072; 5,252,595; 5,281,591; 5,300,504; 5,326,763; 5,373,010; 5,418,234; 5,478,858; 5,541,210; 5,552,403; 5,561,132; 5,576,437; 5,578,607; 5,580,892; 5,587,376; 5,684,156; 5,691,370; 5,703,077; 5,708,015; 5,714,966; 5,739,148; 5,756,503; 5,773,440; 5,804,587; 5,834,470; 5,856,329; 5,914,342; 5,916,900; 5,965,595; 6,066,740; 6,110,952; 6,117,871; 6,162,818; 6,172,095; 6,194,415; 6,225,331; 6,242,442; 6,248,741; 6,294,563; 6,306,877; 6,316,441; 6,316,637; 6,323,204; 6,391,878; 6,395,764; 6,403,626; 6,423,724; 6,436,978; 6,436,982; 6,465,464; 6,486,190; 6,495,583; 6,562,873; 6,627,210; 6,641,834; 6,673,337; and 6,953,813.

For ophthalmic uses, brimonidine is available as brimonidine tartrate ophthalmic solution 0.2%.

### Prostaglandins

Prostaglandins may be used in the methods, compostions, and kits of the invention. Prostaglandins include alprostidil, dinoprostone, misoprostil, limaprost, bimatoprost, travoprost, unoprostone, latanoprost, prostaglandin E2, prostaglandin A1, prostaglandin A2, prostaglandin B1, prostaglandin B2, prostaglandin D2, prostaglandin F1α, prostaglandin F2α, prostaglandin I1, prostaglandin-ici 74205, prostaglandin F2β, 6-keto-prostaglandin F1α, prostaglandin E1 ethyl ester, prostaglandin E1 methyl ester, prostaglandin F2 methyl ester, arbaprostil, ornoprostil, 13,14-dihydroprostaglandin F2α, and prostaglandin J. Ophthalmic formulations of prostaglandins include bimatoprost 0.03%, travoprost 0.004%, unoprostone 0.15%, and latanoprost 0.005%.

### Tetra-substituted pyrimidopyrimidines

In certain embodiments, a tetra-substituted pyrimidopyrimidine or an adenosine activity upregulator can be used in combination with a second agent in the methods, compositions, and kits of the invention. By "tetra-substituted pyrimidopyrimidine" is meant a compound having the formula (V): 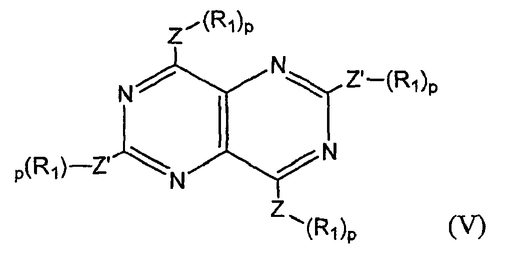 wherein each Z and each Z' is, independently, N, O, C, 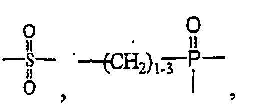 or

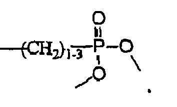

When Z or Z' is O or 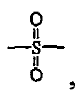 then p=1, when Z or Z' is N, 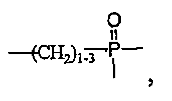 or 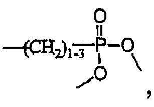 then p=2, and when Z or Z' is C, then p=3. In formula (V), each R₁ is, independently, X, OR, N-alkyl (wherein the alkyl group has 1 to 20, more preferably 1-5, carbon atoms); a branched or unbranched alkyl group having 1 to 20, more preferably 1-5, carbon atoms; or a heterocycle, preferably as defined in formula (V). Alternatively, when p>1, two R₁ groups from a common Z or Z' atom, in combination with each other, may represent -{CY₂)ₖ- in which k is an integer between 4 and 6, inclusive. Each X is, independently, Y, CY₃, C(CY₃)₃, CY₂CY₃, (CY₂)₁₋₅OY, substituted or unsubstituted cycloalkane of the structure CₙY₂ₙ₋₁ wherein n= 3-7, inclusive. Each Y is, independently, H, F, Cl, Br, or I. In one embodiment, each Z is the same moiety, each Z' is the same moiety, and Z and Z' are different moieties.

By "adenosine activity upregulator" is meant adenosine and any compounds that mimic or potentiate the physiological effects of adenosine, such as adenosine receptor agonists, adenosine transport inhibitors, adenosine kinase inhibitors, and phosphodiesterase (PDE) inhibitors, as described herein.

### Adenosine receptor agonists

Adenosine receptor agonists include adenosine hemisulfate salt, adenosine amine congener solid, N⁶-(4-amino-3-iodophenyl)methyl-5'-N-methylcarboxamidoadenosine (I-AB-MECA); N-((2-methylphenyl)methyl)adenosine (Metrifudil); 2-(1-hexynyl)-N-methyladenosine (HEMADO); N-(1-methyl-2-phenylethyl)adenosine (R-PIA); N⁶-(R-4-hydroxyphenylisopropyl) adenosine (HPIA); N⁶-cyclopentyladenosine (CPA); N⁶-cyclopentyl-2-(3-phenylaminocarbonyltriazene-1-yl)adenosine (TCPA); N-((1S,trans)-2-hydroxycyclopentyl)adenosine (GR 79236); N⁶-cyclohexyladenosine (CHA); 2-chloro-N⁶-cyclopentyladenosine (CCPA); N-ethylcarboxamidoadenosine (NECA); 2-(4-(2-carboxyethyl)phenethylamino)-5'-N-ethylcarboxamidoadenosine (CGS 21680); N⁶-(3-iodobenzyl)-5'-N-methylcarboxamidoadenosine (IB-MECA); 2-(cyclohexylmethylidene hydrazino)adenosine (WRC 0470); 2-(4-(2-carboxyethyl)phenethylamino)-5'-N-ethylcarboxamidoadenosine (CGS 21680); N⁶-(2-(3,5-dimethoxyphenyl)-2-(2-methylphenyl)ethyl)adenosine (DPMA); hexynyladenosine-5'-N-ethylcarboxamide (HE-NECA); 2-[(2-aminoethyl-aminocarbonylethyl) phenylethylamino]-5'-N-ethyl- carboxamidoadenosine (APEC) ; 2-chloro-N⁶-(3-iodobenzyl)-5'-N-methylcarboxamidoadenosine (2-Cl-IB-NMCA); 2-phenylaminoadenosine (CV 1808); 3'-Aminoadenosine-5'-uronamides; CV Therapuetics^{™} small molecule drugs Tecadenoson (CVT-510); Regadenoson (CVT 3146); and Carisa (CVT 3033); and Aderis Pharmaceuticals^{™} small drug molecules 2-[2-(4-chlorophenyl)ethoxy]adenosine (MRE 0094), 1-deoxy-1-[6-[[(iodophenyl)methyl] amino]-9H-purme-9-yl]-N-methyl-(-D-ribofuranuronamide) (CF101), Selodenoson (DTI-0009) and Binodenoson (MRE-0470). Other adenosine receptor agonists are those described or claimed in Gao et al., JPET, 298: 209-218 (2001); U.S. Patent Nos. 5,278,150, 5,877,180,6,232,297; U.S. Patent Application Publication No. 2005/0261236, and PCT Publication No. WO/9808855, incorporated herein by reference.

### Adenosine transport inhibitors

Adenosine transport inhibitors that can be employed in the methods, compositions, and kits of the invention include 3-[1-(6,7-diethoxy-2-morpholinoquinazolin-4-yl)piperidin-4-yl]-1,6-dimethyl-2,4(1H,3H)-quinazolinedione hydrochloride (KF24345); 6-(4-nitrobenzyl)-thioinosine (NBI) and 6-(2-hydroxy-5-nitrobenzyl)-thioguanosine (NBG); 6-[4-(1-cyclohexyl-1H-tetrazol-5-yl)butoxy]-3,4-dihydro-2(1H)-quinolinone (Cilostazol); (2-amino-4,5-dimethyl-3-thienyl)-[3-(trifluoromethyl) phenyl]methanone (PD 81723); 3;7-dihydro-3-methyl-1-(5-oxohexyl)-7-propyl-1H-purine-2,6-dione (propentofylline); 6-[(4-nitrobenzyl)thio]-9-β-D-ribofuranosylpurine (nitrobenzylthioinosine) (NBMR); 3,4,5-trimethoxy-, (tetrahydro-1H-1,4-diazepine-1,4(5H)-diyl)di-3,1-propanediyl benzoic acid, ester (dilazep); hexobendine; dipyridamole; and adenosine transport inhibitors described in Fredholm, J. Neurochem. 62:563-573 (1994), Noji et al., J. Pharmacol. Exp. Ther. 300:200-205 (2002); and Crawley et al.; Neurosci Lett. 36:169-174 (1983), each of which is incorporated herein by reference.

### Adenosine kinase inhibitors

Adenosine kinase inhibitors can be used as adenosine activity upregulators in the methods, compositions, and kits of the invention. Adenosine kinase inhibitors are generally described as either nucleoside-like, or nonnucleoside-like.

### Nucleoside-like adenosine kinase inhibitors

Nucleoside-like adenosine kinase inhibitors that can be used in the methods, compositions, and kits of the invention include 5-iodotubercidin (5IT) and 2-diaryltubercidin analogues; 5'-deoxo-5'-deoxy-5-iodotubercidin (5'd-5IT); and 5'-deoxo-5'-aminoadenosine (NH₂dADO). Other nucleoside-like adenosine kinase inhibitors are described in McGaraughty et al., Current Topics in Medicinal Chemistry 5:43-58 (2005); Ugarkar, J. Med. Chem. 43:2883-2893 (2000); Ugarkar et al., J. Med. Chem. 43:2894-2905 (2000); Kaplan and Coyle, Eur. J. Pharmacol. 1:1-8 (1998); and Sinclair et al. Br. J. Pharmacol. 5:1037-1044 (2001), each of which is incorporated herein by reference.

### Nonnucleoside-like adenosine kinase inhibitors

Nonnucleoside-like adenosine kinase inhibitors that can be used in the methods, compositions, and kits of the invention include 5-bromopyrrolopyrrolidine; 4-amino-5-(3-bromophenyl)-7-(6-morpholinopyridin-3-yl)pyrido[2,3-d]pyrimidine (ABT-702). Other nonnucleoside-like AK inhibitors are described in McGaraughty et al., Current Topics in Medicinal Chemistry 5:43-58 (2005), Gomtsyan and Lee, Current Pharmaceutical Design 10:1093-1103 (2004); Jarvis et al. J. Pharm. Exp. Ther. 295:1156-1164 (2000); Kowaluk, et al. J. Pharm. Exp. Ther. 295:1165-1174 (2000); and German Patent Application DE 10141212 A1, each of which is incorporated herein by reference.

### Phosphodiesterase inhibitors

Several isozymes of phosphodiesterases act as regulatory switches by catalyzing the degradation of cAMP to adenosine-5-monophosphate (5'-AMP). Inhibitors of phosphodiesterases can lead to an increase in cAMP levels, which in turn can lead to an increase in antiinflammatory actions.

### Type I phosphodiesterase inhibitors

Type I PDE inhibitors include (3-alpha,16-alpha)-eburnamenine-14-carboxylic acid ethyl ester (Vinpocetine); 18-methoxymethyl-3-isobutyl-1-methylxantine (MIMX); 1-carboxy-2,3,4,4a,4b,5,6,6a,6b,7,8,8a,8b,9,10,10a,14,16, 17,17a,17b,18, 19,19a,19b, 20,21,21a,21b,22,23,23 a-dotriacontahydro-14-hydroxy-8a, 10a-bis(hydroxymethyl)-14-(3-methoxy-3-oxopropyl)-1,4,4a, 6,6a,17b,19b,21b-octamethyl beta-D-glucopyranosiduronic acid (Ks-505a); cis-5,6a,7,8,9,9a-hexahydro-2-(4-(triffuoromethyl)phenylmethyl)-5-methyl-cyclopent (4,5)imidazo(2,1-b)purin-4(3H)-one (SCH 51866); and 2-o-propoxyphenyl-8-azapurine-6-one (Zaprinast). Other Type I PDE inhibitors are described in U.S. Patent Application Publication Nos. 2004/0259792 and 2005/0075795.

### Type II phosphodiesterase inhibitors

Type II PDE inhibitors include erythro-9-(2-hydroxy-3-nonyl)adenine (EHNA); 2,3,6,7-tetrahydro-9,10-dimethoxy-3-methyl-2-((2,4,6-trimethylphenyl)imino)-4H-pyrimido(6,1-a)isoquinolin-4-one (trequinsin); ND7001 (Neuro3D Pharmaceuticals); and BAY 60-7550 (Alexis Biochemicals). Other Type II PDE inhibitors are described in U.S. Patent Application Publication No. 2003/0176316.

### Type III phosphodiesterase inhibitors

Type III PDE inhibitors include 3-isobutyl-1-methylxanthine (IBMX); 6-dihydro-2-methyl-6-oxo-3,4'-bipyridine)-5-carbonitrile (milrinone) and N-cyclohexyl-4-((1,2-dihydro-2-oxo-6-quinolinyl)oxy)-N-methyl-butanaxnide (cilostamide). Other Type III PDE inhibitors are described in the following patents and patent applications: EP 0 653 426, EP 0 294 647, EP 0 357 788, EP 0 220 044, EP 0 326 307, EP 0 207 500, EP 0 406 958, EP 0 150 937, EP 0 075 463, EP 0 272 914, and EP 0 112 987, U.S. Pat. Nos. 4,963,561; 5,141,931, 6,897,229, and 6,156,753; U.S. Patent Application Publication Nos. 2003/0158133, 2004/0097593, 2006/0030611, and 2006/0025463; WO 96/15117; DE 2825048; DE 2727481; DE 2847621; DE 3044568; DE 2837161; and DE 3021792.

### Type IV phosphodiesterase inhibitors

Type IV PDE inhibitors include 4-(3-cyclopentyloxy-4-methoxyphenyl)-2-pyrrolidone (rolipram) and 4-(3-butoxy-4-methoxybenzyl)-2-imidazolidinone (Ro20-1724). Other Type IV PDE inhibitors are described in the following patents, patent applications, and references: U.S. Patent Nos. 3,892,777, 4,193,926, 4,655,074, 4,965,271, 5,096,906, 5,124,455, 5,272,153, 6,569,890, 6,953,853, 6,933,296, 6,919,353, 6,953,810, 6,949,573, 6,909,002, and 6,740,655; U.S. Patent Application Publication Nos. 2003/0187052, 2003/0187257, 2003/0144300, 2003/0130254, 2003/0186974, 2003/0220352, 2003/0134876, 2004/0048903, 2004/0023945, 2004/0044036, 2004/0106641, 2004/0097593, 2004/0242643, 2004/0192701, 2004/0224971, 2004/0220183, 2004/0180900, 2004/0171798, 2004/0167199, 2004/0146561, 2004/0152754, 2004/0229918, 200510192336, 2005/0267196, 2005/0049258, 2006/0014782, 2006/0004003, 2006/0019932, 2005/0267196, 2005/0222207, 2005/0222207, 2006/0009481; PCT Publication No. WO 92/079778; and Molnar-Kimber, K.L. et al. J. Immunol, 150:295A (1993).

### Type V phosphodiesterase inhibitors

Type V PDE inhibitors are described in U.S. Patent Nos. 6,992,192, 6,984,641, 6,960,587, 6,943,166, 6,878,711, and 6,869,950, and U.S. Patent Application Publication Nos. 2003/0144296, 2003/0171384, 2004/0029891, 2004/0038996, 2004/0186046, 2004/4259792, 2004/0087561, 2005/0054660, 2005/0042177, 2005/0245544, and 2006/0009481.

### Type VI phosphodiesterase inhibitors

Type VI PDE inhibitors include those described in U.S. Patent Application Publication Nos. 2004/0259792, 2004/0248957, 2004/0242673, and 2004/0259880.

### Type VII phosphodiesterase inhibitors

Type VII PDE inhibitors include those described in U.S. Patent Nos. 6,838,559, 6,753,340, 6,617,357, and 6,852,720; U.S. Patent Application Publication Nos. 2003/0186988, 2003/0162802, 2003/0191167, 2004/0214843, and 2006/0009481; PCT Publication WO 00/68230; and Martinez et al., J. Med. Chem. 43:683-689, 2000.

### Tricyclic compounds

Tricyclic compounds that can be used in the methods, compositions, and kits of the invention include amitriptyline, amoxapine, clomipramine, desipramine, dothiepin, doxepin, imipramine, lofepramine, maprotiline, mianserin, mirtazapine, nortriptyline, octriptyline, oxaprotiline, protriptyline, trimipramine, 10-(4-methylpiperazin-1-yl)pyrido(4,3-b)(1,4)benzothiazepine; 11-(4-methyl-1-piperazinyl)-5H-dibenzo(b,e)(1,4)diazepine; 5,10-dihydro-7-chloro-10-(2-(morpholino)ethyl)-11H-dibenzo(b,e)(,14)diazepin-11-one; 2-(2-(7-hydroxy-4-dibenzo(b,f)(1,4)thiazepine-11-yl-1-piperazinyl)ethoxy)ethanol; 2-chloro-11-(4-methyl-1-piperazmyl)-5H-dibenzo(b,e)(1,4)diazepine; 4-(11H-dibenz(b,e)azepin-6-yl)piperazine; 8-chloro-11-(4-methyl-1-piperazinyl)- 5H-dibenzo(b,e)(1,4)diazepin-2-ol; 8-chloro-11-(4-methyl-1-piperazinyl)- 5H-dibenzo(b,e)(1,4)diazepine monohydrochloride; (Z)-2-butenedioate 5H-dibenzo(b,e)(1,4)diazepine; adinazolam; amineptine; amitriptylinoxide; butriptyline; clothiapine; clozapine; demexiptiline; 11-(4-methyl-1-piperazinyl)-dibenz(b,f)(1,4)oxazepine; 11-(4-methyl-1-piperazinyl)-2-nitro-dibenz(b,f)(1,4)oxazepine; 2-chloro-11-(4-methyl-1-piperazinyl)-dibenz(b,f)(1,4)oxazepine monohydrochloride; dibenzepin; 11-(4-methyl-1-piperazinyl)-dibenzo(b,f)(1,4)thiazepine; dimetacrine; fluacizine; fluperlapine; imipramine N-oxide; iprindole; lofepramine; melitracen; metapramine; metiapine; metralindole; mianserin; mirtazapine; 8-chloro-6-(4-methyl-1-piperazinyl)-morphanthridine; N-acetylamoxapine; nomifensine; norclomipramine; norclozapine; noxiptilin; opipramol; oxaprotiline; perlapine; pizotyline; propizepine; quetiapine; quinupramine; tianeptine; tomoxetine; flupenthixol; clopenthixol; piflutixol; chlorprothixene; and thiothixene. Other tricyclic compounds are described, for example, in U.S. Patent Nos. 2,554,736; 3,046,283; 3,310,553; 3,177,209; 3,205,264; 3,244,748; 3,271,451; 3,272,826; 3,282,942; 3,299,139; 3,312,689; 3,389,139; 3,399,201; 3,409,640; 3,419,547; 3,438,981; 3,454,554; 3,467,650; 3,505,321; 3,527,766; 3,534,041; 3,539,573; 3,574,852; 3,622,565; 3,637,660; 3,663,696; 3,758,528; 3,922,305; 3,963,778; 3,978,121; 3,981,917; 4,017,542; 4,017,621; 4,020,096; 4,045,560; 4,045,580; 4,048,223; 4,062,848; 4,088,647; 4,128,641; 4,148,919; 4,153,629; 4,224,321; 4,224,344; 4,250,094; 4,284,559; 4,333,935; 4,358,620; 4,548,933; 4,691,040; 4,879,288; 5,238,959; 5,266,570; 5,399,568; 5,464,840; 5,455,246; 5,512,575; 5,550,136; 5,574,173; 5,681,840; 5,688,805; 5,916,889; 6,545,057; and 6,600,065, and phenothiazine compounds that fit Formula (I) of U.S. Patent Application Nos. 10/617,424 or 60/504,310.

Standard recommended dosages for several tricyclic compounds are provided in Table 6, below. Other standard dosages are provided, e.g., in the Merck Manual of Diagnosis & Therapy (17the Ed. MH Beers et al., Merck & Co.) and Physicians' Desk Reference 2003 (57th Ed. Medical Economics Staff et al., Medical Economics Co., 2002).

**Table 6**

| **Compound** | **Standard Dose** |
|---|---|
| Amoxapine | 200-300 mg/day |
| Nortriptyline | 75-150 mg/day |
| Desipramine | 100-200 mg/day |

### Selective serotonin reuptake inhibitors

SSRIs may be used in the methods, compositions, and kits of the invention. Suitable SSRIs are cericlamine (e.g., cericlamine hydrochloride); citalopram (e.g., citalopram hydrobromide); clovoxamine; cyanodothiepin; dapoxetine; escitalopram (escitalopram oxalate); femoxetine (e.g., femoxetine hydrochloride); fluoxetine (e.g., fluoxetine hydrochloride); fluvoxamine (e.g., fluvoxamine maleate); ifoxetine; indalpine (e.g., indalpine hydrochloride); indeloxazine (e.g., indeloxazine hydrochloride); litoxetine; milnacipran (e.g., minlacipran hydrochloride); paroxetine (e.g., paroxetine hydrochloride hemihydrate; paroxetine maleate; paroxetine mesylate); sertraline (e.g., sertraline hydrochloride); tametraline hydrochloride; viqualine; and zimeldine (e.g., zimeldine hydrochloride).

Structural analogs of cericlamine are those having the formula: 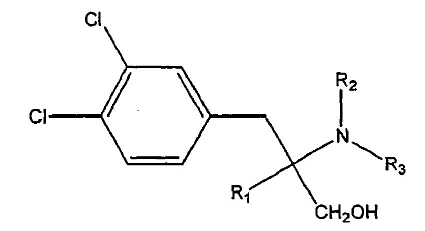 as well as pharmaceutically acceptable salts thereof, wherein R₁ is a C₁-C₄ alkyl and R₂ is H or C₁₋₄ alkyl, R₃ is H, C₁₋₄ alkyl, C₂₋₄ alkenyl, phenylalkyl or cycloalkylalkyl with 3 to 6 cyclic carbon atoms, alkanoyl, phenylalkanoyl or cycloalkylcarbonyl having 3 to 6 cyclic carbon atoms, or R₂ and R₃ form, together with the nitrogen atom to which they are linked, a heterocycle saturated with 5 to 7 chain links which can have, as the second heteroatom not directly connected to the nitrogen atom, an oxygen, a sulphur or a nitrogen, the latter nitrogen heteroatom possibly carrying a C₂₋₄ alkyl.

Exemplary cericlamine structural analogs are 2-methyl-2-amino-3-(3,4-dichlorophenyl)-propanol, 2-pentyl-2-amino-3-(3,4-dichlorophenyl)-propanol, 2-methyl-2-methylamino-3-(3,4-dichlorophenyl)-propanol, 2-methyl-2-dimethylamino-3-(3,4-dichlorophenyl)-propanol, and pharmaceutically acceptable salts of any thereof.

Structural analogs of citalopram are those having the formula: 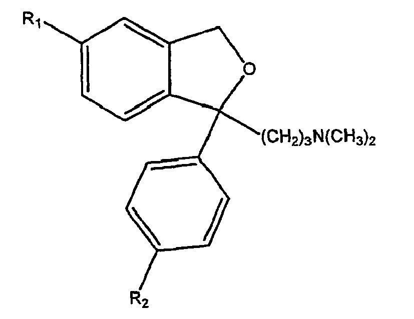 as well as pharmaceutically acceptable salts thereof, wherein each of R₁ and R₂ is independently selected from the group consisting of bromo, chloro, fluoro, trifluoromethyl, cyano and R-CO-, wherein R is C₁₋₄ alkyl.

Exemplary citalopram structural analogs (which are thus SSRI structural analogs according to the invention) are 1-(4'-fluorophenyl)-1-(3-dimethylaminopropyl)-5-bromophthalane; 1-(4'-chlorophenyl)-1-(3-dimethylaminopropyl)-5-chlorophthalane; 1-(4'-bromophenyl)-1-(3-dimethylaminopropyl)-5-chlorophthalane; 1-(4'-fluorophenyl)-1-(3-dimethylaminopropyl)-5-chlorophthalane; 1-(4'-chlorophenyl)-1-(3-dimethylaminopropyl)-5-trifluoromethyl-phthalane; 1-(4'-bromophenyl)-1-(3-d.imethylaminopropyl)-5-trifluoromethyl-phthalane; 1-(4'-fluorophenyl)-1-(3-dimethylaminopropyl)-5-trifluoromethyl-phthalane; 1-(4'-fluorophenyl)-1-(3-dimethylaminopropyl)-5-fluorophthalane; 1-(4'-chlorophenyl)-1-(3-dimethylaminopropyl)-5-fluorophthalane; 1-(4'-chlorophenyl)-1-(3-dimethylaminopropyl)-5-phthalancarbonitrile; 1-(4'-fluorophenyl)-1-(3-dimethylaminopropyl)-5-phthalancarbonitrile; 1-(4'-cyanophenyl)-1-(3-dimethylaminopropyl)-5-phthalancarbonitrile; 1-(4'-cyanophenyl)-1-(3-dimethylaminopropyl)-5-chlorophthalane; 1-(4'-cyanophenyl)-1-(3-dimethylaminopropyl)-5-trifluoromethylphthalane; 1-(4'-fluorophenyl)-1-(3-dimethylaminopropyl)-5-phthalancarbonitrile; 1-(4'-chlorophenyl)-1-(3-dimethylaminopropyl)-5-ionylphthalane; 1-(4-(chlorophenyl)-1-(3-dimethylaminopropyl)-5-propionylphthalane; and pharmaceutically acceptable salts of any thereof.

Structural analogs of clovoxamine are those having the formula: 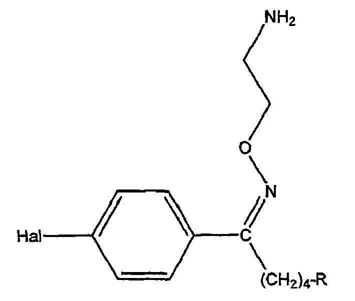 as well as pharmaceutically acceptable salts thereof, wherein Hal is a chloro, bromo, or fluoro group and R is a cyano, methoxy, ethoxy, methoxymethyl, ethoxymethyl, methoxyethoxy, or cyanomethyl group.

Exemplary clovoxamine structural analogs are 4'-chloro-5-ethoxyvalerophenone O-(2-aminoethyl)oxime; 4'-chloro-5-(2-methoxyethoxy)valerophenone O-(2-aminoethyl)oxime; 4'-chloro-6-methoxycaprophenone O-(2-aminoethyl)oxi.me; 4'-chloro-6-ethoxycaprophenone O-(2-aminoethyl)oxime; 4'-bromo-5-(2-methoxyethoxy)valerophenone O-(2-aminoethyl)oxime; 4'-bromo-5-methoxyvalerophenone O-(2-aminoethyl)oxime; 4'-chloro-6-cyanocaprophenone O-(2-ammoethyl)oxi.me; 4'-chloro-5-cyanovalerophenone O-(2-aminoethyl)oxime; 4'-bromo-5-cyanovalerophenone O-(2-aminoethyl)oxime; and pharmaceutically acceptable salts of any thereof.

Structural analogs of femoxetine are those having the formula: 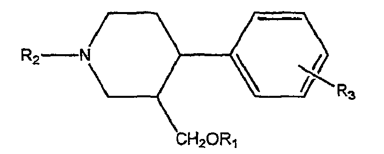 wherein R₁ represents a C₁₋₄ alkyl or C₂₋₄ alkynyl group, or a phenyl group optionally substituted by C₁₋₄ alkyl, C₁₋₄ alkylthio, C₁₋₄ alkoxy, bromo, chloro, fluoro, nitro, acylamino, methylsulfonyl, methylenedioxy, or tetrahydronaphthyl, R₂ represents a C₁₋₄ alkyl or C₂₋₄ alkynyl group, and R₃ represents hydrogen, C₁₋₄ alkyl, C₁₋₄alkoxy, trifluoroalkyl, hydroxy, bromo, chloro, fluoro, methylthio, or aralkyloxy.

Exemplary femoxetine structural analogs are disclosed in Examples 7-67 of U.S. Patent No. 3,912,743, hereby incorporated by reference.

Structural analogs of fluoxetine are those compounds having the formula: 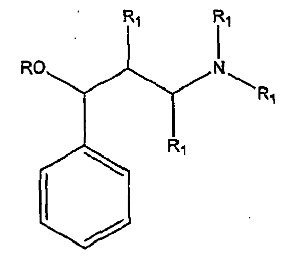 as well as pharmaceutically acceptable salts thereof, wherein each R₁ is independently hydrogen or methyl; R is naphthyl or 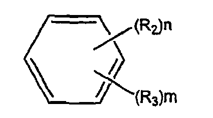 wherein each of R₂ and R₃ is, independently, bromo, chloro, fluoro, trifluoromethyl, C₁₋₄ alkyl, C₁₋₃ alkoxy or C₃₋₄ alkenyl; and each of n and m is, independently, 0, 1 or 2. When R is naphthyl, it can be either α-naphthyl or β-naphthyl.

Exemplary fluoxetine structural analogs are 3-(p-isopropoxyphenoxy)-3-phenylpropylamine methanesulfonate, N,N-dimethyl 3-(3',4'-dimethoxyphenoxy)-3-phenylpropylamine p-hydroxybenzoate, N,N-dimethyl 3-(α-naphthoxy)-3-phenylpropylamine bromide,N,N-dimcthyl 3-(β-naphthoxy)-3-phenyl-1-methylpropylamine iodide, 3-(2'-methyl-4',5'-dichlorophenoxy)-3-phenylpropylamine nitrate, 3-(p-t-butylphenoxy)-3-phenylpropylamine glutarate, N-methyl 3-(2'-chloro-p-tolyloxy)-3-phenyl-1-methylpropylamine lactate, 3-(2',4'-dichlorophenoxy)-3-phenyl-2-methylpropylamine citrate, N,N-dimethyl 3-(m-anisyloxy)-3-phenyl-1-methylpropylamine maleate, N-methyl 3-(p-tolyloxy)-3-phenylpropylamine sulfate, N,N-dimethyl 3-(2',4'-difluorophenoxy)-3-phenylpropylamine 2,4-dinitrobenzoate, 3-(o-ethylphenoxy)-3-phenylpropylamine dihydrogen phosphate, N-methyl 3-(2'-chloro-4'-isopropylphenoxy)-3-phenyl-2-methylpropylamine maleate, N,N-dimethyl 3-(2'-alkyl-4'-fluorophonoxy)-3-phenyl-propylamine succinate, N,N-dimethyl 3-(o-isopropoxyphenoxy)-3-phenyl-propylamine phenylacetate, N,N-dimethyl 3-(o-bromophenoxy)-3-phenyl-propylamine β-phenylpropionate, N-methyl 3-(p-iodophenoxy)-3-phenyl-propylamine propiolate, and N-methyl 3-(3-n-propylphenoxy)-3-phenyl-propylamine decanoate.

Structural analogs of fluvoxamine are those having the formula: 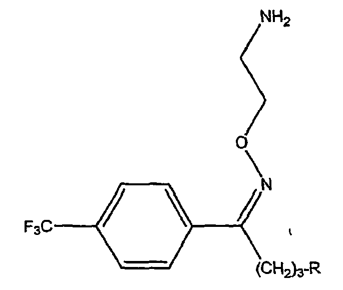 as well as pharmaceutically acceptable salts thereof, wherein R is cyano, cyanomethyl, methoxymethyl, or ethoxymethyl.

Structural analogs of indalpine are those having the formula: 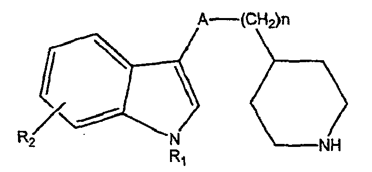 or pharmaceutically acceptable salts thereof, wherein R₁ is a hydrogen atom, a C₁-C₄ alkyl group, or an aralkyl group of which the alkyl has 1 or 2 carbon atoms, R₂ is hydrogen, C₁₋₄ alkyl, C₁₋₄ alkoxy or C₁₋₄ alkylthio, chloro, bromo, fluoro, trifluoromethyl, nitro, hydroxy, or amino, the latter optionally substituted by one or two C₁₋₄ alkyl groups, an acyl group or a C₁₋₄alkylsulfonyl group; A represents -CO or -CH₂- group; and n is 0, 1 or 2.

Exemplary indalpine structural analogs are indolyl-3 (piperidyl-4 methyl) ketone; (methoxy-5-indolyl-3) (piperidyl-4 methyl) ketone; (chloro-5-indolyl-3) (piperidyl-4 methyl) ketone; (indolyl-3)-1(piperidyl-4)-3 propanone, indolyl-3 piperidyl-4 ketone; (methyl-1 indolyl-3) (piperidyl-4 methyl) ketone, (benzyl-1 indolyl-3) (piperidyl-4 methyl) ketone; [(methoxy-5 indolyl-3)-2 ethyl]-piperidine, [(methyl-1 indolyl-3)-2 ethyl]-4-piperidine; [(indolyl-3)-2 ethyl]-4 piperidine; (indolyl-3 methyl)-4 piperidine, [(chloro-5 indolyl-3)-2 ethyl]-4 piperidine; [(indolyl-b 3)-3 propyl]-4 piperidine; [(benzyl-1 indolyl-3)-2 ethyl]-4 piperidine; and pharmaceutically acceptable salts of any thereof.

Structural analogs of indeloxazine are those having the formula: 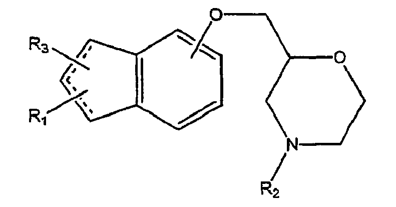 and pharmaceutically acceptable salts thereof, wherein R₁ and R₃ each represents hydrogen, C₁₋₄ alkyl, or phenyl; R₂ represents hydrogen, C₁₋₄ alkyl, C₄₋₇ cycloalkyl, phenyl, or benzyl; one of the dotted lines means a single bond and the other means a double bond, or the tautomeric mixtures thereof.

Exemplary indelokazine structural analogs are 2-(7-indenyloxymethyl)-4-isopropylinorpholine; 4-butyl-2-(7-indenyloxymethyl)morpholine; 2-(7-indenyloxymethyl)-4-methylmorpholine; 4-ethyl-2-(7-indenyloxymethyl)morpholine, 2-(7-indenyloxymethyl)-morpholine; 2-(7-indenyloxymethyl)-4-propylmorpholine; 4-cyclohexyl-2-(7-indenyloxymethyl)morpholine; 4-benzyl-2-(7-indenyloxymethyl)-morpholine; 2-(7-indenyloxymethyl)-4-phenylmorpholine; 2-(4-indenyloxymethyl)morpholine; 2-(3-methyl-7-indenyloxymethyl)-morpholine; 4-isopropyl-2-(3-methyl-7-indenyloxymethyl)morpholine; 4-isopropyl-2-(3-methyl-4-indenyloxymethyl)morpholine; 4-isopropyl-2-(3-methyl-5-indenyloxymethyl)morpholine; 4-isopropyl-2-(1-methyl-3-phenyl-6-indenyloxymethyl)morpholine; 2-(5-indenyloxymethyl)-4-isopropylmorpholine, 2-(6-indenyloxymethyl)-4-isopropylmorpholine; and 4-isopropyl-2-(3-phenyl-6-indenyloxymethyl)morpholine; as well as pharmaceutically acceptable salts of any thereof.

Structural analogs of milnacipram are those having the formula: 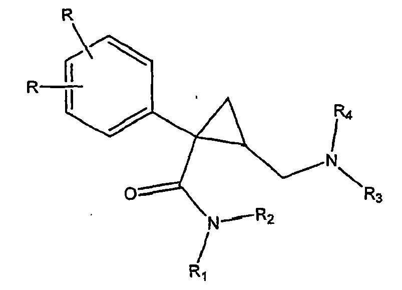 as well as pharmaceutically acceptable salts thereof, wherein each R, independently, represents hydrogen, bromo, chloro, fluoro, C₁₋₄ alkyl, C₁₋₄ alkoxy, hydroxy, nitro or amino; each of R₁ and R₂, independently, represents hydrogen, C₁₋₄ alkyl, C₆₋₁₂ aryl or C₇₋₁₄ alkylaryl, optionally substituted, preferably in para position, by bromo, chloro, or fluoro, or R₁ and R₂ together form a heterocycle having 5 or 6 members with the adjacent nitrogen atoms; R₃ and R₄ represent hydrogen or a C₁₋₄ alkyl group or R₃ and R₄ form with the adjacent nitrogen atom a heterocycle having 5 or 6 members, optionally containing an additional heteroatom selected from nitrogen, sulphur, and oxygen.

Exemplary milnacipram structural analogs are 1-phenyl 1-aminocarbonyl 2-dimethylaminomethyl cyclopropane; 1-phenyl 1-dimethylaminocarbonyl 2-dimethylaminomethyl cyclopropane; 1-phenyl 1-ethylaminocarbonyl 2-dimethylaminomethyl cyclopropane; 1-phenyl 1-diethylaminocarbonyl 2-aminomethyl cyclopropane; 1-phenyl 2-dimethylaminomethyl N-(4'-chlorophenyl)cyclopropane carboxamide; 1-phenyl 2-dimethylaminomethyl N-(4'-chlorobenzyl)cyclopropane carboxamide; 1-phenyl 2-dimethylaminomethyl N-(2-phenylethyl)cyclopropane carboxamide; (3,4-dichloro-1-phenyl) 2-dimethylaminomethyl N,N-dimethylcyclopropane carboxamide; 1-phenyl 1-pyrrolidinocarbonyl 2-morpholinomethyl cyclopropane; 1-p-chlorophenyl 1-aminocarbonyl 2-aminomethyl cyclopropane; 1-orthochlorophenyl 1-aminocarbonyl 2-dimethylaminomethyl cyclopropane; 1-p-hydroxyphenyl 1-aminocarbonyl 2-dimethylaminomethyl cyclopropane; 1-p-nitrophenyl 1-dimethylaminocarbonyl 2-dimethylaminomethyl cyclopropane; 1-p-aminophenyl 1-dimethylaminocarbonyl 2-dimethylaminomethyl cyclopropane; 1-p-tolyl 1-methylaminocarbonyl 2-dimethylaminomethyl cyclopropane; 1-p-methoxyphenyl 1-aminomethylcarbonyl 2-aminomethyl cyclopropane; and pharmaceutically acceptable salts of any thereof.

Structural analogs of paroxetine are those having the formula: 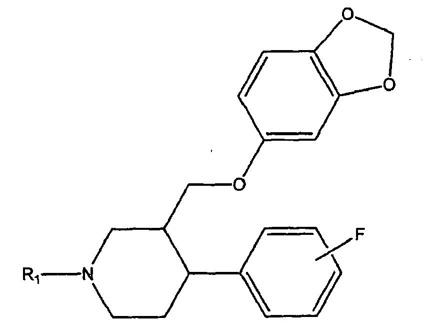 and pharmaceutically acceptable salts thereof, wherein R₁ represents hydrogen or a C₁₋₄ alkyl group, and the fluorine atom may be in any of the available positions.

Structural analogs of sertraline are those having the formula: 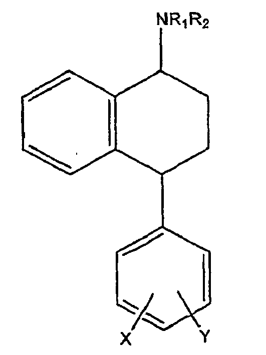 wherein R₁ is selected from the group consisting of hydrogen and C₁₋₄ alkyl; R₂ is C₁₋₄ alkyl; X and Y are each selected from the group consisting of hydrogen, fluoro, chloro, bromo, trifluoromethyl, C₁₋₃ alkoxy, and cyano; and W is selected from the group consisting of hydrogen, fluoro, chloro, bromo, trifluoromethyl and C₁₋₃ alkoxy. Preferred sertraline analogs are in the cis-isomeric configuration. The term "cis-isomeric" refers to the relative orientation of the NR₁R₂ and phenyl moieties on the cyclohexene ring (i.e. they are both oriented on the same side of the ring). Because both the 1- and 4-carbons are asymmetrically substituted, each cis- compound has two optically active enantiomeric forms denoted (with reference to the 1-carbon) as the cis-(1R) and cis-(1S) enantiomers.

Particularly useful are the following compounds, in either the (1S)-enantiomeric or (1S)(1R) racemic forms, and their pharmaceutically acceptable salts: cis-N-methyl-4-(3,4-dichlorophenyl)-1,2,3,4-tetrahydro-1-naphthalenamine; cis-N-methyl-4-(4-bromophenyl)-1,2,3,4-tetrahydro-1-naphthalenamine; cis-N-methyl-4-(4-chlorophenyl)-1,2,3,4-tetrahydro-1-naphthalenamine; cis-N-methyl-4-(3-trifluoromethyl-phenyl)-1,2,3,4-tetrahydro-1-naphthalenamine; cis-N-methyl-4-(3-trifluoromethyl-4-chlorophenyl)-1,2,3,4-tetrahydro-1-naphthalenamine; cis-N,N-dimethyl-4-(4-chlorophenyl)-1,2,3,4-tetrahydro-1-naphthalenamine; cis-N,N-dimethyl-4-(3-trifluoromethyl-phenyl)-1,2,3,4-tetrahydro-1-naphthalenamine; and cis-N-methyl-4-(4-chlorophenyl)-7-chloro-1,2,3,4-tetrahydro-1-naphthalenamine. Of interest also is the (1R)-enantiomer of cis-N-methyl-4-(3,4-dichlorophenyl)-1,2,3,4-tetrahydro-1-naphthalenamine.

Structural analogs of zimeldine are those compounds having the formula: 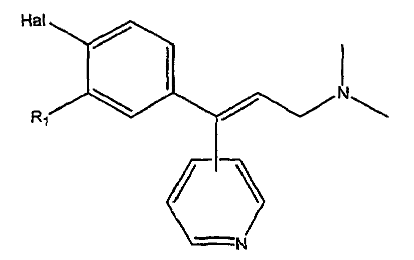 and pharmaceutically acceptable salts thereof, wherein the pyridine nucleus is bound in ortho-, meta- or para-position to the adjacent carbon atom and where R₁ is selected from the group consisting of H, chloro, fluoro, and bromo.

Exemplary zimeldine analogs are (e)- and (z)- 3-(4'-bromophenyl-3-(2"-pyridyl)-dimethylallylamine; 3-(4'-bromophenyl)-3-(3"-pyridyl)-dimethylallylamine; 3-(4'-bromophenyl)-3-(4"-pyridyl)-dimethylallylamine; and pharmaceutically acceptable salts of any thereof.

Structural analogs of any of the above SSRIs are considered herein to be SSRI analogs and thus may be employed in any of the methods, compositions, and kits of the invention.

### Metabolites

Pharmacologically active metabolites of any of the foregoing SSRIs can also be used in the methods, compositions, and kits of the invention. Exemplary metabolites are didesmethylcitalopram, desmethylcitalopram, desmethylsertraline, and norfluoxetine.

### Analogs

Functional analogs of SSRIs can also be used in the methods, compositions, and kits of the invention. Exemplary SSRI functional analogs are provided below. One class of SSRI analogs includes SNRIs (selective serotonin norepinephrine reuptake inhibitors), which include venlafaxine, duloxetine, and 4-(2-fluorophenyl)-6-methyl-2-piperazinothieno [2,3-d] pyrimidine.

Structural analogs of venlafaxine are those compounds having the formula: 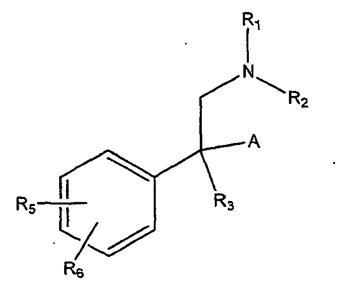 as well as pharmaceutically acceptable salts thereof, wherein A is a moiety of the formula: 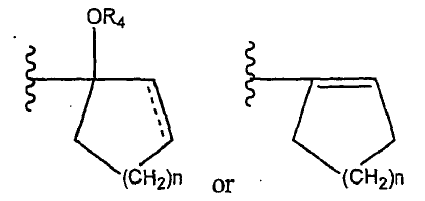 where the dotted line represents optional unsaturation; R₁ is hydrogen or alkyl; R₂ is C₁₋₄ alkyl; R₄ is hydrogen, C₁₋₄ alkyl, formyl or alkanoyl; R₃ is hydrogen or C₁₋₄ alkyl; R₅ and R₆ are, independently, hydrogen, hydroxyl, C₁₋₄ alkyl, C₁₋₄ alkoxy, C₁₋₄ alkanoyloxy, cyano, nitro, alkylmercapto, amino, C₁₋₄ alkylamino, dialkylamino, C₁₋₄ alkanamido, halo, trifluoromethyl or, taken together, methylenedioxy; and n is 0, 1, 2, 3 or 4.

Structural analogs of duloxetine are those compounds described by the formula disclosed in U.S. Patent No. 4,956,388, hereby incorporated by reference.

Other SSRI analogs are 4-(2-fluorophenyl)-6-methyl-2-piperazinothieno [2,3-d] pyrimidine, 1,2,3,4-tetrahydro-N-methyl-4-phenyl-1-naphthylamine hydrochloride; 1,2,3,4-tetrahydro-N-methyl-4-phenyl-(E)-1-naphthylamine hydrochloride; N,N-dimethyl-1-phenyl-1-phthalanpropylamine hydrochloride; gamma-(4-(trifluorornethyl)phenoxy)-benzenepropanamine hydrochloride; BP 554; CP 53261; O-desmethylvenlafaxine; WY 45,818; WY 45,881; N-(3-fluoropropyl)paroxetine; Lu 19005; and SNRIs described in PCT Publication No. WO04/004734.

### Standard recommended dosages

Standard recommended dosages for exemplary SSRIs are provided in Table 7, below. Other standard dosages are provided, e.g., in the Merck Manual of Diagnosis & Therapy (17th Ed. MH Beers et al., Merck & Co.) and Physicians' Desk Reference 2003 (57th Ed. Medical Economics Staff et al., Medical Economics Co., 2002).

**Table 7**

| **Compound** | **Standard Dose** |
|---|---|
| Fluoxetine | 20 - 80 mg / day |
| Sertraline | 50 - 200 mg / day |
| Paroxetine | 20 - 50 mg / day |
| Fluvoxamine | 50-300 mg / day |
| Citalopram | 10 - 80 mg qid |
| Escitalopram | 10 mg qid |

### Antihistamines

In yet another embodiment of the invention, the methods, compositions, and kits of the invention employ a histamine receptor antagonist (or analog thereof). Antihistamines are compounds that block the action of histamine. Classes of antihistamines include:
(1) Ethanolamines (e.g., bromodiphenhydramine, carbinoxamine, clemastine, dimenhydrinate, diphenhydramine, diphenylpyraline, and doxylamine);
(2) Ethylenediamines (e.g., pheniramine, pyrilamine, tripelennamine, and triprolidine);
(3) Phenothiazines (e.g., diethazine, ethopropazine, methdilazine, promethazine, thiethylperazine, and trimeprazine);
(4) Alkylamines (e.g., acrivastine, brompheniramine, chlorpheniramine, desbrompheniramine, dexchlorpheniramine, pyrrobutamine, and triprolidine);
(5) Piperazines (e.g., buclizine, cetirizine, chlorcyclizine, cyclizine, meclizine, hydroxyzine);
(6) Piperidines (e.g., astemizole, azatadine, cyproheptadine, desloratadine, fexofenadine, loratadine, ketotifen, olopatadine, phenindamine, and terfenadine);
(7) Atypical antihistamines (e.g., azelastine, levocabastine, methapyrilene, and phenyltoxamine).

In the methods, compositions, and kits of the invention, both non-sedating and sedating antihistamines may be employed. Particularly desirable antihistamines for use in the methods, compositions, and kits of the invention are non-sedating antihistamines such as loratadine, and desloratadine. Sedating antihistamines can also be used in the methods, compositions, and kits of the invention. Preferred sedating antihistamines for use in the methods, compositions, and kits of the invention are azatadine, bromodiphenhydramine; chlorpheniramine; clemizole; cyproheptadine; dimenhydrinate; diphenhydramine; doxylamine; meclizine; promethazine; pyrilamine; thiethylperazine; and tripelennamine.

Other antihistamines suitable for use in the methods and compositions of the invention are acrivastine; ahistan; antazoline; astemizole; azelastine (e.g., azelsatine hydrochloride); bamipine; bepotastine; bietanautine; brompheniramine (e.g., brompheniramine maleate); carbinoxamine (e.g., carbinoxamine maleate); cetirizine (e.g., cetirizine hydrochloride); cetoxime; chlorocyclizine; chloropyramine; chlorothen; chlorphenoxamine; cinnarizine; clemastine (e.g., clemastine fumarate); clobenzepam; clobenztropine; clocinizine; cyclizine (e.g., cyclizine hydrochloride; cyclizine lactate); deptropine; dexchlorpheniramine; dexchlorpheniramine maleate; diphenylpyraline; doxepin; ebastine; embramine; emedastine (e.g., emedastine difumarate); epinastine; etymemazine hydrochloride; fexofenadine (e.g., fexofenadine hydrochloride); histapyrrodine; hydroxyzine (e.g., hydroxyzine hydrochloride; hydroxyzine pamoate); isopromethazine; isothipendyl; levocabastine (e.g., levocabastine hydrochloride); mebhydroline; mequitazine; methafurylene; methapyrilene; metron; mizolastine; olapatadine (e.g., olopatadine hydrochloride); orphenadrine; phenindamine (e.g., phenindamine tartrate); pheniramine; phenyltoloxamine; p-methyldiphenhydramine; pyrrobutamine; setastine; talastine; terfenadine; thenyldiamine; thiazinamium (e.g., thiazinamium methylsulfate); thonzylamine hydrochloride; tolpropamine; triprolidine; and tritoqualine.

Structural analogs of antihistamines may also be used in according to the invention. Antihistamine analogs include, without limitation, 10-piperazinylpropylphenothiazine; 4-(3-(2-chlorophenothiazin-10-yl)propyl)-1-piperazineethanol dihydrochloride; 1-(10-(3-(4-methyl-1-piperazinyl)propyl)-10H-phenothiazin-2-yl)-(9CI) 1-propanone; 3-methoxycyproheptadine; 4-(3-(2-Chloro-10H-phenothiazin-10-yl)propyl)piperazine-1-ethanol hydrochloride; 10,11-dihydro-5-(3-(4-ethoxycarbonyl-4-phenylpiperidino)propylidene)-5H-dibenzo(a,d)cycloheptene; aceprometazine; acetophenazine; alimemazin (e.g., alimemazin hydrochloride); aminopromazine; benzimidazole; butaperazine; carfenazine; chlorfenethazine; chlormidazole; cinprazole; desmethylastemizole; desmethylcyproheptadine; diethazine (e.g., diethazine hydrochloride); ethopropazine (e.g., ethopropazine hydrochloride); 2-(p-bromophenyl-(p'-tolyl)methoxy)-N,N-dimethyl-ethylamine hydrochloride; NN-dimethyl-2-(diphenylmethoxy)-ethylamine methylbromide; EX-10-542A; fenethazine; fuprazole; methyl 10-(3-(4-methyl-1-piperazinyl)propyl)phenothiazin-2-yl ketone; lerisetron; medrylamine; mesoridazine; methylpromazine; N-desmethylpromethazine; nilprazole; northioridazine; perphenazine (e.g., perphenazine enanthate); 10-(3-dimethylaminopropyl)-2-methylthiophenothiazine; 4-(dibenzo(b,e)thiepin-6(11H)-ylidene)-1-methyl-pipcridine hydrochloride; prochlorperazine; promazine; propiomazine (e.g., propiomazine hydrochloride); rotoxamine; rupatadine; Sch 37370; Sch 434; tecastemizole; thiazinamium; thiopropazate; thioridazine (e.g., thioridazine hydrochloride); and 3-(10,11-dihydro-5H-dibenzo(a,d)cyclohepten-5-ylidene)-tropane.

Other compounds that are suitable for use in the invention are AD-0261; AHR-5333; alinastine; arpromidine; ATI-19000; bermastine; bilastin; Bron-12; carebastine; chlorphenamine; clofurenadine; corsym; DF-1105501; DF-11062; DF-1111301; EL-301; elbanizine; F-7946T; F-9505; HE-90481; HE-90512; hivenyl; HSR-609; icotidine; KAA-276; KY-234; lamiakast; LAS-36509; LAS-36674; levocetirizine; levoprotiline; metoclopramide; NIP-531; noberastine; oxatomide; PR-881-884A; quisultazine; rocastine; selenotifen; SK&F-94461; SODAS-HC; tagorizine; TAK-427; temelastine; UCB-34742; UCB-35440; VUF-K-8707; Wy-49051; and ZCR-2060.

Still other compounds that are suitable for use in the invention are described in U.S. Patent Nos. 3,956,296; 4,254,129; 4,254,130; 4,282,833; 4,283,408; 4,362,736; 4,394,508; 4,285,957; 4,285,958; 4,440,933;.4,510,309; 4,550,116; 4,692,456; 4,742,175; 4,833,138; 4,908,372; 5,204,249; 5,375,693; 5,578,610; 5,581,011; 5,589,487; 5,663,412; 5,994,549; 6,201,124; and 6,458,958.

### Standard recommended dosages

Standard recommended dosages for several exemplary antihistamines are shown in Table 8. Other standard dosages are provided, e.g., in the Merck Manual of Diagnosis & Therapy (17th Ed. MH Beers et al., Merck & Co.) and Physicians' Desk Reference 2003 (57th Ed. Medical Economics Staff et al., Medical Economics Co., 2002).

**Table 8**

| **Compound** | **Standard Dose** |
|---|---|
| Desloratadine | 5 mg / once daily |
| Thiethylperazine | 10 mg / 1-3 times daily |
| Bromodiphenhydramine | 12.5-25 mg / every 4-6 hours |
| Promethazine | 25 mg / twice daily |
| Cyproheptadine | 12-16 mg/day |
| Loratadine | 10 mg / once daily |
| Clemizole | 100 mg given as IV or IM |
| Azatadine | 1-2 mg / twice daily |
| Cetirizine | 5-10 mg / once daily |
| Chlorpheniramine | 2 mg / every 6 hours or 4 mg / every 6 hours |
| Dimenhydramine | 50-100 mg / every 4-6 hours |
| Diphenydramine | 25 mg / every 4 -6 hours or 38 mg / every 4-6 hours * |
| Doxylamine | 25 mg / once daily or 12.5 mg / every four hours * |
| Fexofenadine | 60 mg/ twice daily or 180 mg/ once daily |
| Meclizine | 25 - 100 mg / day |
| Pyrilamine | 3 0 mg / every 6 hours |
| Tripelennamine | 25 - 50 mg / every 4 to 6 hours or 100 mg / twice daily (extended release) * |

### Loratadine

Loratadine (CLARITIN) is a tricyclic piperidine that acts as a selective peripheral histamine H1-receptor antagonist. Loratadine and structural and functional analogs thereof, such as piperidines, tricyclic piperidines, histamine H1-receptor antagonists, may be used in the anti-immunoinflammatory combination of the invention for the treatment of immunoinflammatory disorders, transplanted organ rejection, and graft versus host disease.

Loratadine functional and/or structural analogs include other H1-receptor antagonists, such as AHR-11325, acrivastine, antazoline, astemizole, azatadine, azelastine, bromopheniramine, carebastine, cetirizine, chlorpheniramine, chlorcyclizine, clemastine, cyproheptadine, descarboethoxyloratadine, dexchlorpheniramine, dimenhydrinate, diphenylpyraline, diphenhydramine, ebastine, fexofenadine, hydroxyzine ketotifen, lodoxamide, levocabastine, methdilazine, mequitazine, oxatomide, pheniramine pyrilamine, promethazine, pyrilamine, setastine, tazifylline, temelastine, terfenadine, trimeprazine, tripelennamine, triprolidine, utrizine, and similar compounds (described, e.g., in U.S. Patent Nos. 3,956,296, 4,254,129, 4,254,130, 4,283,408, 4,362,736, 4,394,508, 4,285,957; 4,285,958, 4,440,933, 4,510,309, 4,550,116, 4,692,456, 4,742,175, 4,908,372, 5,204,249, 5,375,693, 5,578,610, 5,581,011, 5,589,487, 5,663,412, 5,994,549, 6,201,124, and 6,458,958).

Loratadine, cetirizine, and fexofenadine are second-generation H1-receptor antagonists that lack the sedating effects of many first generation H1-receptor antagonists. Piperidine H1-receptor antagonists include loratadine, cyproheptadine hydrochloride (PERIACTIN), and phenindiamine tartrate (NOLAHIST). Piperazine H1-receptor antagonists include hydroxyzine hydrochloride (ATARAX), hydroxyzine pamoate (VISTARIL), cyclizine hydrochloride (MAREZINE), cyclizine lactate, and meclizine hydrochloride.

### Standard recommended dosages

Loratadine oral formulations include tablets, redi-tabs, and syrup. Loratadine tablets contain 10 mg micronized loratadine. Loratadine syrup contains 1 mg/ml micronized loratadine, and reditabs (rapidly-disintegrating tablets) contain 10 mg micronized loratadine in tablets that disintegrate quickly in the mouth. While suggested dosages will vary with a patient's condition, standard recommended dosages are provided below. Loratadine is typically administered once daily in a 10 mg dose, although other daily dosages useful in the anti-immunoinflammatory combination of the invention include 0.01-0.05 mg, 0.05-1 mg, 1-3 mg, 3-5 mg, 5-10 mg, 10-15 mg, 15-20 mg, 20-30 mg, and 30-40 mg.

Loratadine is rapidly absorbed following oral administration. It is metabolized in the liver to descarboethoxyloratadine by cytochrome P450 3A4 and cytochrome P450 2D6. Loratadine metabolites are also useful in the anti-immunoinflammatory combination of the invention.

### Bufexamac

Bufexamac (p-butoxyphenylacetydroxamic, 4-butoxyphenylacetohydroxamic) acts as an anti-pruritic and anti-inflammatory drug.

Bufexamac analogs include, but are not limited to, 2-(p-propoxyphenyl)acetohydroxamic acid; 2-(4-butoxy-m-tolyl)acetohydroxamic acid; 2-(4-butoxy-3-ethylphenyl)acetohydroxamic acid; 2-(4-butoxy-3-chlorophenyl)acetohydroxamic acid.

The indications for bufexamac include but are not limited to eczema, dermatitis, pruritus, and hemorrhoids. Bufexamac is currently available in a cream, oral, gel, ointment, or suppository formulation. The cream is typically used 1 to 3 times daily. Creams, ointments, suppositories, and gels have a concentration of bufexamac ranging from 0.25% to 0.5%. Additionally, oral dosages of bufexamac are often given for patients afflicted with rheumatoid arthritis. In this case, a dosage of bufexamac of 250 mg is given four times a day. Clinical dosages of bufexamac up to 2.0 g per day have been given.

### Calcium channel inhibitors

Calcium channel inhibitors may be used in the methods, compositions, and kits of the invention. Calcium channel inhibitors include verapamil, anipamil, bepridil, gallopamil, devapamil, falipamil, tiapamil, nifedipine, amlodipine, dazodipine, felodipine, isradipine, lanicardipine, nicardipine, nimodipine, nisoldipine, nitrendipine, ryosidie, diltiazem, cinnarizine, and flunarizine, BAY-m 4786, and diperdipine.

### Antidyskinetics

Antidyskinetic compounds that may be used in the methods, compositions, and kits of the invention include D-AP5 (D(-)-2-amino-5-phosphonopentanoate), CGS19755 (4-phosphonomethyl-2-piperidine carboxylic acid), CGP37849 (D,L-(E)-2-amino-4-methylphosphono-3-pentanoic acid), LY233053 (cis-(.+-.)-4-(2H-tetrazol-5-yl)methyl-piperidine-2-carboxyl acid), AIDA (1-aminoindan-1,5(RS)-dicarboxylic acid), (s)-(+)-CBPG ((S)-(+)-2-(3'-carboxy-bicyclo(1.1.1.)pentyl)glycine), CPCCOEt (cyclopropan(b)chromen-1a-carboxylate), EGLU ((s)-(.alpha.)-ethylglutamate), LY307452 (2s,4s-2-amino-4-(4,4-diphenylbut-1-yl)pentan-1,5-dioc acid), LY341495 (2s-2-amino-2-(1s,2s-2-carboxy-cyclopropan-1-yl)-3-(xanth-9-yl)propanoic acid), PCCG-4 (2s,1's,2's,3'R)-2-(2'-carboxy-3'-phenylcyclopropyl)glycine), 4-CPG (4-carboxyphenylglycine), remacemide, dextromethorphan ((+)-3-hydroxy-N-methylmorphinan), its metabolite dextrorphan ((+)-3-hydroxy-N-methylmorphinan), amantadine (1-amino adamantine), memantine (3,5 dimethylaminoadamantone), pyrroloquinoline quinone, and cis-4-(phosphonomethyl)-2-piperidinecarboxylic acid.

### Anticoccidial compounds

Anticoccidial compounds may be employed in the methods, compositions, and kits of the invention. Such compounds include salinomycin, monensin, narasin, lasalocid, nicarbazin, maduramycin, nicarbazin and narasin in combination, diclazuril, dinitolmide, halofuginone, robenidine, amprolium and clopidol. Other anticoccidial compounds are described in U.S. Patent Nos. 4,582,822; 4,824,863; 5,552,386; 6,384,052; and 6,528,531.

### Therapy

The invention features methods for treating an immunoinflammatory disorder. While the examples describe a two-drug combination, it is understood that the combination of multiple agents is often desirable. For example, methotrexate, hydroxychloroquine, and sulfasalazine are commonly administered for the treatment of rheumatoid arthritis, and may also me employed in the methods, compositions, and kits of the invention. Additional therapies are described below.

Desirably, the methods, compositions, and kits of the invention are more effective than other methods, compositions, and kits. By "more effective" is meant that a method, composition, or kit exhibits greater efficacy, is less toxic, safer, more convenient, better tolerated, or less expensive, or provides more treatment satisfaction than another method, composition, or kit with which it is being compared.

### Psoriasis

The methods, compositions, and kits of the invention may be used for the treatment of psoriasis. If desired, one or more antipsoriatic agents typically used to treat psoriasis may be used in the methods, compositions, and kits of the invention. Such agents include biologics (e.g. alefacept, infliximab, adalimumab, efalizumab, etanercept, and CDP-870), small molecule immunomodulators (e.g., VX 702, SCIO 469, doramapimod, RO 30201195, SCIO 323, DPC 333, pralnacasan, mycophenolate, and merimepodib), non-steroidal immunophilin-dependent immunosuppressants (e.g. cyclosporine, tacrolimus, pimecrolimus, ABT-281, and ISAtx247), vitamin D analogs (e.g. calcpotriene, calcipotriol), psoralens (e.g. methoxsalen), retinoids (e.g. acitretin, tazarotene), DMARDs (e.g. methotrexate), anthralin, topical glucocorticosteroids (e.g. clobetasol, triamcinolone, betamethasone, hydrocortisone, halobetasol, diflorasone, mometasone, halcinonide, fluticasone), and systemic glucocorticosteroids (e.g. prednisone, dexamethasone).

### Atopic dermatitis

The methods, compositions, and kits of the invention may be used for the treatment of atopic dermatitis. If desired, one or more atopic dermatitis agents typically used to treat atopic dermatitis may be used in the methods, composition, and kits of the invention. Such agents include topical and systemic non-steroidal immunophilin-dependent immunosuppressants (e.g. cyclosporine, tacrolimus, pimecrolimus, ABT-281, and ISAtx247), topical glucocorticosteroids (e.g. clobetasol, triamcinolone, betamethasone, hydrocortisone, halobetasol, diflorasone, mometasone, halcinonide, fluticasone), systemic glucocorticosteroids (e.g. prednisone, dexamethasone) and antihistamines (e.g. hydroxyzine, loratadine, cetirizine, diphenhydramine, cyproheptadine, fexofenadine).

### Hand dermatitis

The methods, compositions, and kits of the invention may be used for the treatment of hand dermatitis. If desired, one or more hand dermatitis agents typically used to treat hand dermatitis may be used in the methods, composition, and kits of the invention. Such agents include topical and systemic non-steroidal immunophilin-dependent immunosuppressants (e.g. cyclosporine, tacrolimus, pimecrolimus, ABT-281, and ISAtx247), topical glucocortieosteroids (e.g. clobetasol, triamcinolone, betamethasone, hydrocortisone, halobetasol, diflorasone, mometasone, halcinonide, fluticasone), systemic glucocorticosteroids (e.g. prednisone, dexamethasone) antihistamines (e.g. hydroxyzine, loratadine, cetirizine, diphenhydramine, cyproheptadine, fexofenadine), and emollients, ointments, humectants, and lotions.

### Actinic keratosis

The methods, compositions, and kits of the invention may be used for the treatment of actinic keratosis. If desired, one or more hand dermatitis agents typically used to treat hand dermatitis may be used in the methods, composition, and kits of the invention. Such agents include chemotherapeutic agents (e.g. 5-fluorouracil, imiquimod), non-steroid inflammatory agents (e.g. diclofenac), topical retinoids (e.g. adapalene), and photodynamic therapy using topical aminolevulinic acid.

### Basal cell carcinoma

The methods, compositions, and kits of the invention may be used for the treatment of basal cell carcinoma. If desired, one or more basal cell carcinoma agents typically used to treat basal cell carcinoma may be used in the methods, composition, and kits of the invention. Such agents include chemotherapeutic agents (e.g. 5-fluorouracil, imiquimod).

### Chronic obstructive pulmonary disease

In one embodiment, the methods, compositions, and kits of the invention are used for the treatment of chronic obstructive pulmonary disease (COPD). If desired, one or more agents typically used to treat COPD may be used in the methods, compositions, and kits of the invention. Such agents include xanthines (e.g., theophylline), anticholinergic compounds (e.g., ipratropium, tiotropium), biologics, small molecule immunomodulators, and beta receptor agonists/bronchodilators (e.g., ibuterol sulfate, bitolterol mesylate, epinephrine, formoterol fumarate, isoproteronol, levalbuterol hydrochloride, metaproterenol sulfate, pirbuterol acetate, salmeterol xinafoate, and terbutaline).

### Inflammatory bowel disease

The methods, compositions, and kits of the invention may be used for the treatment of inflammatory bowel disease. If desired, one or more agents typically used to treat inflammatory bowel disease may be used in the methods, compositions, and kits of the invention. Such agents include biologics (e.g., inflixamab, adelimumab, and CDP-870), small molecule immunomodulators (e.g., VX 702, SCIO 469, doramapimod, RO 30201195, SCIO 323, DPC 333, pranalcasan, mycophenolate, and merimepodib), non-steroidal immunophilin-dependent immunosuppressants (e.g., cyclosporine, tacrolimus, pimecrolimus, ABT-281, and ISAtx247), 5-amino salicylic acid (e.g., mesalamine, sulfasalazine, balsalazide disodium, and olsalazine sodium), DMARDs (e.g., methotrexate and azathioprine) and alosetron.

### Rheumatoid arthritis

The methods, compositions, and kits of the invention may be used for the treatment of rheumatoid arthritis. If desired, one or more agents typically used to treat rheumatoid arthritis may be used in the methods, compositions, and kits of the invention. Such agents include NSAIDs (e.g., naproxen sodium, diclofenac sodium, diclofenac potassium, aspirin, sulindac, diflunisal, piroxicam, indomethacin, ibuprofen, nabumetone, choline magnesium trisalicylate, sodium salicylate, salicylsalicylic acid (salsalate), fenoprofen, flurbiprofen, ketoprofen, meclofenamate sodium, meloxicam, oxaprozin, sulindac, and tolmetin), COX-2 inhibitors (e.g., rofecoxib, celecoxib, valdecoxib, and lumiracoxib), biologics (e.g., inflixamab, adelimumab, etanercept, CDP-870, rituximab, and atlizumab), small molecule immunomodulators (e.g., VX 702, SCIO 469, doramapimod, RO 30201195, SCIO 323, DPC 333, pranalcasan, mycophenolate, and merimepodib), non-steroidal immunophilin-dependent immunosuppressants (e.g., cyclosporine, tacrolimus, pimecrolimus, ABT-281, and ISAtx247), 5-amino salicylic acid (e.g., mesalamine, sulfasalazine, balsalazide disodium, and olsalazine sodium), DMARDs (e.g., methotrexate, leflunomide, minocycline, auranofin, gold sodium thiomalate, aurothioglucose, and azathioprine), hydroxychloroquine sulfate, and penicillamine.

### Asthma

The methods, compositions, and kits of the invention may be used for the treatment of asthma. If desired, one or more agents typically used to treat asthma may be used in the methods, compositions, and kits of the invention. Such agents include beta 2 agonists/bronchodilators/leukotriene modifiers (e.g., zafirlukast, montelukast, and zileuton), biologics (e.g., omalizumab), small molecule immunomodulators, anticholinergic compounds, xanthines, ephedrine, guaifenesin, cromolyn sodium, nedocromil sodium, and potassium iodide.

### Osteoarthritis

The methods, compositions, and kits of the invention may be used for the treatment of osteoarthritis, or pain, tenderness, impairment in mobility, soft tissue swelling, or bony swelling associated therewith. If desired, one or more agents typically used to treat osteoarthritis may be used as a substitute for or in addition to a corticosteroid in the methods, compositions, and kits of the invention. Such agents include NSAIDs (e.g., naproxen sodium, diclofenac sodium, diclofenac potassium, aspirin, sulindac, diflunisal, piroxicam, indomethacin, ibuprofen, nabumetone, choline magnesium trisalicylate, sodium salicylate, salicylsalicylic acid (salsalate), fenoprofen, flurbiprofen, ketoprofen, meclofenamate sodium, meloxicam, oxaprozin, sulindac, and tolmetin), COX-2 inhibitors (e.g., rofecoxib, celecoxib, valdecoxib, and lumiracoxib), biologics (e.g., inflixamab, adelimumab, etanercept, CDP-870, rituximab, and atlizumab), small molecule immunomodulators (e.g., VX 702, SCIO 469, doramapimod, RO 30201195, SCIO 323, DPC 333, pranalcasan, mycophenolate, and merimepodib), DMARDs (e.g., methotrexate, leflunomide, minocycline, auranofin, gold sodium thiomalate, aurothioglucose, and azathioprine), xanthines (e.g., theobromine, theophylline, aminophylline, and caffeine), NsIDIs (e.g., cyclosporine, tacrolimus, pimecrolimus, and ISAtx247), vitamin D analogs (e.g., calcipotriol, tacalcitol, and maxacalcitol), psoralens (e.g., methoxsalen and trioxsalen), retinoids (e.g., tretinoin, isotretinoin, and acetretin), 5-amino salicylic acids (e.g., mesalamine, sulfasalazine, balsalazide disodium, and olsalazine sodium), hydroxychloroquine sulfate, penicillamine, or analogs thereof.

### Ophthalmic disorders

The methods, compositions, and kits of the invention may be used for the treatment of ophthalmic disorders. Unless the intended purpose of use is affected adversely, the prophylactic and therapeutic medicament of the present invention may contain or may be used together with other appropriate pharmacologically effective substances, for example, dipivefrin (e.g., dipivefrin hydrochloride ophthalmic 0.1%), anti-VEGF therapies (e.g., pegaptanib (MACUGEN) ranibizumab, anecortave, and squalamine lactate), photodynamic therapy (e.g., VISUDYNE (verteporfin)), corticosteroids (e.g., dexamethasone, prednisolone), NSAIDs (e.g., diclofenac sodium, pranoprofen), antiallergic agents (e.g., tranilast, ketotifen fumarate, sodium cromoglicate), antihistamines (e.g., diphenhydramine hydrochloride), glaucoma-treating agents (e.g., pilocarpine hydrochloride, physostigmine salicylate, timolol, isopropylunoprostone), artificial tears, antibiotics (e.g., gentamycin sulfate, fradiomycin sulfate, tobramycin, sulbenicillin, cefmenoxime, erythromycin, colistin, oxytetracycline, polymyxin B, chloramphenicol, micronomicin, dibekacin, sisomicin, sulfamethizole, sulfamethoxazole, ofloxacin, norfloxacin, lomefloxacin hydrochloride, enoxacin, ciprofloxacin hydrochloride, cinoxacin, sparfloxacin, tosufloxacin tosylate, nalidixic acid, pipemidic acid trihydrate, pipemidic acid, fleroxacin, levofloxacin), antiviral agents (e.g., idoxuridine, acyclovir), and antimycotic agents (e.g., pimaricin, fluconazole, miconazole, amphotericin B, flucytosine, itraconazole).

### Administration

In particular embodiments of any of the methods of the invention, the compounds are administered within 14 days of each other, within 10 days of each other, within five days of each other, within twenty-four hours of each other, or simultaneously. The compounds may be formulated together as a single composition, or may be formulated and administered separately. One or both compounds may be administered in a low dosage or in a high dosage, each of which is defined herein. It may be desirable to administer to the patient other compounds, such as a corticosteroid, humectants, NSAID (e.g., naproxen sodium, diclofenac sodium, diclofenac potassium, aspirin, sulindac, diflunisal, piroxicam, indomethacin, ibuprofen, nabumetone, choline magnesium trisalicylate, sodium salicylate, salicylsalicylic acid, fenoprofen, flurbiprofen, ketoprofen, meclofenamate sodium, meloxicam, oxaprozin, sulindac, and tolmetin), COX-2 inhibitor (e.g., rofecoxib, celecoxib, valdecoxib, and lumiracoxib), glucocorticoid receptor modulator, or DMARD. Combination therapies of the invention are especially useful for the treatment of immunoinflammatory disorders in combination with other agents - either biologics or small molecules - that modulate the immune response to positively affect disease. Such agents include those that deplete key inflammatory cells, influence cell adhesion, or influence cytokines involved in immune response. This last category includes both agents that mimic or increase the action of anti-inflammatory cytokines such as IL-10, as well as agents inhibit the activity of pro-inflammatory cytokines such as IL-6, IL-1, IL-2, IL-12, IL-15 or TNFα. Agents that inhibit TNFα include etanercept, adelimumab, infliximab, and CDP-870. In this example (that of agents blocking the effect of TNFα), the combination therapy reduces the production of cytokines, etanercept or infliximab act on the remaining fraction of inflammatory cytokines, providing enhanced treatment. Small molecule immunodulators include, e.g., p38 MAP kinase inhibitors such as VX 702, SCIO 469, doramapimod, RO 30201195, SCIO 323, TACE inhibitors such as DPC 333, ICE inhibitors such as pranalcasan, and IMPDH inhibitors such as mycophenolate and merimepodib.

Therapy according to the invention may be performed alone or in conjunction with another therapy and may be provided at home, the doctor's office, a clinic, a hospital's outpatient department, or a hospital. Treatment optionally begins at a hospital so that the doctor can observe the therapy's effects closely and make any adjustments that are needed, or it may begin on an outpatient basis. The duration of the therapy depends on the type of disease or disorder being treated, the age and condition of the patient, the stage and type of the patient's disease, and how the patient responds to the treatment. Additionally, a person having a greater risk of developing an inflammatory disease (e.g., a person who is undergoing age-related hormonal changes) may receive treatment to inhibit or delay the onset of symptoms.

Routes of administration for the various embodiments include, but are not limited to, topical, transdermal, nasal, and systemic administration (such as, intravenous, intramuscular, subcutaneous, inhalation, rectal, buccal, vaginal, intraperitoneal, intraarticular, ophthalmic, otic, or oral administration). As used herein, "systemic administration" refers to all nondermal routes of administration, and specifically excludes topical and transdermal routes of administration.

In combination therapy, the dosage and frequency of administration of each component of the combination can be controlled independently. For example, one compound may be administered three times per day, while the second compound may be administered once per day. Combination therapy may be given in on-and-off cycles that include rest periods so that the patient's body has a chance to recover from any as yet unforeseen side effects. The compounds may also be formulated together such that one administration delivers both compounds.

Each compound of the combination may be formulated in a variety of ways that are known in the art. For example, the first and second agents may be formulated together or separately. Desirably, the first and second agents are formulated together for the simultaneous or near simultaneous administration of the agents. Such co-formulated compositions can include the two drugs together in the same pill, ointment, cream, foam, capsule, liquid, etc. It is to be understood that, when referring to the formulation of combinations of the invention, the formulation technology employed is also useful for the formulation of the individual agents of the combination, as well as other combinations of the invention (e.g., a glucocorticoid receptor modulator in lieu of a corticosteroid combination). By using different formulation strategies for different agents, the pharmacokinetic profiles for each agent can be suitably matched.

The individually or separately formulated agents can be packaged together as a kit. Non-limiting examples include kits that contain, e.g., two pills, a pill and a powder, a suppository and a liquid in a vial, two topical creams, ointments, foams etc. The kit can include optional components that aid in the administration of the unit dose to patients, such as vials for reconstituting powder forms, syringes for injection, customized IV delivery systems, inhalers, etc. Additionally, the unit dose kit can contain instructions for preparation and administration of the compositions. The kit may be manufactured as a single use unit dose for one patient, multiple uses for a particular patient (at a constant dose or in which the individual compounds may vary in potency as therapy progresses); or the kit may contain multiple doses suitable for administration to multiple patients ("bulk packaging"). The kit components may be assembled in cartons, blister packs, bottles, tubes, and the like.

### Topical formulations

For the prophylaxis and/or treatment of inflammatory dermatoses, the combinations of the invention are, desirably, formulated for topical administration. Topical formulations which can be used with the combinations of the invention include, without limitation, creams, foams, lotions, gels, sticks, sprays, solutions (e.g., for soaking, as with a bath salt), and ointments.

Any conventional pharmacologically and cosmetically acceptable vehicles may be used. For example, the compounds may also be administered in liposomal formulations that allow compounds to enter the skin. Such liposomal formulations are described in U.S. Patent Nos. 5,169,637; 5,000,958; 5,049,388; 4,975,282; 5,194,266; 5,023,087; 5,688,525; 5,874,104; 5,409,704; 5,552,155; 5,356,633; 5,032,582; 4,994,213; and PCT Publication No. WO 96/40061. Examples of other appropriate vehicles are described in U.S. Patent No. 4,877,805 and EP Publication No. 0586106A1. Suitable vehicles of the invention may also include mineral oil, petrolatum, polydecene, stearic acid, isopropyl myristate, polyoxyl 40 stearate, stearyl alcohol, or vegetable oil.

The formulations can include various conventional colorants, fragrances, thickeners (e.g., xanthan gum), preservatives, emollients (e.g., hydrocarbon oils, waxes, or silicones), demulcents, solubilizing excipients, dispersants, penetration enhancers, plasticizing agents, preservatives, stabilizers, demulsifiers, wetting agents, emulsifiers, moisturizers, astringents, deodorants, and the like can be added to provide additional benefits and improve the feel and/or appearance of the topical preparation.

Where one drug has poor solubility in water at physiological pH, one or more solubilizing excipients may be a necessary component in the topical formulations.

Solubilization is taken to mean an improvement in the solubility by virtue of surface-active compounds that can convert substances that are insoluble or virtually insoluble in water into clear, or opalescent, aqueous solutions without changing the chemical structure of these substances in the process.

The solubilizates formed are notable for the fact that the substance is present in dissolved form in the molecular associations, micelles, of the surface-active compounds, which form in aqueous solution. The resulting solutions appear optically clear to opalescent.

Solubilizing excipients that may be used in the formulations of the invention include, without limitation, compounds belonging to the following classes: polyethoxylated fatty acids, PEG-fatty acid diesters, PEG-fatty acid mono-ester and di-ester mixtures, polyethylene glycol glycerol fatty acid esters, alcohol-oil transesterification products, polyglycerized fatty acids, propylene glycol fatty acid esters, mixtures of propylene glycol esters and glycerol esters, mono- and diglycerides, sterol and sterol derivatives, polyethylene glycol sorbitan fatty acid esters, polyethylene glycol alkyl ethers, sugar esters, polyethylene glycol alkyl phenols, polyoxyethylene-polyoxypropylene block copolymers, sorbitan fatty acid esters, lower alcohol fatty acid esters, ionic surfactants, tocopherol esters, and sterol esters. Each of these classes of excipient are commercially available and well known to those in the field of formulations.

### Ophthalmic formulations

Ophthalmic formulations include but are not limited to ocular injections such as intravitreal, subtenons, subconjunctival, periocular, retrobulbar injections; topical ophthalmic aqueous solutions, such as suspensions, ointments, and gels; intraocular biodegradable and non-biodegradable implants; implants that are inserted through incisions made in the eye wall or sutured around the globe of the eye; tack for intraocular drug delivery; and bioadhesive ophthalmic inserts.

For topical ophthalmic administration the novel formulations of this invention may take the form of solutions, gels, ointments, suspensions or solid inserts, formulated so that a unit dosage comprises a therapeutically effective amount of each active component or some submultiple thereof.

Typical ophthalmologically acceptable carriers for the novel formulations are, for example, water, mixtures of water and water-miscible solvents such as lower alkanols or aralkanols, vegetable oils, polyalkylene glycols, petroleum based jelly, ethyl cellulose, ethyl oleate, carboxymethylcellulose, polyvinylpyrrolidone, isopropyl myristate and other conventionally employed acceptable carriers. The pharmaceutical preparation may also contain non-toxic auxiliary substances such as emulsifying, preserving, wetting agents, bodying agents and the like, as for example, polyethylene glycols 200, 3 00, 400 and 600, carbowaxes 1,000, 1,500, 4,000, 6,000 and 10,000, antibacterial components such as quaternary ammonium compounds, phenylmercuric salts known to have cold sterilizing properties and which are non-injurious in use, thimerosal, benzalkonium chloride, methyl and propyl paraben, benzyldodecinium bromide, benzyl alcohol, phenylethanol, buffering ingredients such as sodium chloride, sodium borate, sodium acetate, or gluconate buffers, and other conventional ingredients such as sorbitan monolaurate, triethanolamine, polyoxyethylene sorbitan monopalmitylate, dioctyl sodium sulfosuccinate, monothioglycerol, thiosorbitol, ethylenediamine tetra-acetic acid, and the like. Additionally, suitable ophthalmic vehicles can be used as carrier media for the present purpose including conventional phosphate buffer vehicle systems, isotonic boric acid vehicles, isotonic sodium chloride vehicles, isotonic sodium borate vehicles and the like.

The formulation may also include a gum such as gellan gum at a concentration of 0.1 to 2% by weight so that the aqueous eyedrops gel on contact with the eye, thus providing the advantages of a solid ophthalmic insert as described in U.S. Patent No. 4,861,760.

The pharmaceutical preparation may also be in the form of a solid insert such as one which after dispensing the drug remains essentially intact as described in U.S. Patent Nos. 4,256,108; 4,160,452; and 4,265,874; or a bio-erodible insert that either is soluble in lacrimal fluids, or otherwise disintegrates as described in U.S. Patent No. 4,287,175 or EPO publication 0077261.

Other ophthalmic formulations and delivery devices are described in U.S. Patent Nos. 4,014,335; 4,300,557; 5,098,443; 5,188,826; 5,378,475; 5,422,116; 5,424,078; 5,466,233; 5,725,493; 5,773,019; 5,773,021; 5,776,445; 5,814,635; 5,888,493; 6,235,781; 6,297,228; 6,372,245; 6,511,660; 6,579,519; 6,582,422; 6,713,081; 6,719,750; and U.S. Patent Application Publication Nos. 2002-0064513; 2003-0232089; and 2005-0234018.

### Kits

In general, kits of the invention contain a corticosteroid and/or an NsIDI. These compounds can be provided in the kit as separate compositions, or combined into a single composition. The kits of the invention can also contain instructions for the administration of both the corticosteroid and NsIDI.

Kits of the invention can also contain instructions for administering an additional pharmacologically acceptable substance (e.g., dipivefrin, anti-VEGF therapies, photodynamic therapy, NSAIDs, antiallergic agents, antihistamines, glaucoma-treating agents, artificial tears, antibiotics, antiviral agents, and antimycotic agents) with a corticosteroid and/or an NsIDI. This kit may contain any combination of the corticosteroid, NsIDI, and additional pharmaceutically acceptable substance (i.e., any one, two, or three of the above compounds).

### Dosages

The dosage of each compound of the claimed combinations depends on several factors, including: the administration method, the disease to be treated, the severity of the disease, whether the disease is to be treated or prevented, and the age, weight, and health of the person to be treated. Additionally, pharmacogenomic (the effect of genotype on the pharmacokinetic, pharmacodynamic or efficacy profile of a therapeutic) information about a particular patient may affect dosage used.

Continuous daily dosing with the combinations of the invention may not be required. A therapeutic regimen may require cycles, during which time a drug is not administered, or therapy may be provided on an as needed basis during periods of acute inflammation.

As described above, the compound in question may be administered orally in the form of tablets, capsules, elixirs or syrups, or rectally in the form of suppositories. The compound may also be administered topically in the form of foams, lotions, drops, creams, ointments, emollients, or gels. Parenteral administration of a compound is suitably performed, for example, in the form of saline solutions or with the compound incorporated into liposomes. In cases where the compound in itself is not sufficiently soluble to be dissolved, a solubilizer such as ethanol can be applied.

### Conjugates

If desired, the drugs used in any of the combinations described herein may be covalently attached to one another to form a conjugate of formula (VII).

(A)-(L)-(B) (VII)

In formula (VII), (A) is a "Drug 1" listed on Table 1A or Table 1B covalently tethered via a linker (L) to (B), the corresponding "Drug 2" listed on Table 1A or Table 1B.

Conjugates of the invention can be administered to a subject by any route and for the treatment of any disease described herein.

The conjugates of the invention can be prodrugs, releasing drug (A) and drug (B) upon, for example, cleavage of the conjugate by intracellular and extracellular enzymes (e.g., amidases, esterases, and phosphatases). The conjugates of the invention can also be designed to largely remain intact in vivo, resisting cleavage by intracellular and extracellular enzymes. The degradation of the conjugate in vivo can be controlled by the design of linker (L) and the covalent bonds formed with drug (A) and drug (B) during the synthesis of the conjugate.

Conjugates can be prepared using techniques familiar to those skilled in the art. For example, the conjugates can be prepared using the methods disclosed in G. Hermanson, Bioconjugate Techniques, Academic Press, Inc., 1996. The synthesis of conjugates may involve the selective protection and deprotection of alcohols, amines, ketones, sulfhydryls or carboxyl functional groups of drug (A), the linker, and/or drug (B). For example, commonly used protecting groups for amines include carbamates, such as tert-butyl, benzyl, 2,2,2-trichloroethyl, 2-trimethylsilylethyl, 9-fluorenylmethyl, allyl, and m-nitrophenyl. Other commonly used protecting groups for amines include amides, such as formamides, acetamides, trifluoroacetamides, sulfonamides, trifluoromethanesulfonyl amides, trimethylsilylethanesulfonamides, and tert-butylsulfonyl amides. Examples of commonly used protecting groups for carboxyls include esters, such as methyl, ethyl, tert-butyl, 9-fluorenylmethyl, 2-(trimethylsilyl)ethoxy methyl, benzyl, diphenylmethyl, O-nitrobenzyl, orthoesters, and halo-esters. Examples of commonly used protecting groups for alcohols include ethers, such as methyl, methoxymethyl, methoxyethoxymethyl, methylthiomethyl, benzyloxymethyl, tetrahydropyranyl, ethoxyethyl, benzyl, 2-napthylmethyl, O-nitrobenzyl, P-nitrobenzyl, P-methoxybenzyl, 9-phenylxanthyl, trityl (including methoxy-trityls), and silyl ethers. Examples of commonly used protecting groups for sulfhydryls include many of the same protecting groups used for hydroxyls. In addition, sulfhydryls can be protected in a reduced form (e.g., as disulfides) or an oxidized form (e.g., as sulfonic acids, sulfonic esters, or sulfonic amides). Protecting groups can be chosen such that selective conditions (e.g., acidic conditions, basic conditions, catalysis by a nucleophile, catalysis by a lewis acid, or hydrogenation) are required to remove each, exclusive of other protecting groups in a molecule. The conditions required for the addition of protecting groups to amine, alcohol, sulfhydryl, and carboxyl functionalities and the conditions required for their removal are provided in detail in T.W. Green and P.G.M. Wuts, Protective Groups in Organic Synthesis (2nd Ed.), John Wiley & Sons, 1991 and P.J. Kocienski, Protecting Groups, Georg Thieme Verlag, 1994. Additional synthetic details are provided below.

### Linkers

The linker component of the invention is, at its simplest, a bond between drug (A) and drug (B), but typically provides a linear, cyclic, or branched molecular skeleton having pendant groups covalently linking drug (A) to drug (B). Thus, linking of drug (A) to drug (B) is achieved by covalent means, involving bond formation with one or more functional groups located on drug (A) and drug (B). Examples of chemically reactive functional groups which may be employed for this purpose include, without limitation, amino, hydroxyl, sulfhydryl, carboxyl, carbonyl, carbohydrate groups, vicinal diols, thioethers, 2-aminoalcohols, 2-aminothiols, guanidinyl, imidazolyl, and phenolic groups.

The covalent linking of drug (A) and drug (B) may be effected using a linker which contains reactive moieties capable of reaction with such functional groups present in drug (A) and drug (B). For example, an amine group of drug (A) may react with a carboxyl group of the linker, or an activated derivative thereof, resulting in the formation of an amide linking the two.

Examples of moieties capable of reaction with sulfhydryl groups include α-haloacetyl compounds of the type XCH₂CO- (where X=Br, Cl or I), which show particular reactivity for sulfhydryl groups, but which can also be used to modify imidazolyl, thioether, phenol, and amino groups as described by Gurd, Methods Enzymol. 11:532 (1967). N-Maleimide derivatives are also considered selective towards sulfhydryl groups, but may additionally be useful in coupling to amino groups under certain conditions. Reagents such as 2-iminothiolane (Traut et al., Biochemistry 12:3266 (1973)), which introduce a thiol group through conversion of an amino group, may be considered as sulfhydryl reagents if linking occurs through the formation of disulphide bridges.

Examples of reactive moieties capable of reaction with amino groups include, for example, alkylating and acylating agents. Representative alkylating agents include:
(i) α-haloacetyl compounds, which show specificity towards amino groups in the absence of reactive thiol groups and are of the type XCH₂CO-(where X=Cl, Br or I), for example, as described by Wong Biochemistry 24:5337 (1979);
(ii) N-maleimide derivatives, which may react with amino groups either through a Michael type reaction or through acylation by addition to the ring carbonyl group, for example, as described by Smyth et al., J. Am. Chem. Soc. 82:4600 (1960) and Biochem. J. 91:589 (1964);
(iii) aryl halides such as reactive nitrohaloaromatic compounds;
(iv) alkyl halides, as described, for example, by McKenzie et al., J. Protein Chem. 7:581 (1988);
(v) aldehydes and ketones capable of Schiff's base formation with amino groups, the adducts formed usually being stabilized through reduction to give a stable amine;
(vi) epoxide derivatives such as epichlorohydrin and bisoxiranes, which may react with amino, sulfhydryl, or phenolic hydroxyl groups;
(vii) chlorine-containing derivatives of s-triazines, which are very reactive towards nucleophiles such as amino, sufhydryl, and hydroxyl groups;
(viii) aziridines based on s-triazine compounds detailed above, e.g., as described by Ross, J. Adv. Cancer Res. 2:1 (1954), which react with nucleophiles such as amino groups by ring opening;
(ix) squaric acid diethyl esters as described by Tietze, Chem. Ber. 124:1215 (1991); and
(x) α-haloalkyl ethers, which are more reactive alkylating agents than normal alkyl halides because of the activation caused by the ether oxygen atom, as described by Benneche et al., Eur. J. Med. Chem. 28:463 (1993).

Representative amino-reactive acylating agents include:
(i) isocyanates and isothiocyanates, particularly aromatic derivatives, which form stable urea and thiourea derivatives respectively;
(ii) sulfonyl chlorides, which have been described by Herzig et al., Biopolymers 2:349 (1964);
(iii) acid halides;
(iv) active esters such as nitrophenylesters or N-hydroxysuccinimidyl esters;
(v) acid anhydrides such as mixed, symmetrical, or N-carboxyanhydrides;
(vi) other useful reagents for amide bond formation, for example, as described by M. Bodansky, Principles of Peptide Synthesis, Springer-Verlag, 1984;
(vii) acylazides, e.g. wherein the azide group is generated from a preformed hydrazide derivative using sodium nitrite, as described by Wetz et al., Anal. Biochem. 58:347 (1974); and
(viii) imidoesters, which form stable amidines on reaction with amino groups, for example, as described by Hunter and Ludwig, J. Am. Chem. Soc. 84:3491 (1962).

Aldehydes and ketones may be reacted with amines to form Schiff's bases, which may advantageously be stabilized through reductive amination. Alkoxylamino moieties readily react with ketones and aldehydes to produce stable alkoxamines, for example, as described by Webb et al., in Bioconjugate Chem. 1:96 (1990).

Examples of reactive moieties capable of reaction with carboxyl groups include diazo compounds such as diazoacetate esters and diazoacetamides, which react with high specificity to generate ester groups, for example, as described by Herriot, Adv. Protein Chem. 3:169 (1947). Carboxyl modifying reagents such as carbodiimides, which react through O-acylurea formation followed by amide bond formation, may also be employed.

It will be appreciated that functional groups in drug (A) and/or drug (B) may, if desired, be converted to other functional groups prior to reaction, for example, to confer additional reactivity or selectivity. Examples of methods useful for this purpose include conversion of amines to carboxyls using reagents such as dicarboxylic anhydrides; conversion of amines to thiols using reagents such as N-acetylhomocysteine thiolactone, S-acetylmercaptosuccinic anhydride, 2-iminothiolane, or thiol-containing succinimidyl derivatives; conversion of thiols to carboxyls using reagents such as α-haloacetates; conversion of thiols to amines using reagents such as ethylenimine or 2-bromoethylamine; conversion of carboxyls to amines using reagents such as carbodiimides followed by diamines; and conversion of alcohols to thiols using reagents such as tosyl chloride followed by transesterification with thioacetate and hydrolysis to the thiol with sodium acetate.

So-called zero-length linkers, involving direct covalent joining of a reactive chemical group of drug (A) with a reactive chemical group of drug (B) without introducing additional linking material may, if desired, be used in accordance with the invention.

Most commonly, however, the linker will include two or more reactive moieties, as described above, connected by a spacer element. The presence of such a spacer permits bifunctional linkers to react with specific functional groups within drug (A) and drug (B), resulting in a covalent linkage between the two. The reactive moieties in a linker may be the same (homobifunctional linker) or different (heterobifunctional linker, or, where several dissimilar reactive moieties are present, heteromultifunctional linker), providing a diversity of potential reagents that may bring about covalent attachment between drug (A) and drug (B).

Spacer elements in the linker typically consist of linear or branched chains and may include a C₁₋₁₀ alkyl, C₂₋₁₀ alkenyl, C₂₋₁₀ alkynyl, C₂₋₆ heterocyclyl, C₆₋₁₂ aryl, C₇₋₁₄ alkaryl, C₃₋₁₀ alkheterocyclyl, or C₁₋₁₀ heteroalkyl.

In some instances, the linker is described by formula (VIII):

G¹-(Z¹)ₒ-(Y¹)ᵤ-(Z²)ₛ-(R₃₀)-(Z³)ₜ-(Y²)ᵥ-(Z⁴)ₚ-G² (VIII)

In formula (VIII), G¹ is a bond between drug (A) and the linker; G² is a bond between the linker and drug (B); Z¹, Z², Z³, and Z⁴ each, independently, is selected from O, S, and NR₃₁; R₃₁ is hydrogen, C₁₋₄ alkyl, C₂₋₄ alkenyl, C₂₋₄ alkynyl, C₂₋₆ heterocyclyl, C₆₋₁₂ aryl, C₇₋₁₄ alkaryl, C₃₋₁₀ alkheterocyclyl, or C₁₋₇ heteroalkyl; Y¹ and Y² are each, independently, selected from carbonyl, thiocarbonyl, sulphonyl, or phosphoryl; o, p, s, t, u, and v are each, independently, 0 or 1; and R₃₀ is a C₁₋₁₀ alkyl, C₂₋₁₀ alkenyl, C₂₋₁₀ alkynyl, C₂₋₆ heterocyclyl, C₆₋₁₂ aryl, C₇₋₁₄ alkaryl, C₃₋₁₀ alkheterocyclyl, or C₁₋₁₀ heteroalkyl, or a chemical bond linking G¹-(Z¹)ₒ-(Y¹)ᵤ(Z²)ₛ- to -(Z³)ₜ-(Y²)ᵥ-(Z⁴)ₚ-G²_{.} Examples of homobifunctional linkers useful in the preparation of conjugates of the invention include, without limitation, diamines and diols selected from ethylenediamine, propylenediamine and hexamethylenediamine, ethylene glycol, diethylene glycol, propylene glycol, 1,4-butanediol, 1,6-hexanediol, cyclohexanediol, and polycaprolactone diol.

### Additional applications

The compounds of the invention can be employed in immunomodulatory or mechanistic assays to determine whether other combinations, or single agents, are as effective as the combination in inhibiting secretion or production of proinflammatory cytokines or modulating immune response using assays generally known in the art, examples of which are described herein. After a suitable time, the cells are examined for cytokine secretion or production or other suitable immune response. The relative effects of the combinations versus each other, and versus the single agents are compared, and effective compounds and combinations are identified.

The combinations of the invention are also useful tools in elucidating mechanistic information about the biological pathways involved in inflammation. Such information can lead to the development of new combinations or single agents for inhibiting inflammation caused by proinflammatory cytokines. Methods known in the art to determine biological pathways can be used to determine the pathway, or network of pathways affected by contacting cells stimulated to produce proinflammatory cytokines with the compounds of the invention. Such methods can include, analyzing cellular constituents that are expressed or repressed after contact with the compounds of the invention as compared to untreated, positive or negative control compounds, and/or new single agents and combinations, or analyzing some other metabolic activity of the cell such as enzyme activity, nutrient uptake, and proliferation. Cellular components analyzed can include gene transcripts, and protein expression. Suitable methods can include standard biochemistry techniques, radiolabeling the compounds of the invention (e.g., ¹⁴C or ³H labeling), and observing the compounds binding to proteins, e.g. using 2d gels, gene expression profiling. Once identified, such compounds can be used in in vivo models to further validate the tool or develop new anti-inflammatory agents.

The following examples are to illustrate the invention. They are not meant to limit the invention in any way.

### Example 1

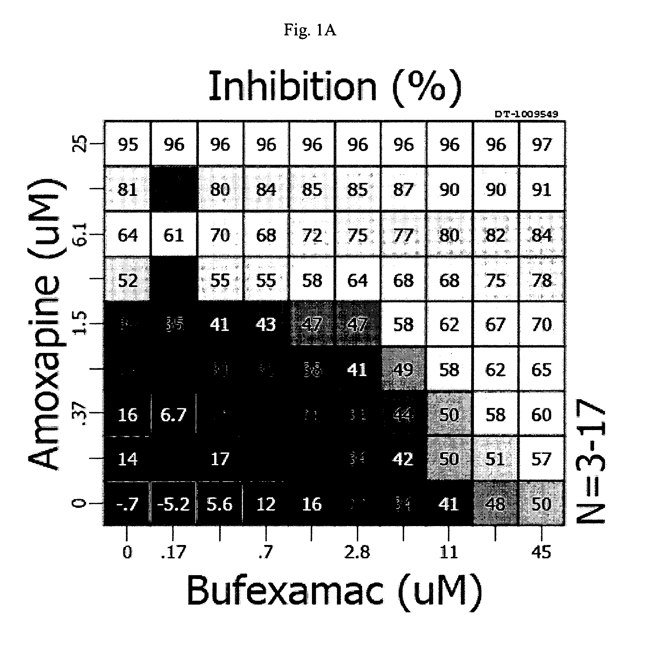

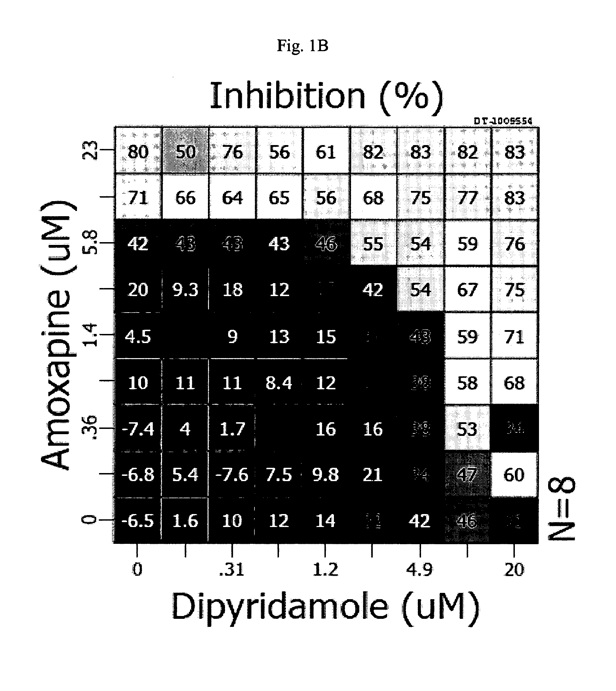

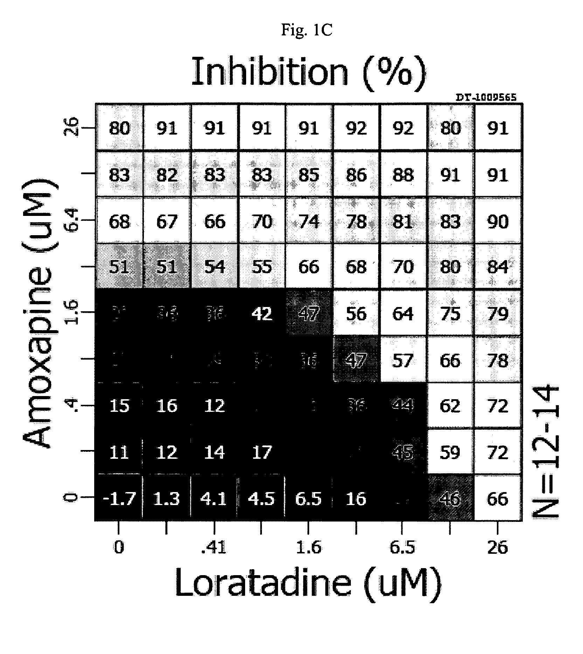

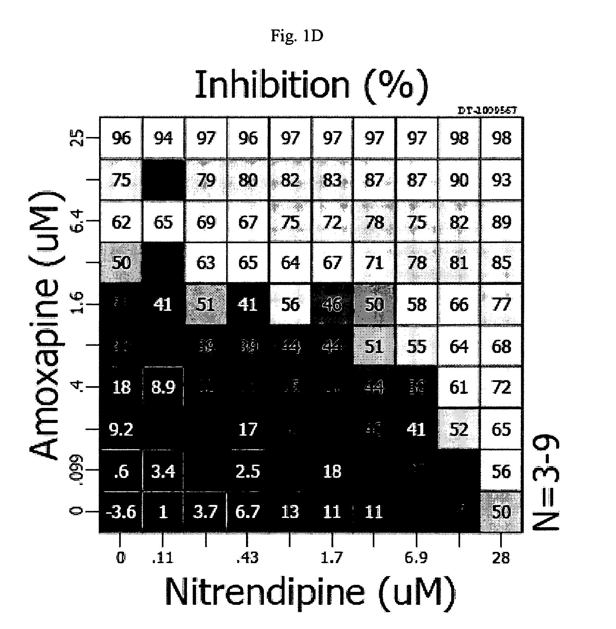

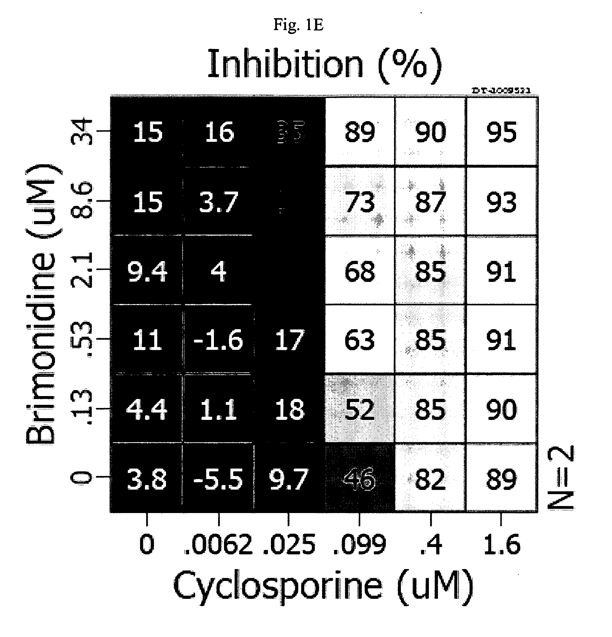

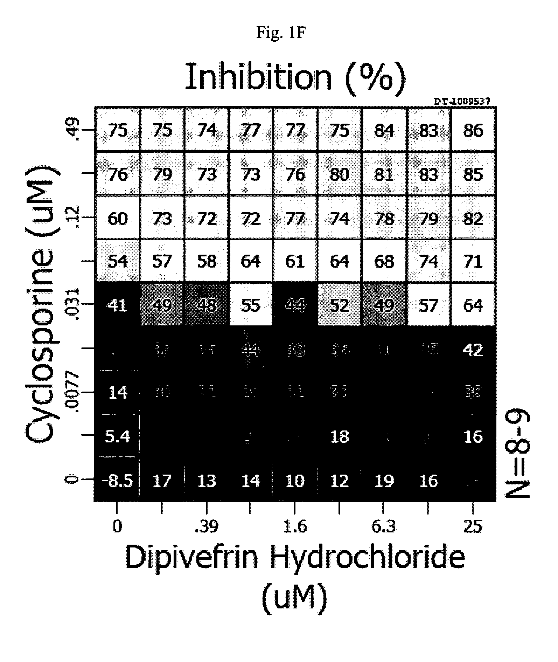

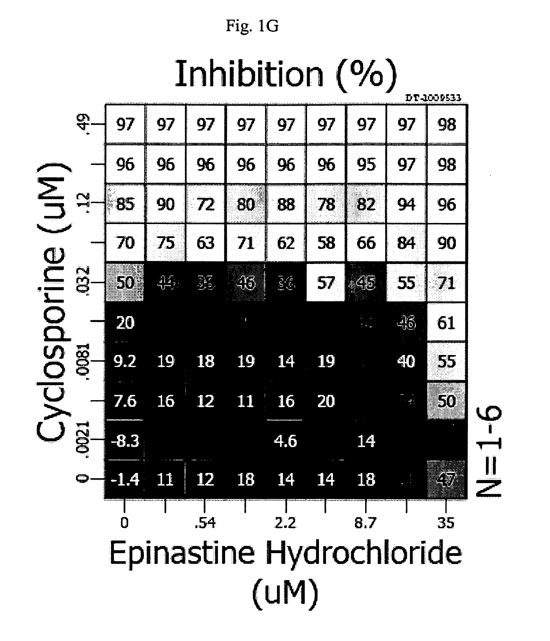

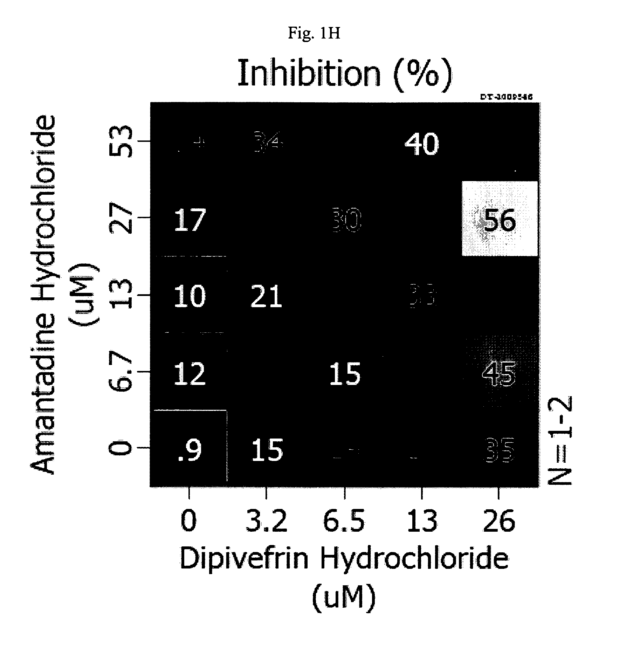

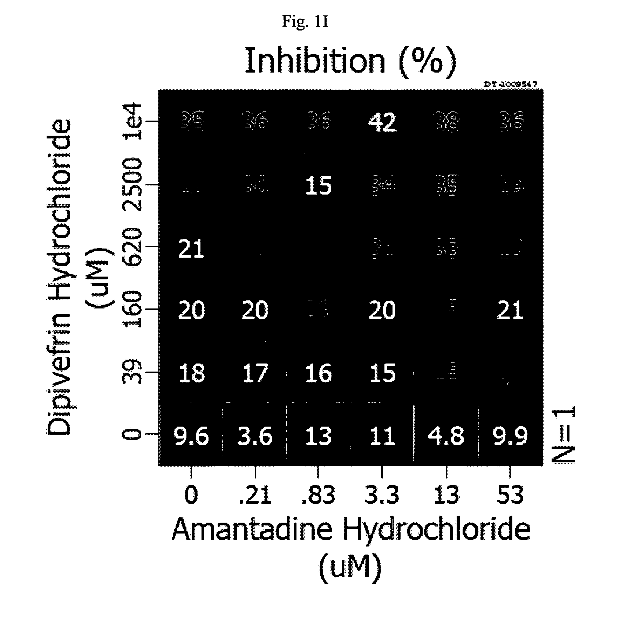

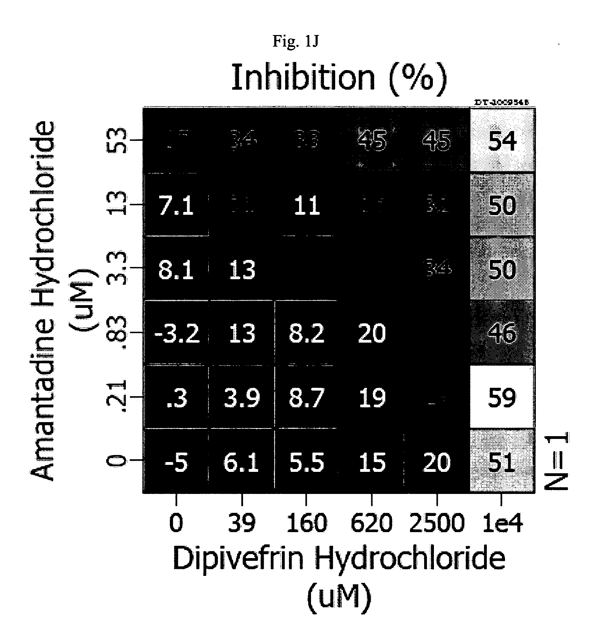

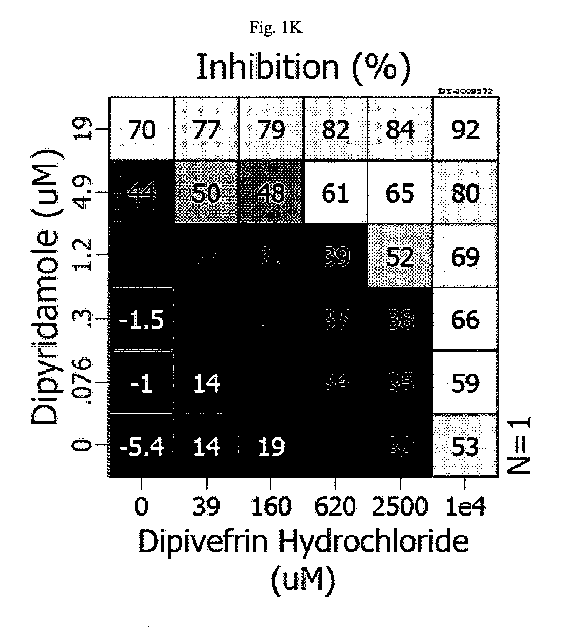

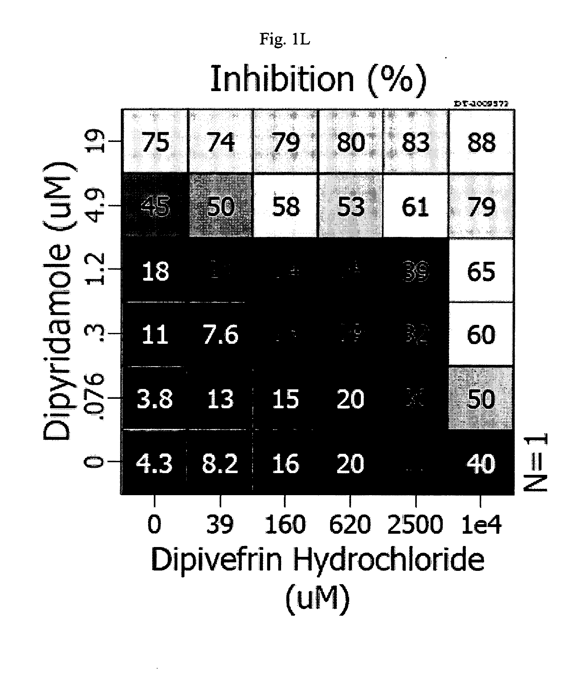

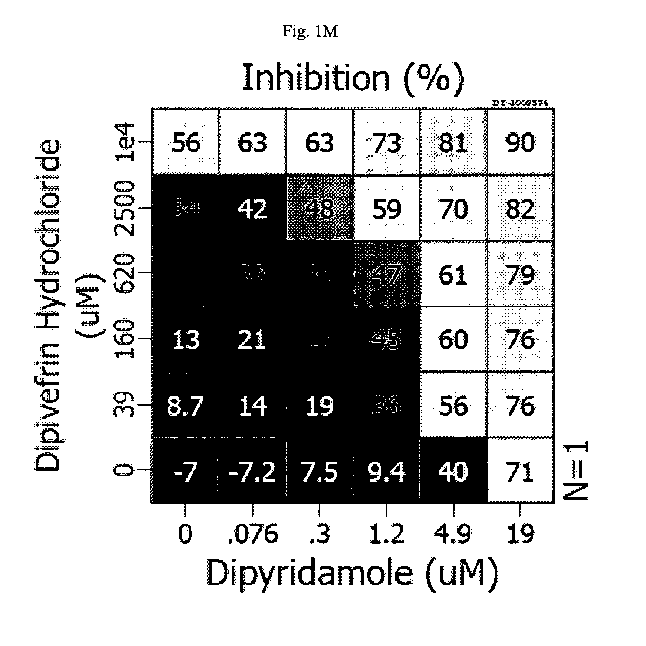

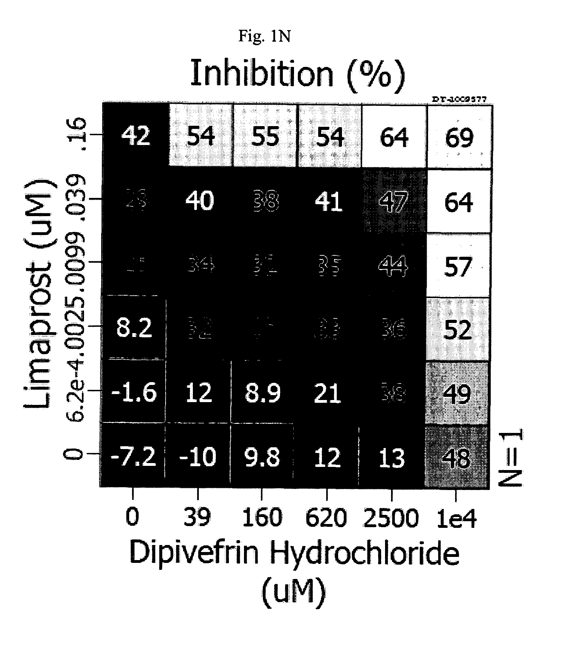

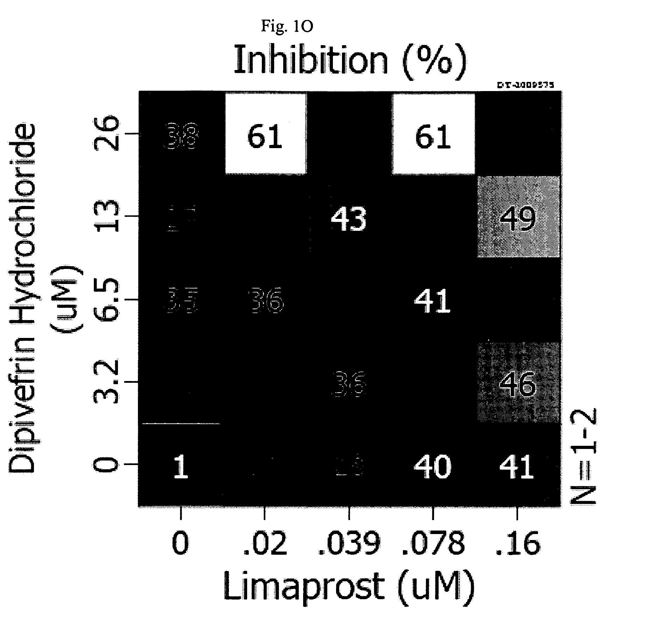

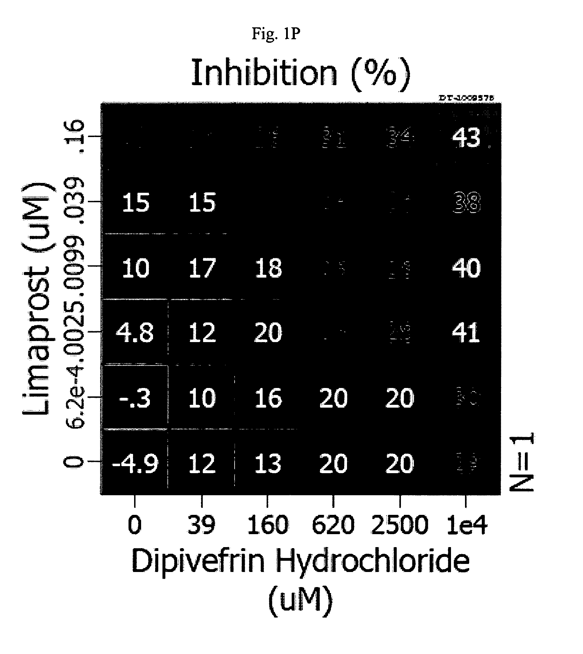

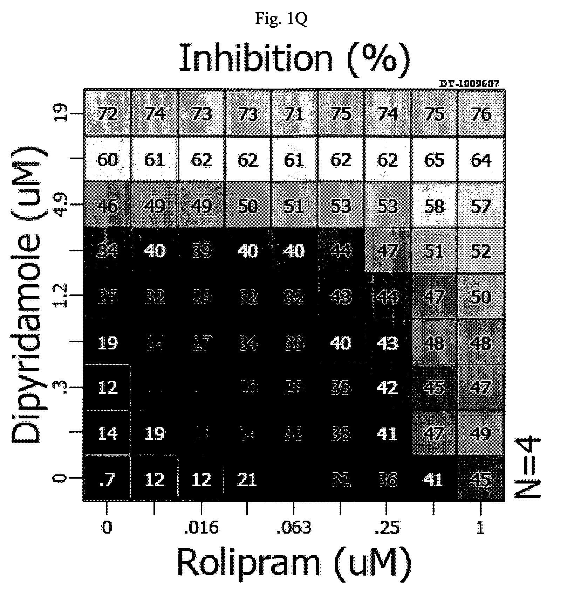

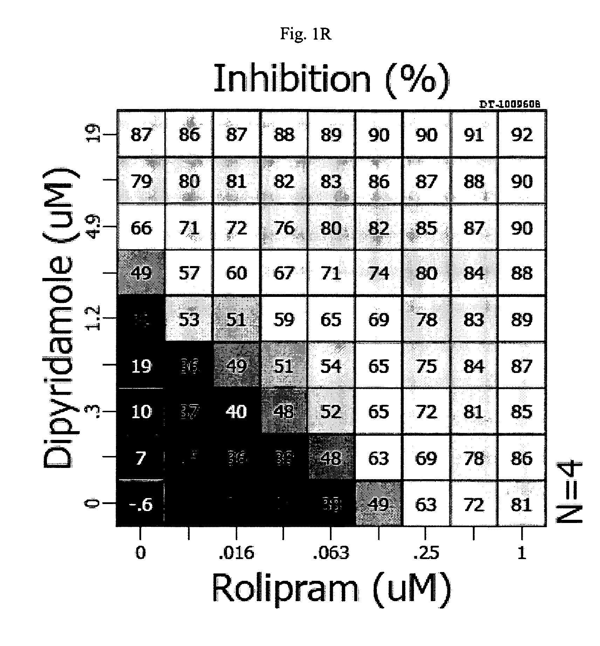

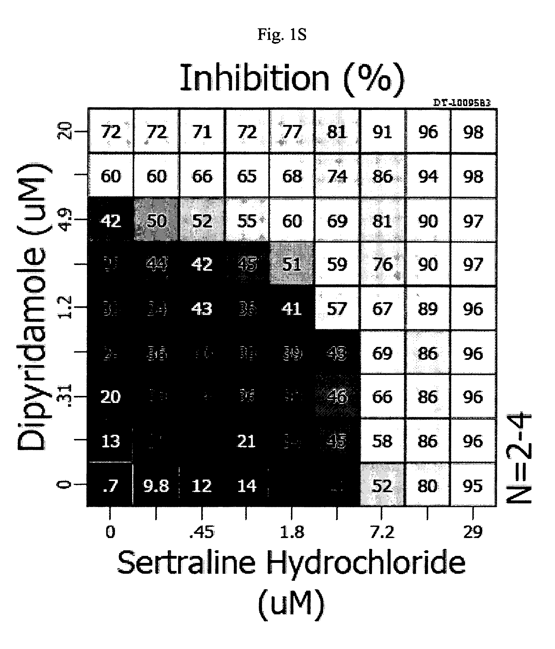

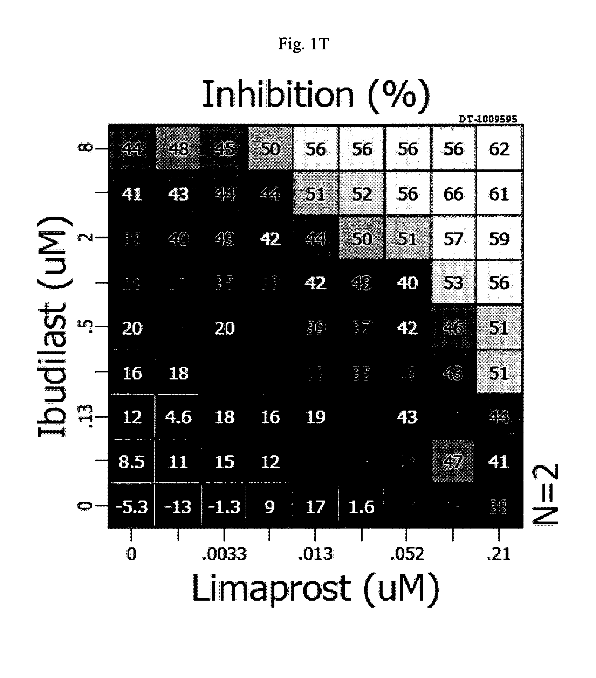

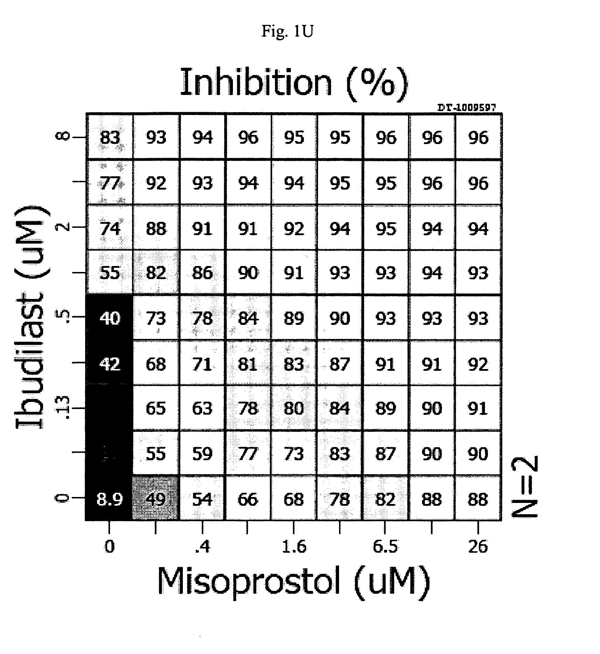

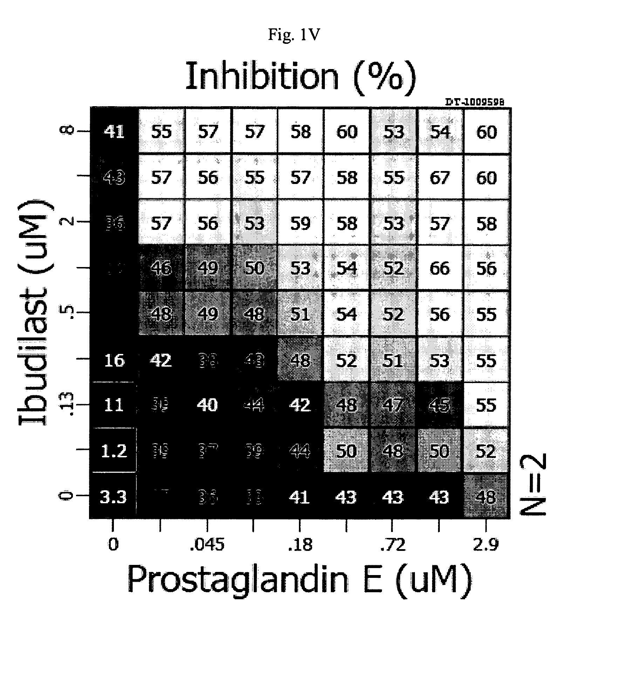

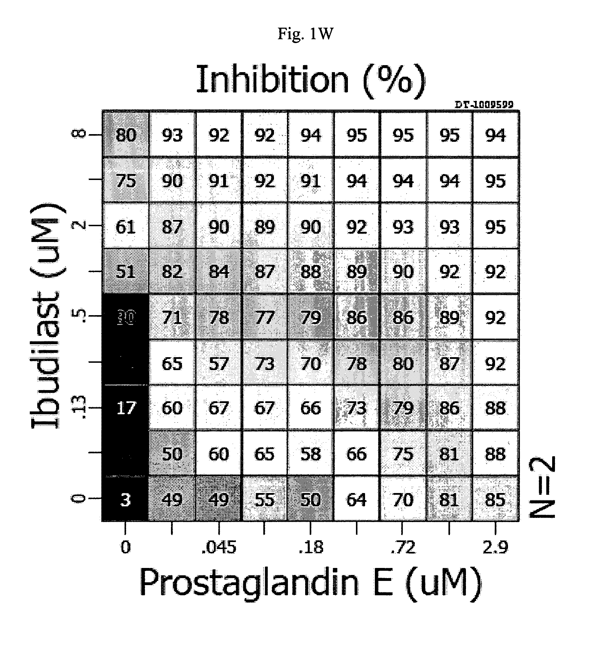

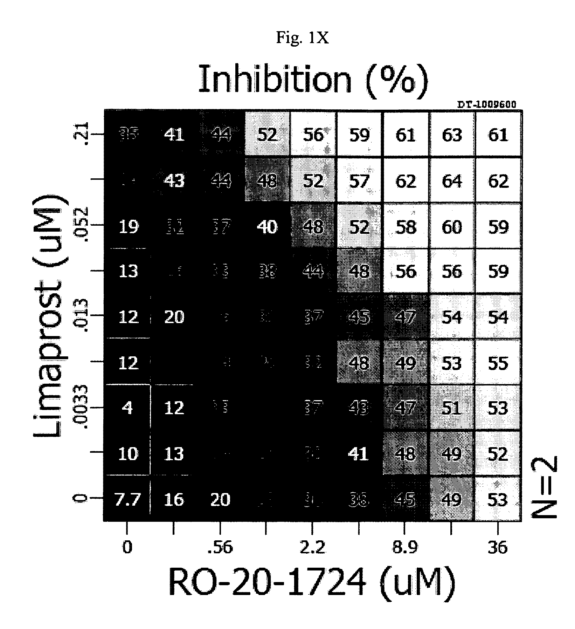

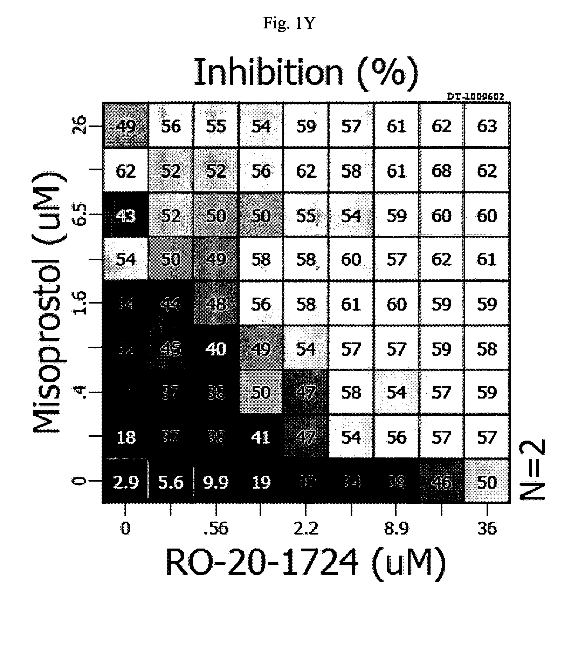

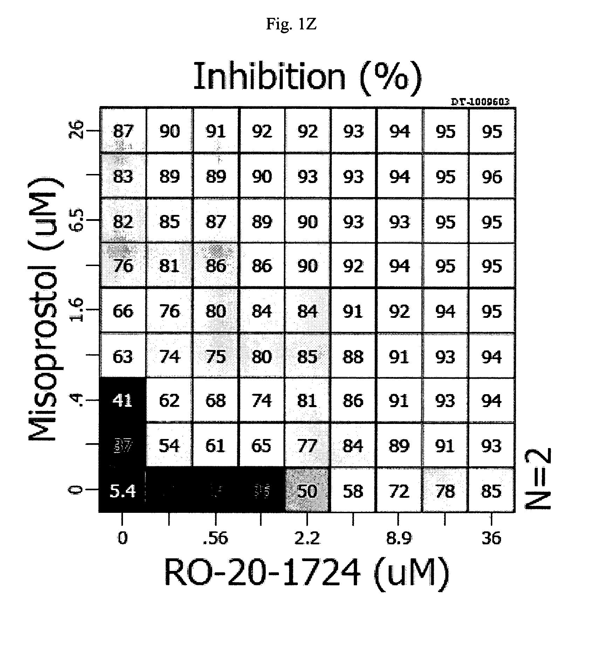

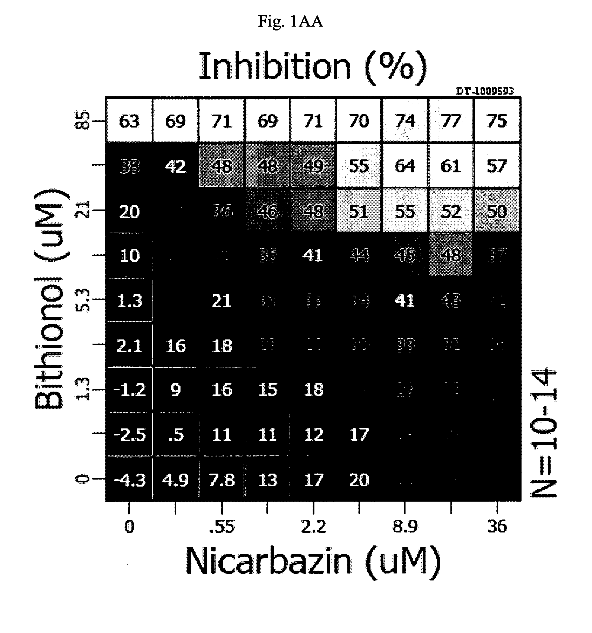

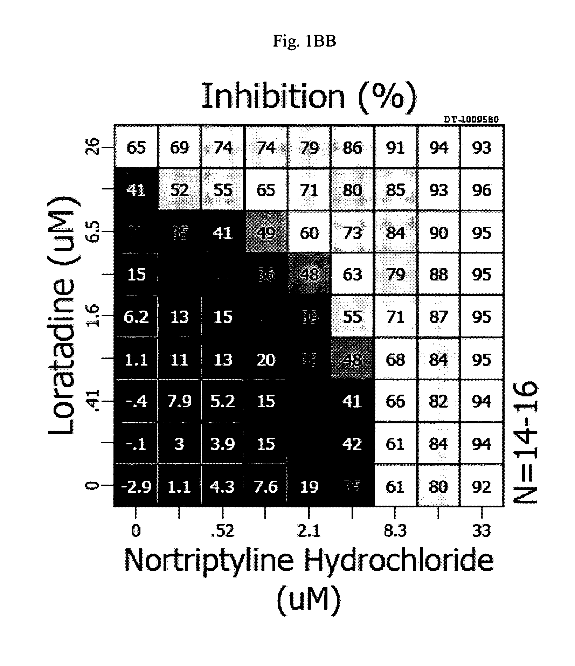

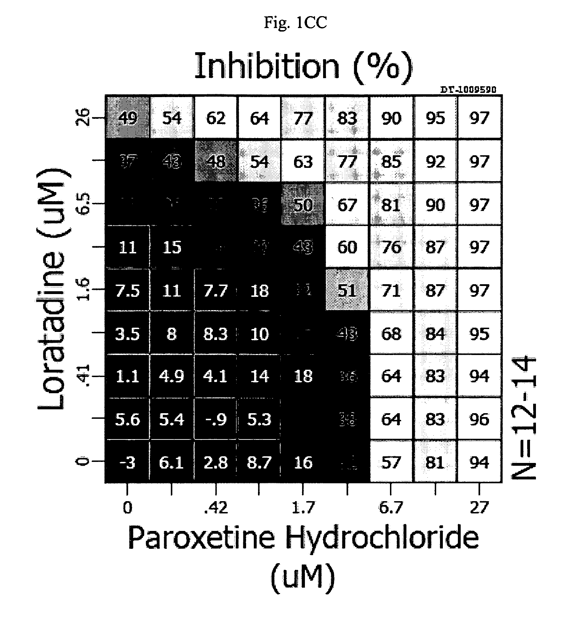

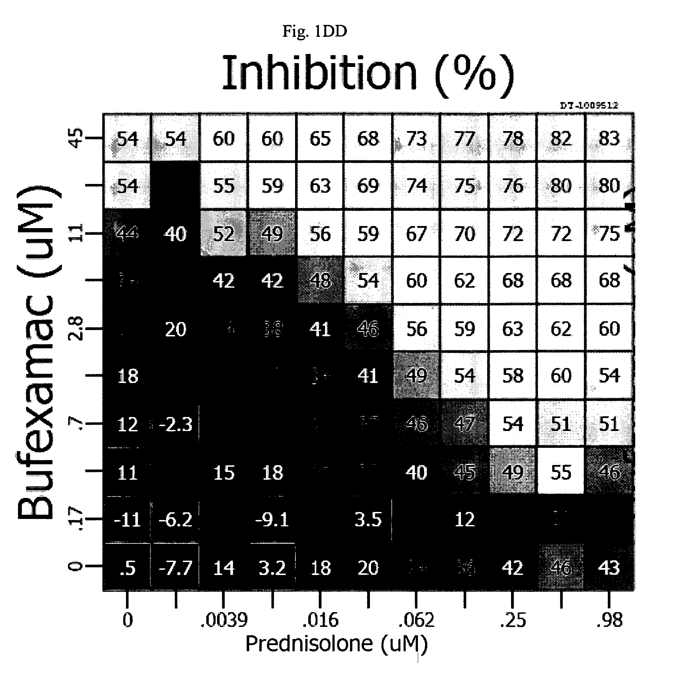

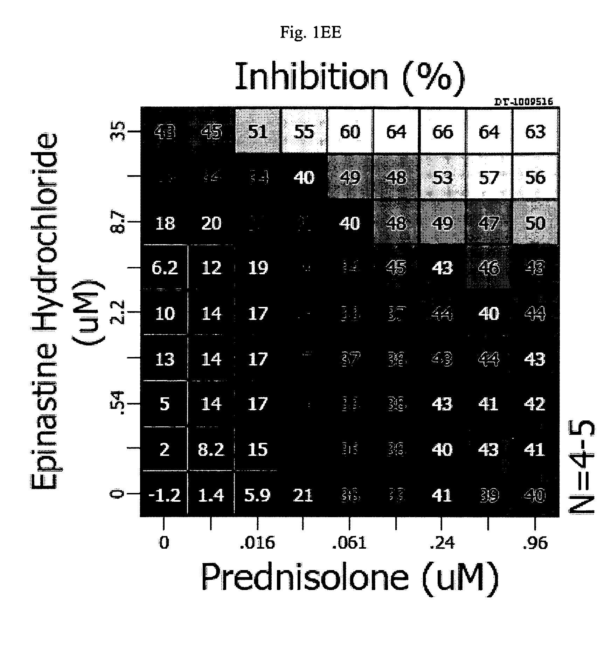

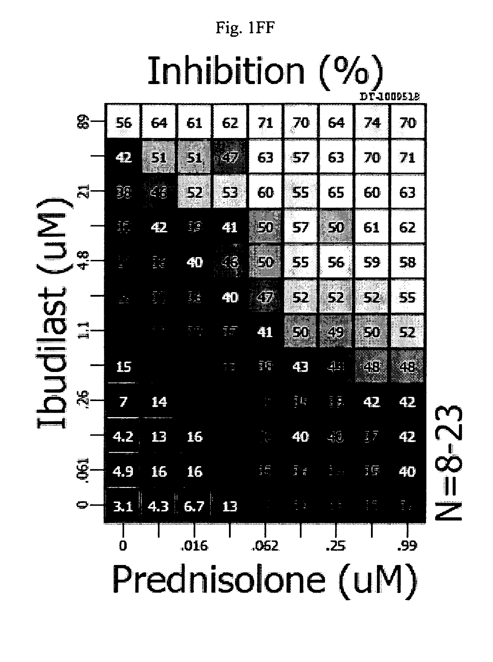

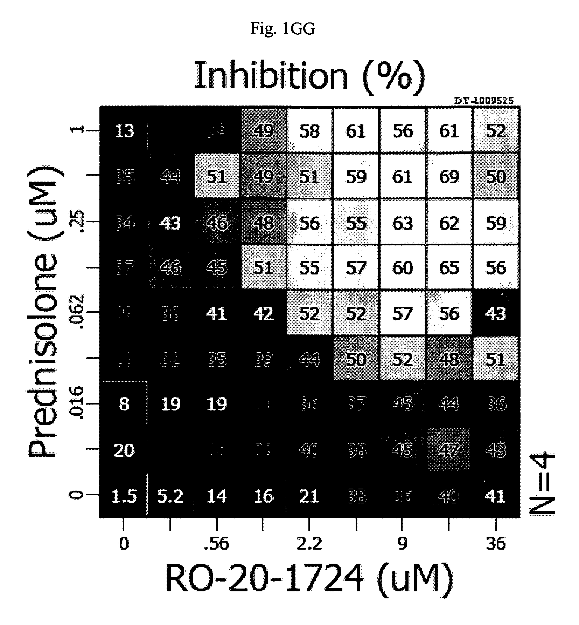

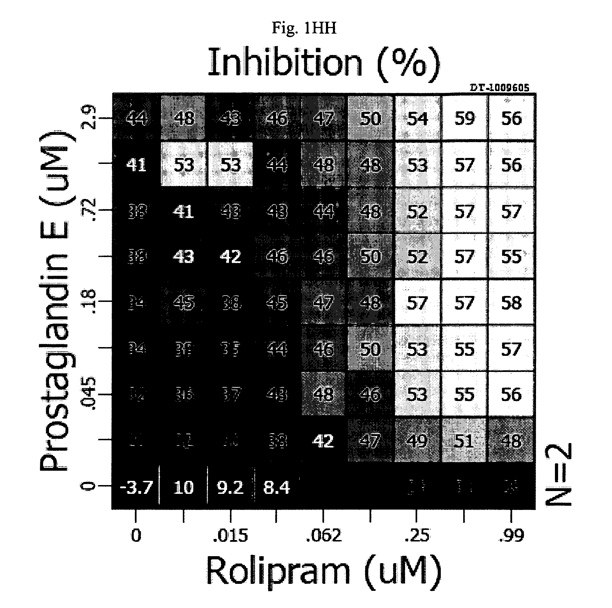

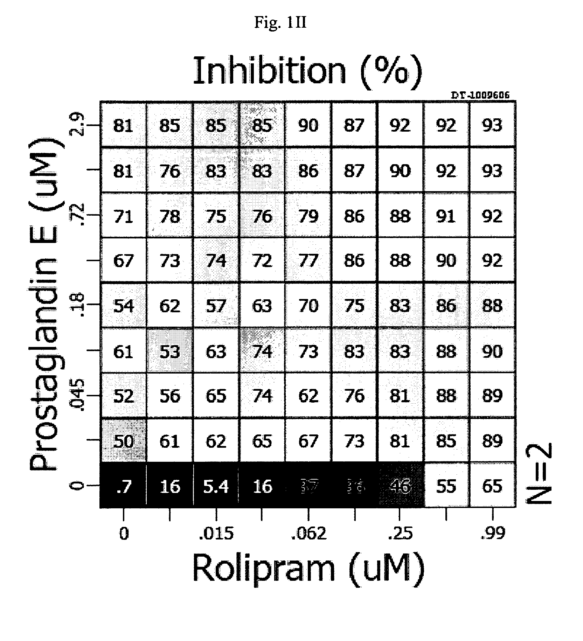

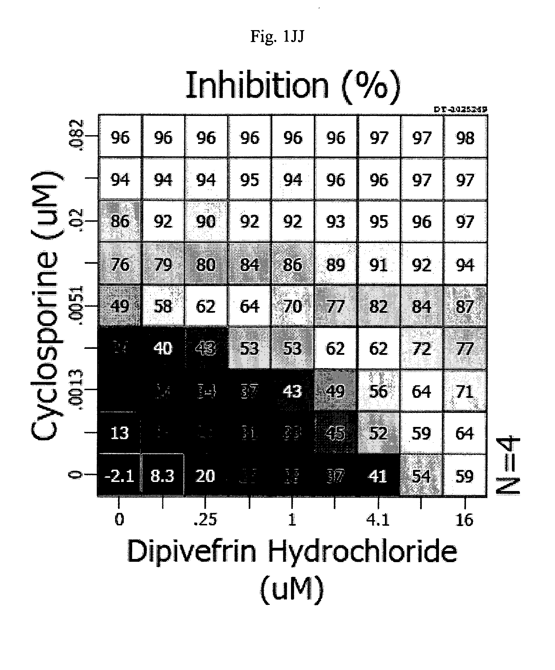

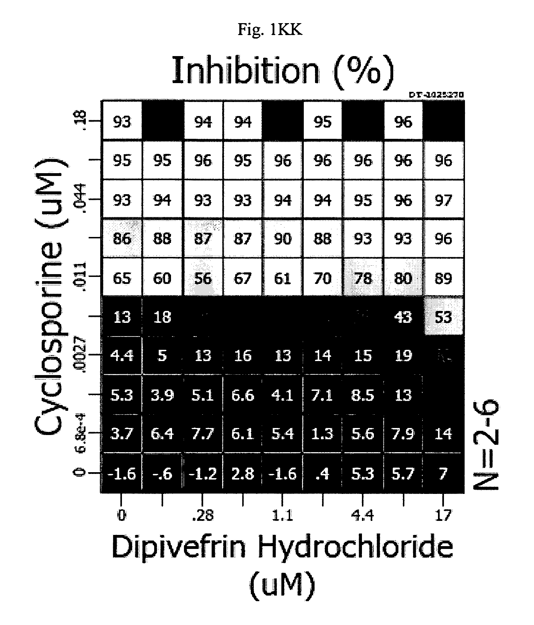

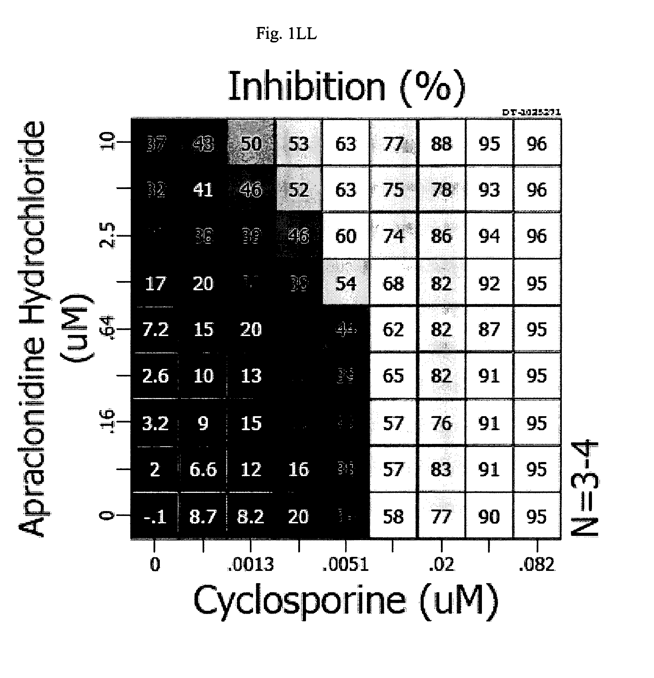

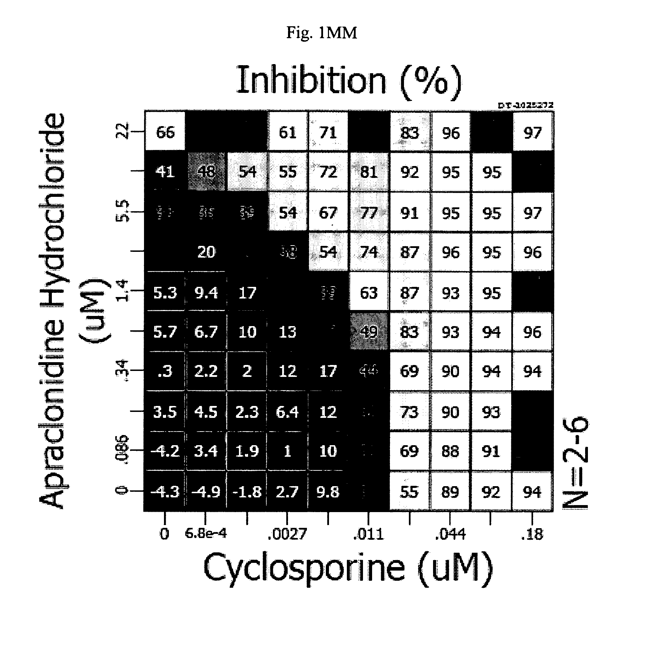

The effects of test compound combinations on TNFα secretion were assayed in white blood cells from human buffy coat stimulated with lipopolysaccharide or phorbol 12-myristate 13-acetate (PMA) and ionomycin as follows. The results from these experiments are set forth in Figs. 1A-1MM.

### Lipopolysaccharide (LPS)

A 100 µl suspension of diluted human white blood cells contained within each well of a polystyrene 3 84-well plate (NalgeNunc) was stimulated to secrete TNFα by treatment with a final concentration of 2 µg/mL lipopolysaccharide (Sigma L-4130). Various concentrations of each test compound were added at the time of stimulation. After 16-18 hours of incubation at 37°C in a humidified incubator, the plate was centrifuged and the supernatant transferred to a white opaque polystyrene 384-well plate (NalgeNunc, Maxisorb) coated with an anti-TNFα antibody (PharMingen, #551220). After a two-hour incubation, the plate was washed (Tecan PowerWasher 384) with PBS containing 0.1% Tween 20 and incubated for an additional one hour with another anti-TNFα antibody that was biotin labeled (PharMingen, #554511) and HRP coupled to strepavidin (PharMingen, #13047E). After the plate was.washed with 0.1% Tween 20/PBS, an HRP-luminescent substrate was added to each well and light intensity measured using a LJL Analyst plate luminometer.

### PMA/ionomycin

A 100 µl suspension of diluted human white blood cells contained within each well of a polystyrene 384-well plate (NalgeNunc) was stimulated to secrete TNFα by treatment with a final concentration of 10 ng/mL phorbol 12-myristate 13-acetate (Sigma, P-1585) and 750 ng/mL ionomycin (Sigma, I-0634). Various concentrations of each test compound were added at the time of stimulation. After 16-18 hours of incubation at 37°C in a humidified incubator, the plate was centrifuged and the supernatant transferred to a white opaque polystyrene 3 84-well plate (NalgeNunc, Maxisorb) coated with an anti-TNFα antibody (PharMingen, #551220). After a two-hour incubation, the plate was washed (Tecan PowerWasher 384) with PBS containing 0.1% Tween 20 and incubated for an additional one hour with another anti-TNFα antibody that was biotin labeled (PharMingen, #554511) and HRP coupled to strepavidin (PharMingen, #13047E). After the plate was washed with 0.1% Tween 20/PBS, an HRP-luminescent substrate was added to each well and light intensity measured using a LJL Analyst plate luminometer.

### Example 2

Both corticosteroids and NsIDIs suppress cytokine production in cell culture models of immune function. We tested the effect of the combination of various concentrations of NsIDIs and corticosteroids on cytokine production in a cell culture model of immune function. We propose that combinations that demonstrate synergistic or superaddative effects can be used to treat ophthalmic disorders at concentrations low enough to avoid undesired side effects.

### Assay for proinflammatory cytokine-suppressing activity

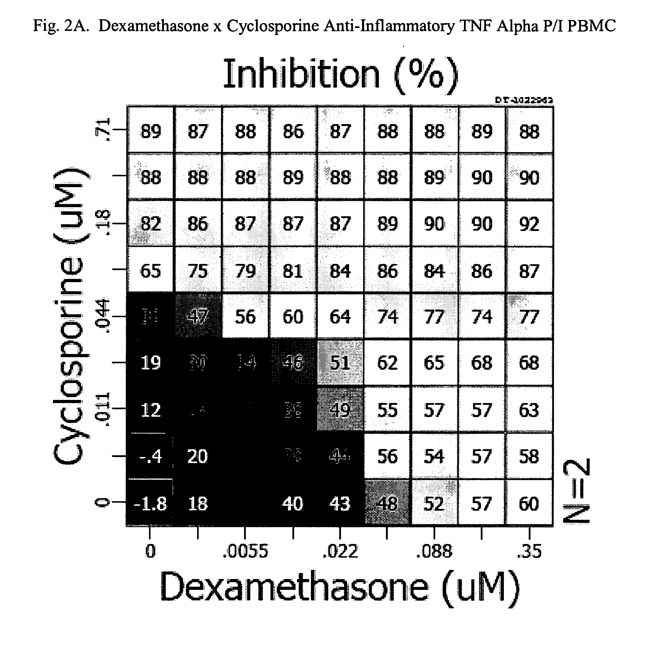

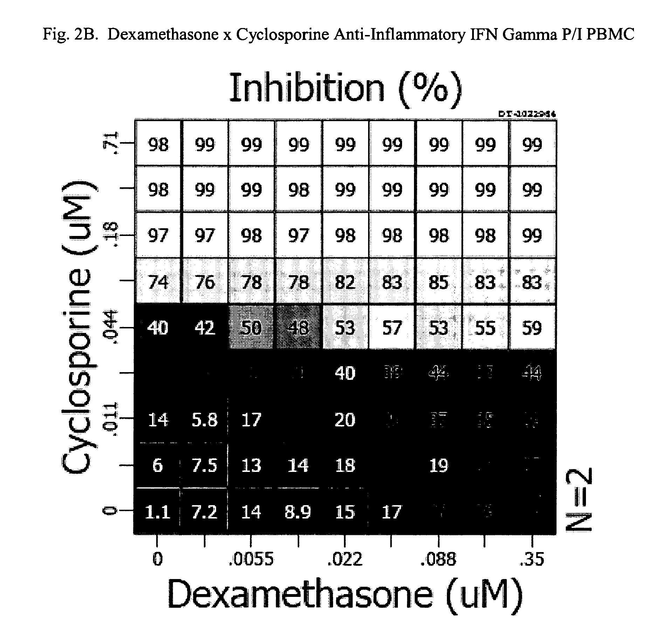

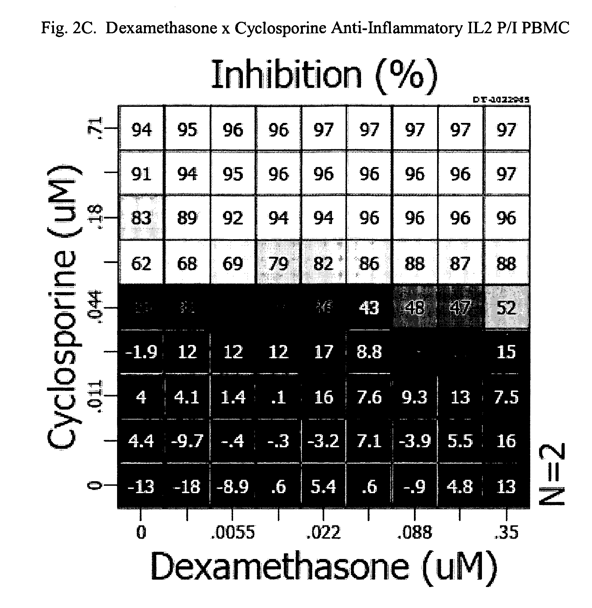

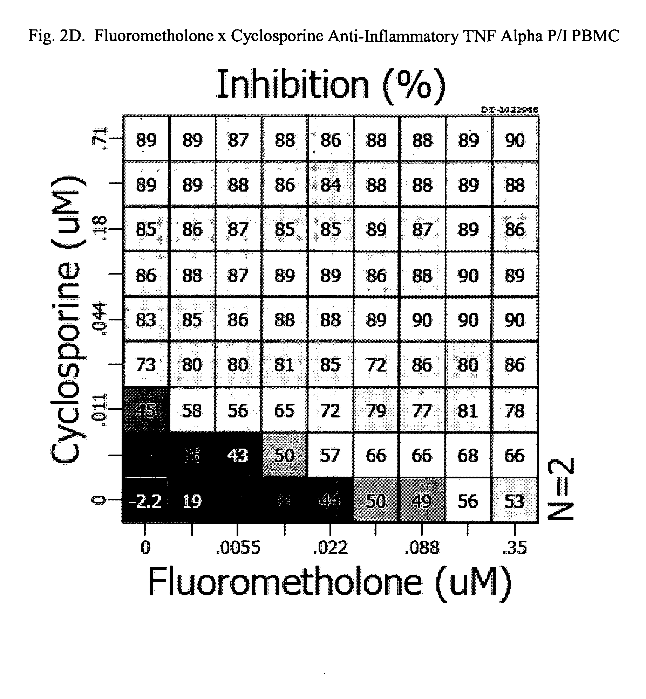

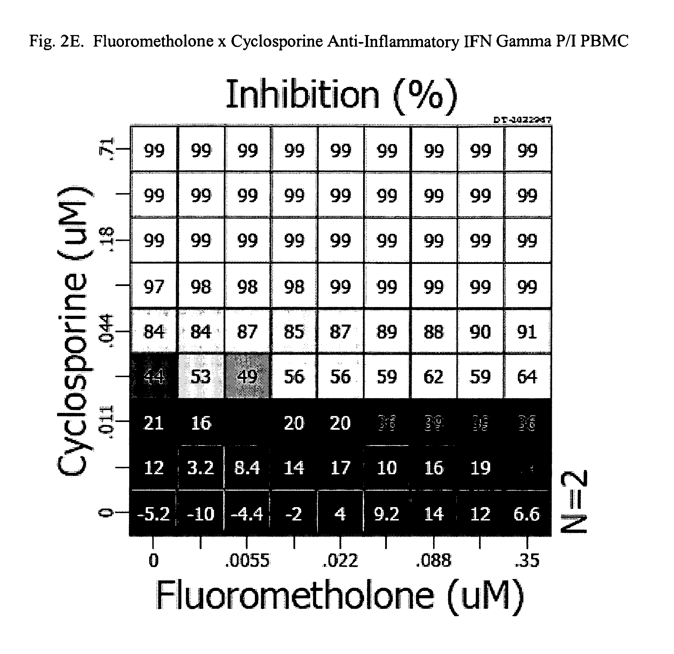

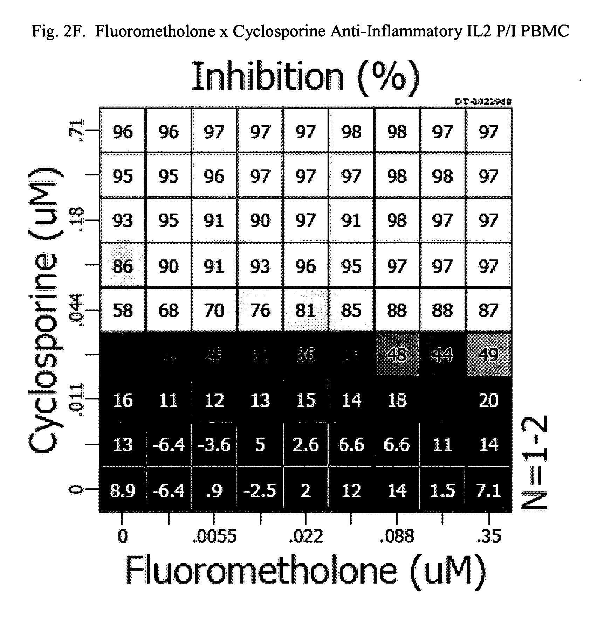

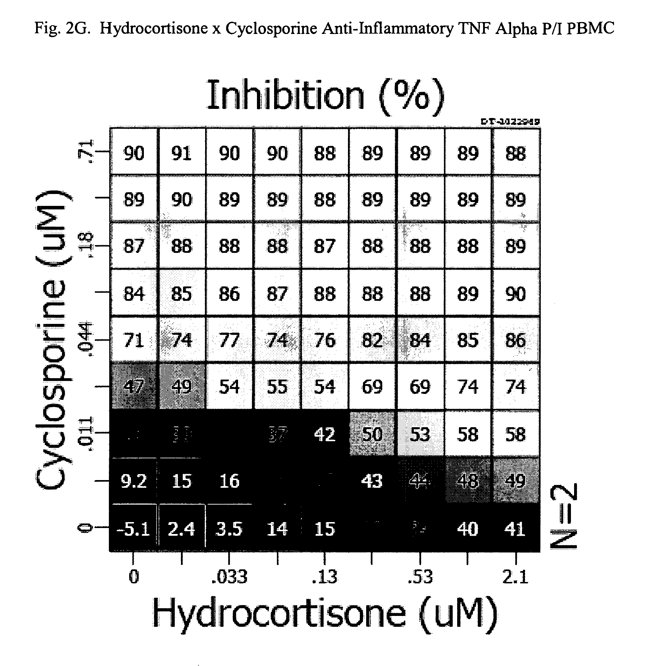

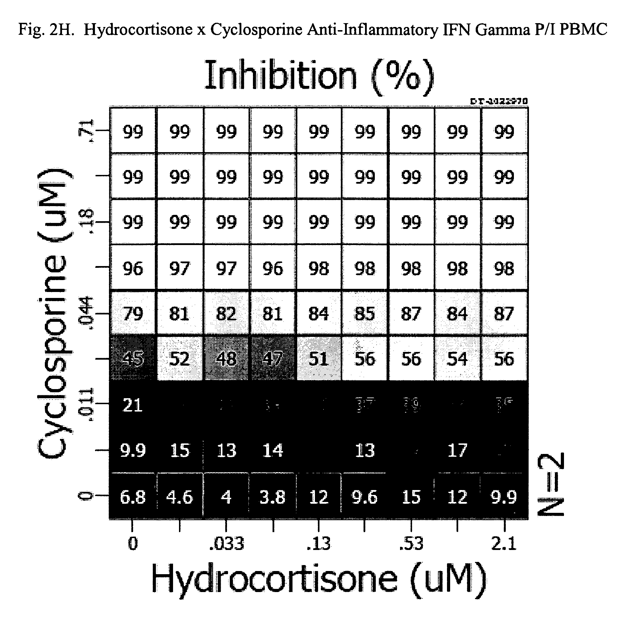

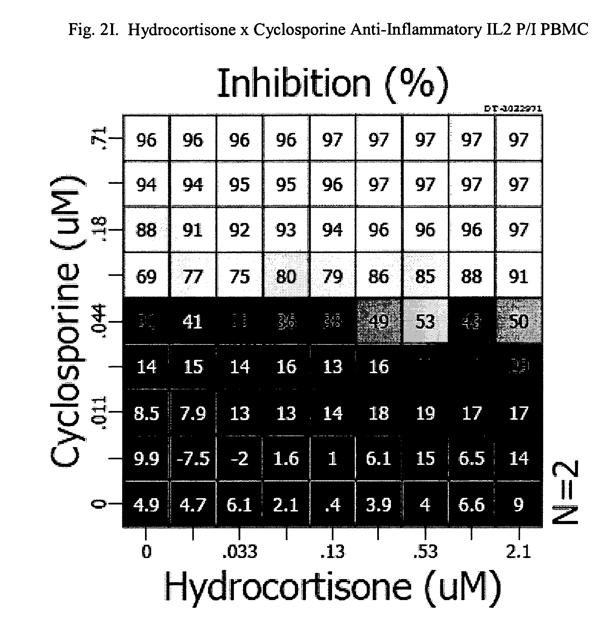

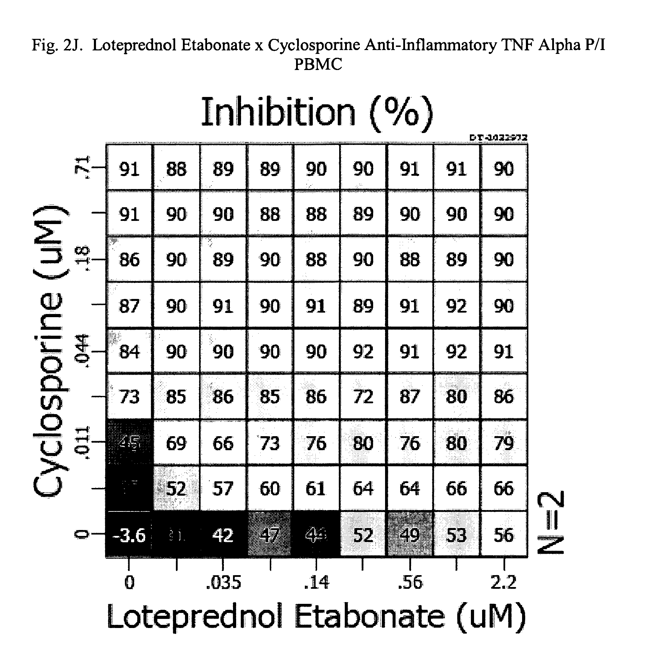

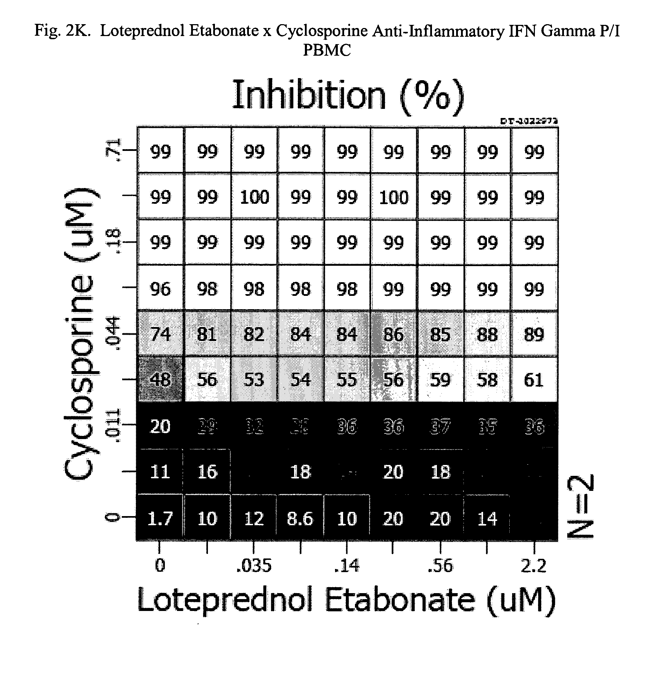

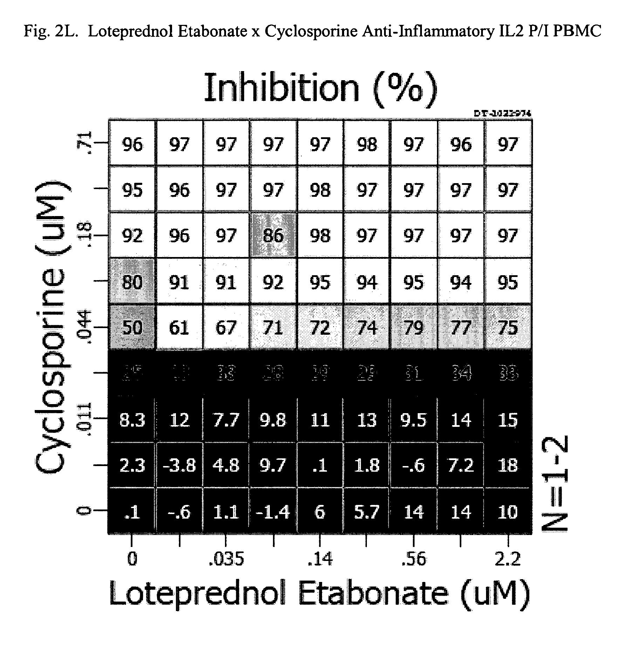

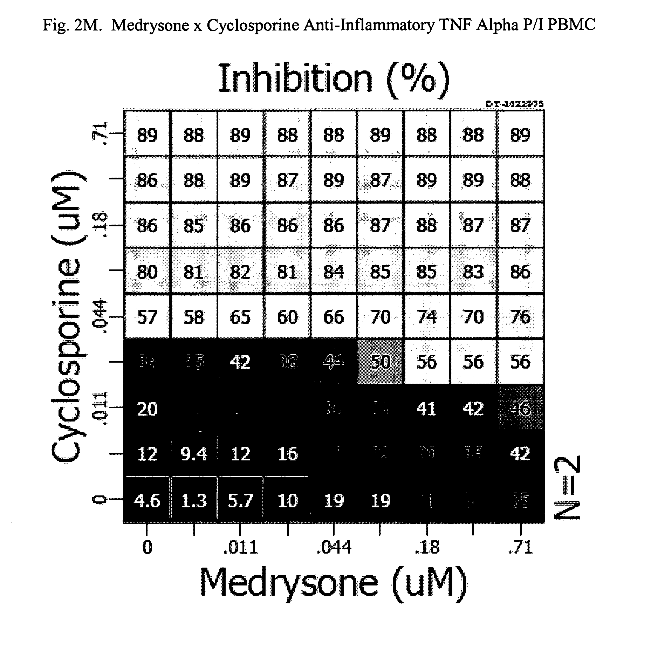

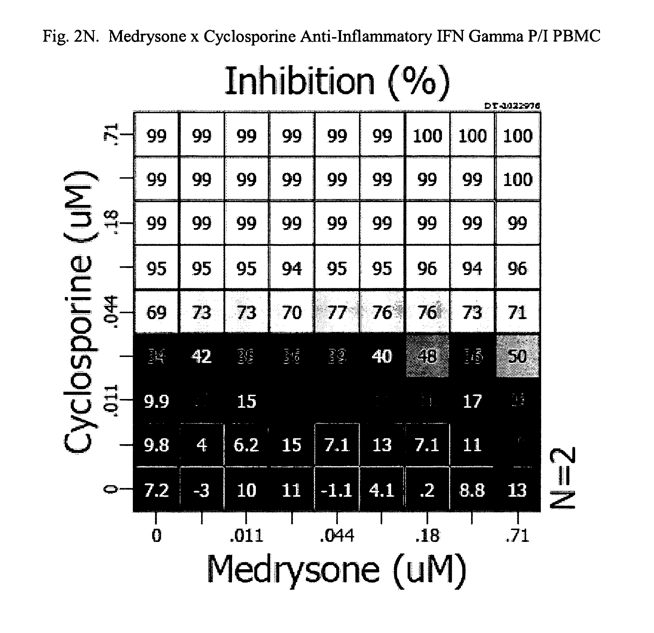

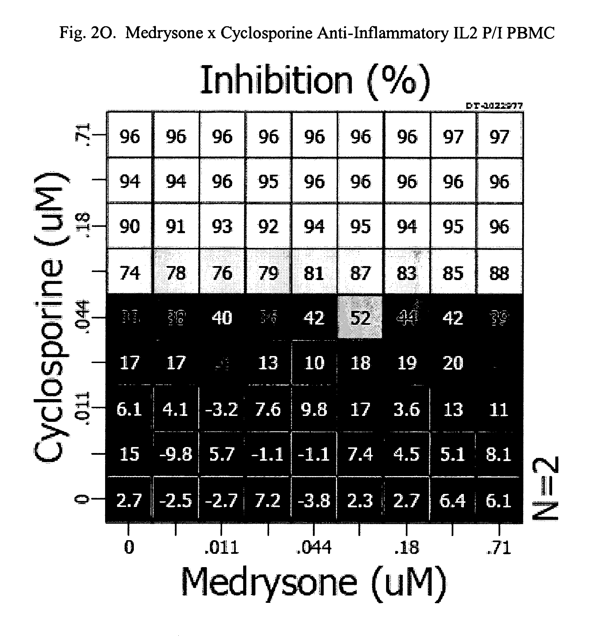

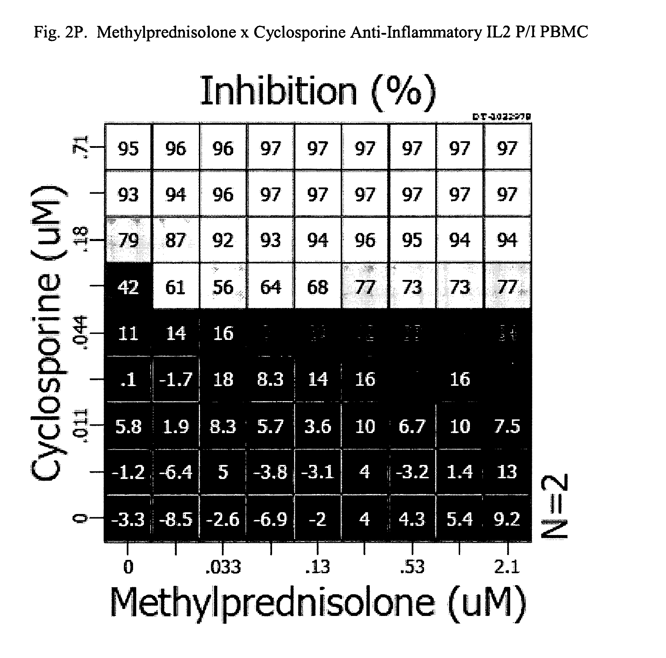

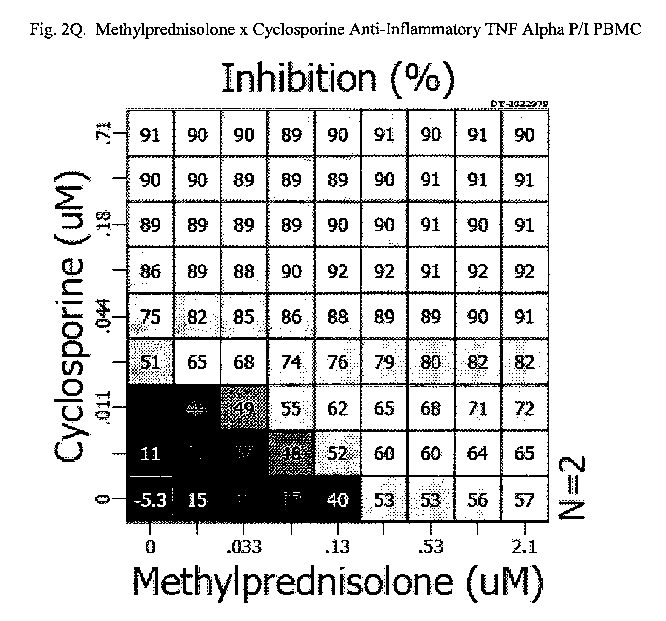

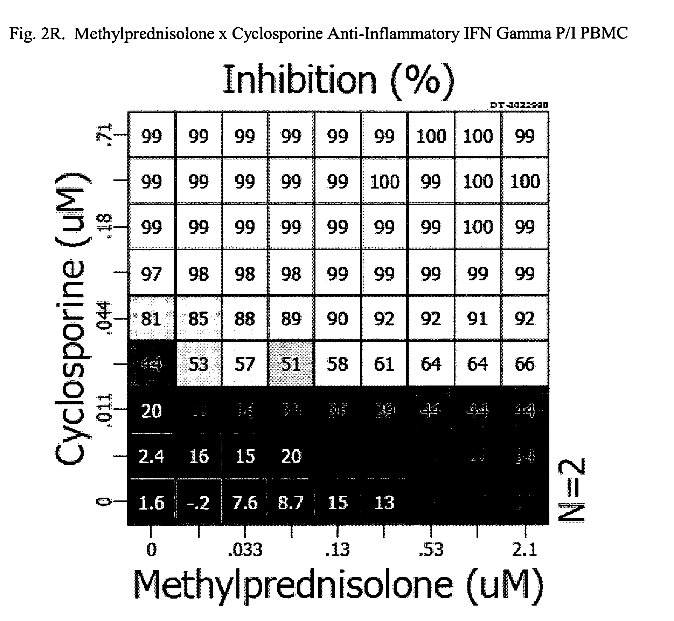

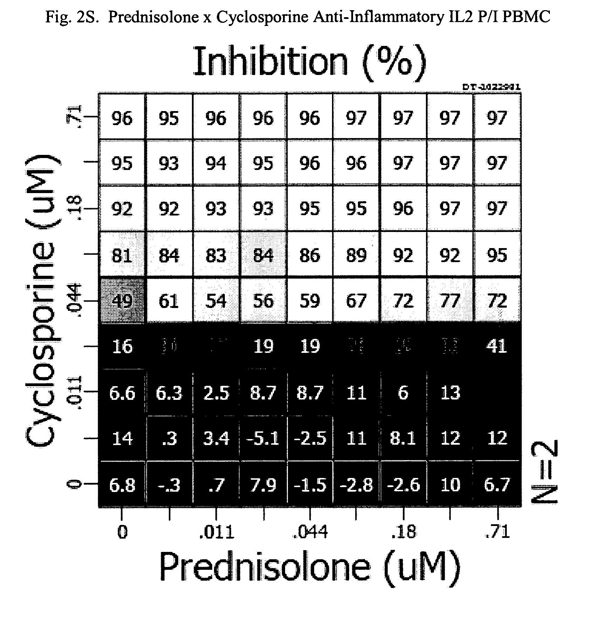

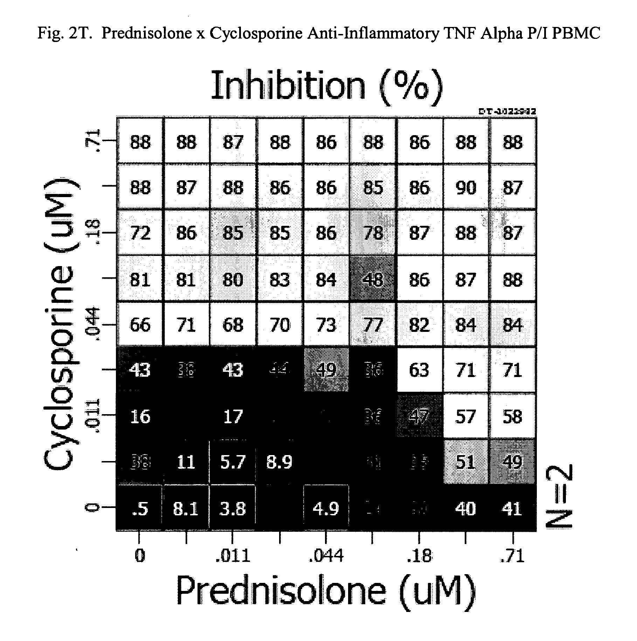

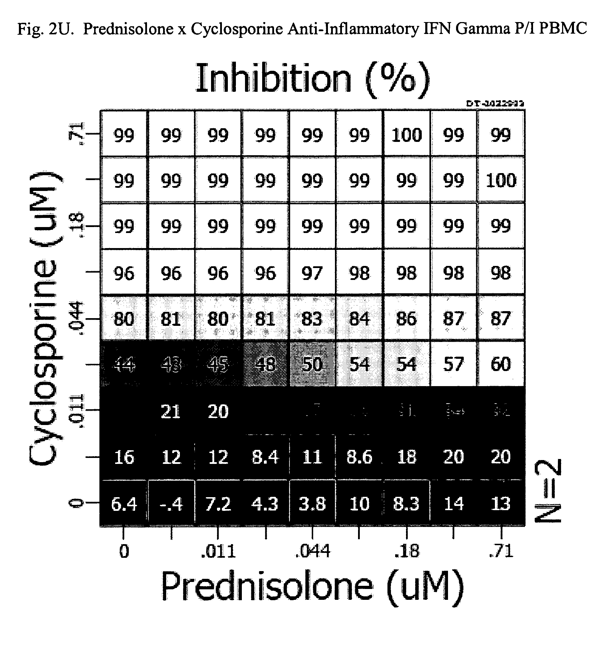

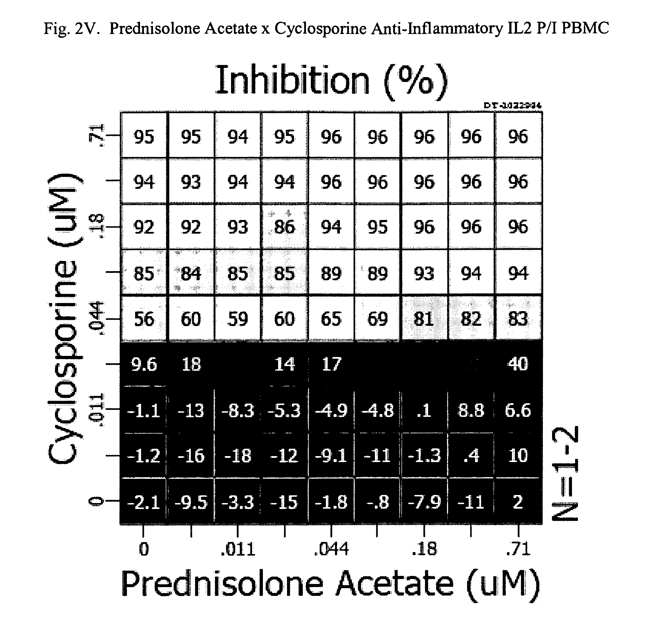

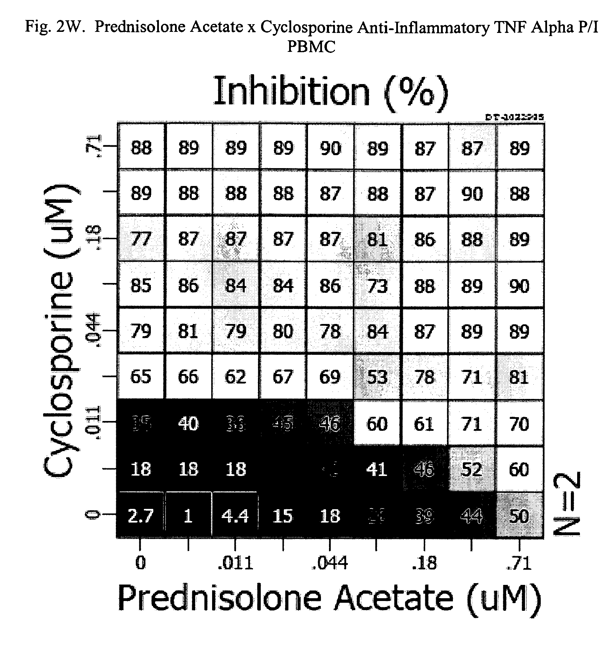

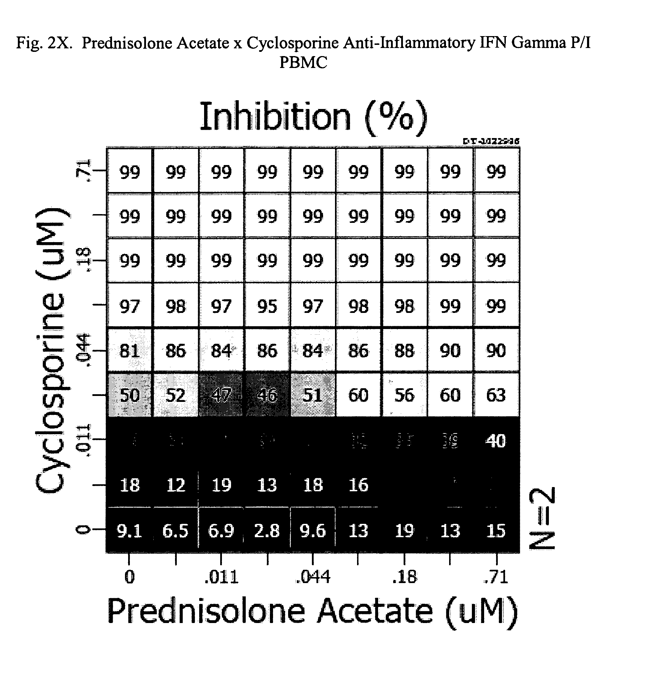

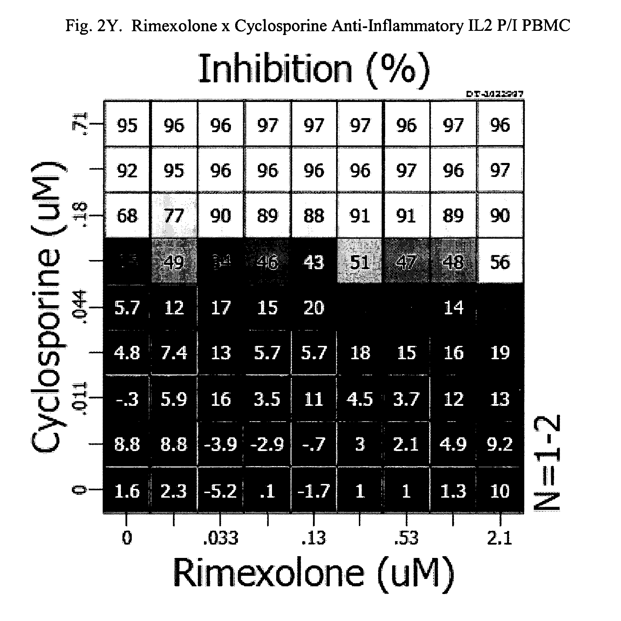

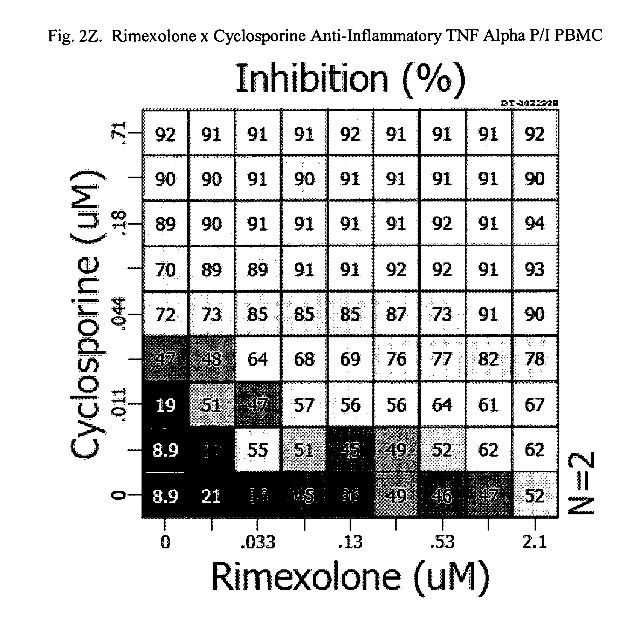

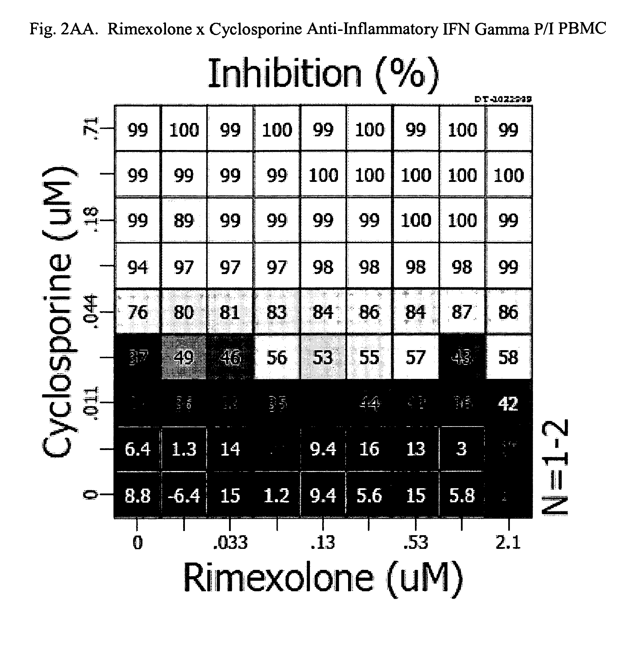

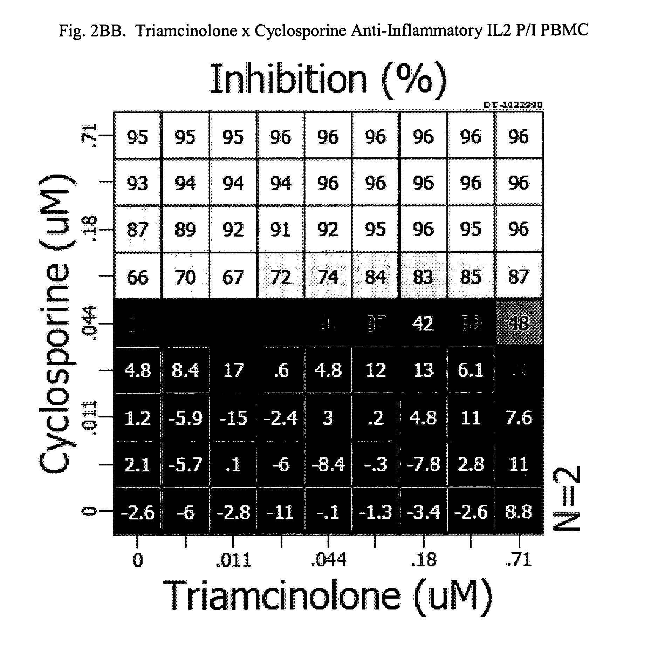

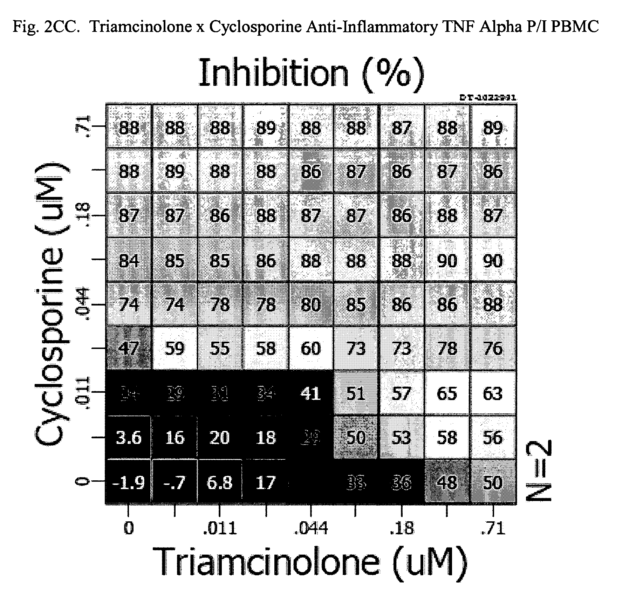

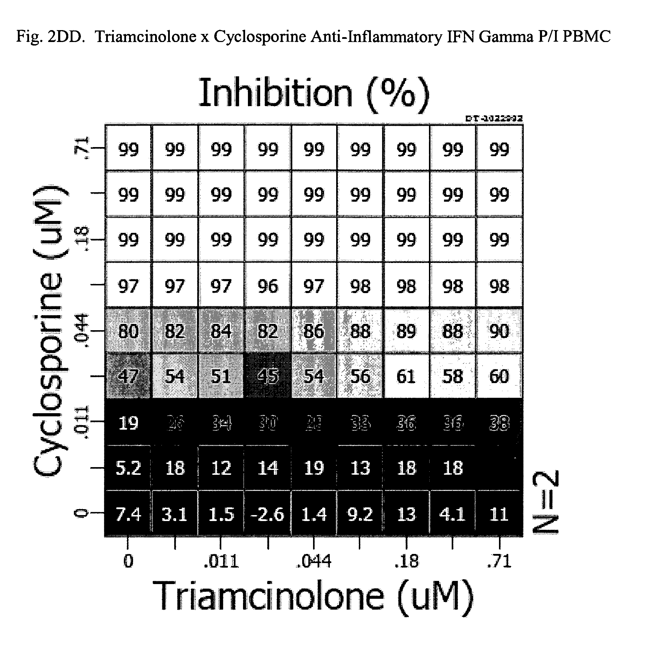

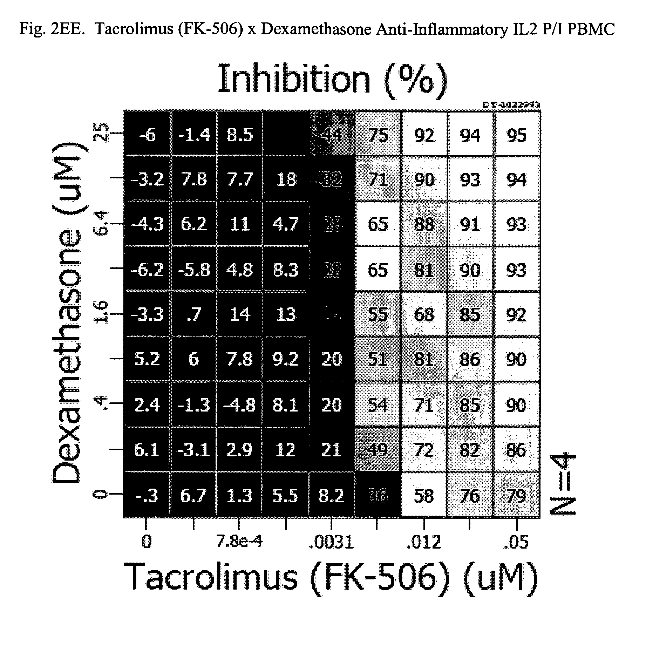

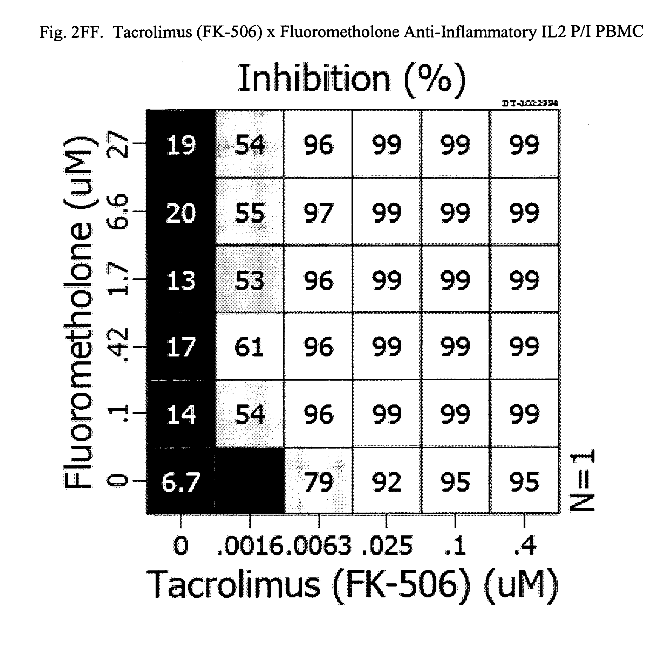

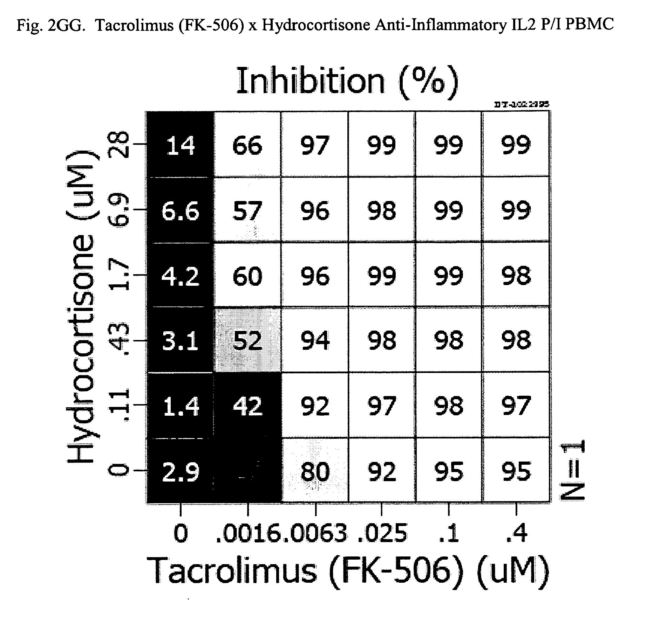

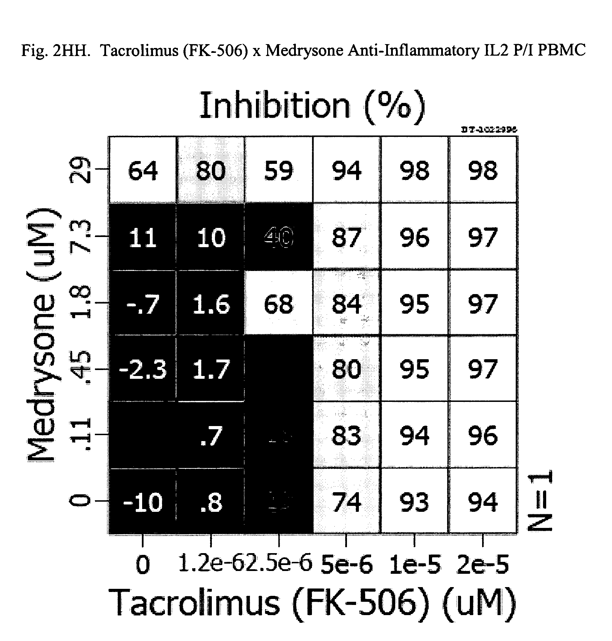

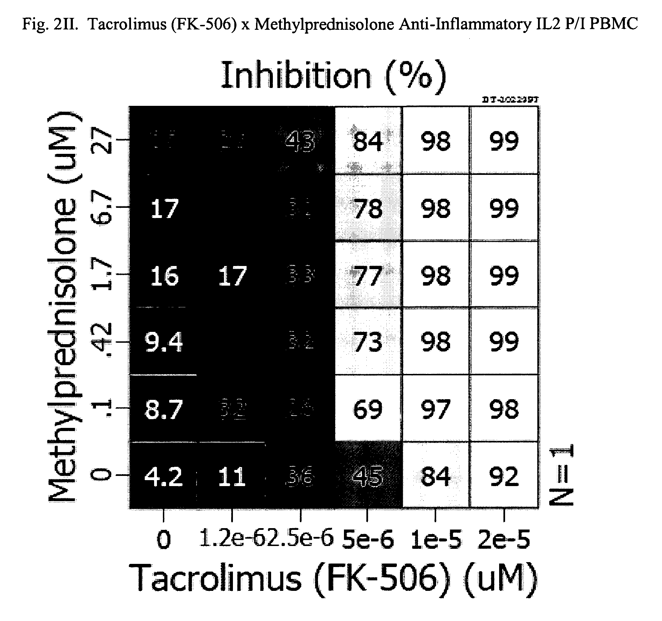

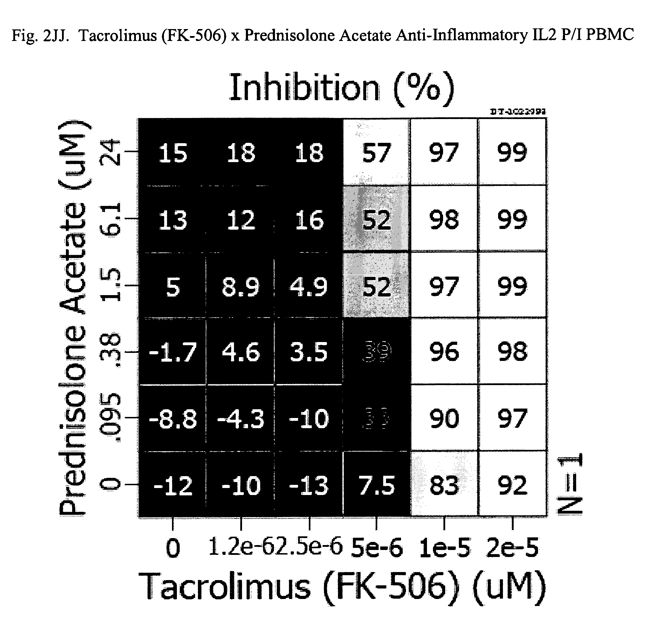

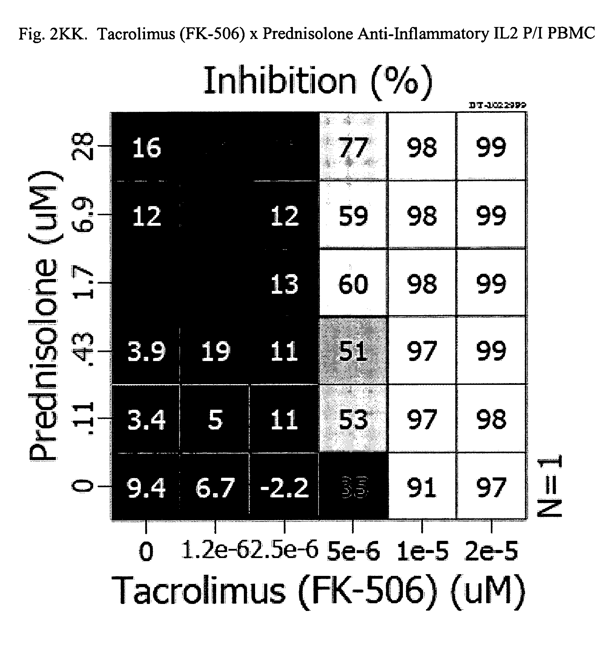

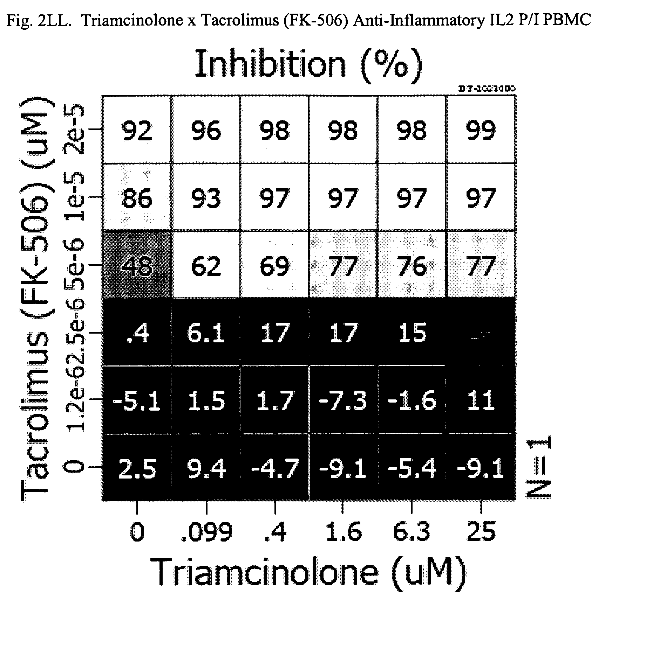

Compound dilution matrices were assayed for the suppression of IFNγ, IL-2, and TNFα, as described below. The results from these experiments are set forth in Figs. 2A-2LL.

### IFNγ

A 100 µL suspension of diluted human white blood cells contained within each well of a polystyrene 384-well plate (NalgeNunc) was stimulated to secrete IFNγ by treatment with a final concentration of 10 ng/mL phorbol 12-myristate 13-acetate (Sigma, P-1585) and 750 ng/mL ionomycin (Sigma, I-0634). Various concentrations of each test compound were added at the time of stimulation. After 16-18 hours of incubation at 37°C in a humidified incubator, the plate was centrifuged and the supernatant transferred to a white opaque polystyrene 384 well plate (NalgeNunc, Maxisorb) coated with an anti-IFNγ antibody (Endogen, #M-700A-E). After a two-hour incubation, the plate was washed (Tecan PowerWasher 384) with phosphate buffered saline (PBS) containing 0.1% Tween 20 (polyoxyethylene sorbitan monolaurate) and incubated for an additional one hour with another anti-IFNγ antibody that was biotin labeled (Endogen, M701B) and horseradish peroxidase (HRP) coupled to strepavidin (PharMingen, #13047E). After the plate was washed with 0.1% Tween 20/PBS, an HRP-luminescent substrate was added to each well and light intensity measured using a LJL Analyst plate luminometer.

### IL-2

A 100 µL suspension of diluted human white blood cells contained within each well of a polystyrene 384-well plate (NalgeNunc) was stimulated to secrete IL-2 by treatment with a final concentration of 10 ng/mL phorbol 12-myristate 13-acetate (Sigma, P-1585) and 750 ng/mL ionomycin (Sigma, I-0634). Various concentrations of each test compound were added at the time of stimulation. After 16-18 hours of incubation at 37°C in a humidified incubator, the plate was centrifuged and the supernatant transferred to a white opaque polystyrene 384 well plate (NalgeNunc, Maxisorb) coated with an anti-IL-2 antibody (PharMingen, #555051). After a two-hour incubation, the plate was washed (Tecan PowerWasher 384) with PBS containing 0.1% Tween 20 and incubated for an additional one hour with another anti-IL-2 antibody that was biotin labeled (Endogen, M600B) and HRP coupled to strepavidin (PharMingen, # 3047E). After the plate was washed with 0.1% Tween 20/PB S, an HRP-luminescent substrate was added to each well and light intensity measured using a LJL Analyst plate luminometer.

### TNFα

The effects of test compound combinations on TNFα secretion were assayed in white blood cells from human buffy coat stimulated with phorbol 12-myistate 13-acetate as follows. Human white blood cells from buffy coat were diluted 1:50 in media (RPMI; Gibco BRL, #11875-085), 10% fetal bovine serum (Gibco BRL, #25140-097), 2% penicillin/streptomycin (Gibco BRL, #15140-122)) and 50 µL of the diluted white blood cells was placed in each well of the assay plate. Drugs were added to the indicated concentration. After 16-18 hours of incubation at 37°C with 5% CO₂ in a humidified incubator, the plate was centrifuged and the supernatant transferred to a white opaque polystyrene 384-well plate (NalgeNunc, Maxisorb) coated with an anti-TNFα antibody (PharMingen, #551220). After a two-hour incubation, the plate was washed (Tecan Powerwasher 3 84) with PBS containing 0.1% Tween 20 and incubated for one additional hour with biotin labeled anti-TNFa antibody (PharMingen, #554511) and HRP coupled to streptavidin (PharMingen, #13047E). The plate was then washed again with 0.1% Tween 20/PB S. An HRP-luminescent substrate was added to each well, and the light intensity of each well was measured using a plate luminometer.

### Percent inhibition

The percent inhibition (%I) for each well was calculated using the following formula: $% I = \left[\left(avg. untreated wells-treated well\right)/\left(avg. untreated wells\right)\right] \times 100$ The average untreated well value (avg. untreated wells) is the arithmetic mean of 40 wells from the same assay plate treated with vehicle alone. Negative inhibition values result from local variations in treated wells as compared to untreated wells.

### Preparation of compounds

The stock solution containing cyclosporin A was made at a concentration of 1.2 mg/ml in DMSO. The stock solution of tacrolimus was made at a concentration of 0.04 mg/ml in DMSO. Stock solutions containing a corticosteroid were made in dimethylsulfoxide (DMSO) at a final concentration of between 0 and 40 µM. Master plates were prepared to contain dilutions of the stock solutions of the compounds described above. Master plates were sealed and stored at -20°C until ready for use.

The final single agent plates were generated by transferring 1 µL of stock solution from the specific master plate to a dilution plate containing 100 µL of media (RPMI; Gibco BRL, #11875-085), 10% fetal bovine serum (Gibco BRL, #25140-097), 2% Penicillin/Streptomycin (Gibco BRL, #15140-122)) using the Packard Mini-Trak liquid handler. This dilution plate was then mixed and a 5 mL aliquot transferred to the final assay plate, which had been pre-filled with 50mL/well RPMI media containing the appropriate stimulant to activate IFNγ, IL-2, or TNFα secretion.

### Other Embodiments

Various modifications and variations of the described methods and compositions of the invention will be apparent to those skilled in the art without departing from the scope and spirit of the invention. Although the invention has been described in connection with specific desired embodiments, it should be understood that the invention as claimed should not be unduly limited to such specific embodiments. Indeed, various modifications of the described modes for carrying out the invention that are obvious to those skilled in the fields of medicine, immunology, pharmacology, endocrinology, or related fields are intended to be within the scope of the invention.

All publications mentioned in this specification are herein incorporated by reference to the same extent as if each independent publication was specifically and individually incorporated by reference.

The following pages 108 to 118 contain specific embodiments.
1. A composition comprising a drug pair selected from the pairs listed on Table 1A or Table 3 in amounts that together are sufficient to treat an immunoinflammatory disorder, an ophthalmic disorder, a musculoskeletal disorder or pain associated therewith, a periodontal disease, or a disease or condition associated with an increased serum CRP level when administered to a patient in need thereof.
2. The composition of 1, wherein said drug pair is selected from: antihistamine and phosphodiesterase inhibitor; antihistamine and SSRI; antihistamine and tricyclic compound; antinfective and anticoccidial compound; corticosteroid and antihistamine; corticosteroid and phosphodiesterase inhibitor; corticosteroid and prostaglandin; NsIDI and alpha-2 adrenoceptor agonist; NsIDI and antihistamine; NsmI and NMDA antagonist/antidyskinetic; NsIDI and prostaglandin; NsIDI and sympathomimetic; prostaglandin and phosphodiesterase inhibitor; prostaglandin and tetra-substituted pyrimidopyrimidine; sympathomimetic and NMDA antagonist/antidyskinetic; sympathomimetic and prostaglandin; sympathomimetic and tetra-substituted pyrimidopyrimidine; sympathomimetic and tricyclic compound; tetra-substituted pyrimidopyrimidine and phosphodiesterase inhibitor; tetra-substituted pyrimidopyrimidine and SSRI; tetra-substituted pyrimidopyrimidine and tricyclic compound; tricyclic compound and calcium channel blocker.
3. The composition of 1, wherein one or both drugs in said pair are present in said composition in a low dosage.
4. The composition of 1, wherein one or both drugs in said pair are is present in said composition in a high dosage.
5. The composition of 1, wherein said composition is formulated for topical administration.
6. The composition of 1, wherein said composition is formulated for systemic administration.
7. The composition of 1, wherein said composition is formulated for ophthalmic admistration.
8. A method for treating a patient diagnosed with an immunoinflammatory disorder, said method comprising administering to the patient a drug pair selected from the pairs listed on Table 1A or Table 3, wherein the first and second drug of said drug pair are administered simultaneously or within 14 days of each other in amounts that together are sufficient to treat said patient.
9. The method of 8, wherein said immunoinflammatory disorder is rheumatoid arthritis, Crohn's disease, ulcerative colitis, asthma, chronic obstructive pulmonary disease, polymylagia rheumatica, giant cell arteritis, systemic lupus erythematosus, atopic dermatitis, multiple sclerosis, myasthenia gravis, psoriasis, ankylosing spondylitis, or psoriatic arthritis.
10. The method of 8, further comprising administering to said patient an NSAID, corticosteroid, COX-2 inhibitor, biologic, DMARD, small molecule immunomodulator, xanthine, anticholinergic compound, beta receptor agonist, bronchodilator, non-steroidal immunophilin-dependent immunosuppressant, vitamin D analog, psoralen, retinoid, or 5-amino salicylic acid.
11. A method for treating a patient diagnosed with an ophthalmic disorder, said method comprising administering to the patient a drug pair selected from the pairs listed on Table 1A or Table 3, wherein the first and second drug of said drug pair are administered simultaneously or within 14 days of each other in amounts that together are sufficient to treat said patient.
12. The method of 11, wherein said ophthalmic disorder is age related macular degeneration; alkaline erosive keratoconjunctivitis; allergic conjunctivitis; allergic keratitis; anterior uveitis; Behcet's syndrome; blepharitis; chorioiditis; chronic uveitis; conjunctivitis; contact lens-induced keratoconjunctivitis; corneal trauma; corneal ulcer; crystalline retinopathy; cystoid macular edema; dacryocystitis; diabetic keratophathy; diabetic macular edema; diabetic retinopathy; dry eye disease; dry age-related macular degeneration; episcleritis; exudative macular edema; Fuchs' Dystrophy; giant cell arteritis; giant papillary conjunctivitis; glaucoma; glaucoma surgery failure; graft rejection; herpes zoster; inflammation after cataract surgery; iridocorneal endothelial syndrome; iritis; keratoconjunctiva sicca; keratoconjunctival inflammatory disease; keratoconus; lattice dystrophy; map-dot-fingerprint dystrophy; necrotic keratitis; neovascular diseases involving the retina, uveal tract or cornea such as neovascular glaucoma, corneal neovascularization; neuroparalytic keratitis; non-infectious uveitisocular herpes; ocular rosacea; ophthalmic infections; ophthalmic pemphigoid; optic neuritis; panuveitis; papillitis; pars planitis; persistent macular edema; phacoanaphylaxis; posterior uveitis; post-operative inflammation; proliferative diabetic retinopathy; proliferative sickle cell retinopathy; proliferative vitreoretinopathy; retinal artery occlusion; retinal detachment; retinal vein occlusion; retinitis pigmentosa; retinopathy of prematurity; rubeosis iritis; scleritis; Stevens-Johnson syndrome; sympathetic ophthalmia; temporal arteritis; uveitis; vernal conjunctivitis; vitamin A insufficiency-induced keratomalacia; vitreitis; or wet age-related macular degeneration.
13. The method of 11, further comprising administering to said patient an anti-VEGF compound, corticosteroid, NSAID, antiallergic agent, antihistamine, glaucoma-treating agent, antibiotic, antiviral agent, or antimycotic agent.
14. A method for treating pain associated with a musculoskeletal disorder, said method comprising administering to the patient a drug pair selected from the pairs listed on Table 1A or Table 3, wherein the drugs are administered simultaneously or within fourteen days of each other in amounts sufficient to treat said patient.
15. A method for treating a musculoskeletal disorder, said method comprising administering to the patient a drug pair selected from the pairs listed on Table 1A or Table 3, wherein the drugs are administered simultaneously or within fourteen days of each other in amounts sufficient to treat said patient.
16. A method for treating periodontal disease, said method comprising administering to the patient a drug pair selected from the pairs listed on Table 1A or Table 3, wherein the drugs are administered in amounts and for a duration that together are sufficient to treat periodontal disease.
17. A method for reducing the serum C-reactive protein (CRP) level in a patient in need thereof, said method comprising administering to the patient a drug pair selected from the pairs listed on Table 1A or Table 3, wherein the drugs are administered in amounts and for a duration that together are sufficient to reduce the serum CRP level in said patient.
18. A method for treating a disease or condition associated with an increased serum CRP level in a patient in need thereof, said method comprising administering to the patient a drug pair selected from the pairs listed on Table 1A or Table 3, wherein the drugs are administered in amounts and for a duration that together are sufficient to reduce the serum CRP level in said patient.
19. The method of any one of 8, 11, and 14-18, wherein one or both drugs in said pair are administered in a low dosage.
20. The method of any one of 8, 11, and 14-18, wherein one or both drugs in said pair are administered in a high dosage.
21. The method of any one of 8, 11, and 14-18, wherein said first drug and said second drug are administered within 10 days of each other.
22. The method of 21, wherein said first drug and said second drug are administered within five days of each other.
23. The method of 22, wherein said said first drug and said second drug are administered within twenty-four hours of each other.
24. The method of 23, wherein said first drug and said second drug are administered simultaneously.
25. A composition comprising (i) drug listed on Table 1A or Table 3; and (ii) a second compound selected from the group consisting of an anti-VEGF compound, corticosteroid, NSAID, antiallergic agent, antihistamine, glaucoma-treating agent, antibiotic, antiviral agent, or antimycotic agent.
26. A kit, comprising: (i) a composition comprising a drug pair selected from the pairs listed on Table 1A or Table 3; and (ii) instructions for administering said composition to a patient diagnosed with or at risk of developing an immunoinflammatory disorder, an ophthalmic disorder, a musculoskeletal disorder or pain associated therewith, a periodontal disease, or a disease or condition associated with an increased serum CRP level.
27. A kit, comprising: (i) a first drug from a drug pair selected from the pairs listed on Table 1A or Table 3; (ii) a second drug from said drug pair; and (iii) instructions for administering said first drug and said second drug to a patient diagnosed with or at risk of developing an immunoinflammatory disorder, an ophthalmic disorder, a musculoskeletal disorder or pain associated therewith, a periodontal disease, or a disease or condition associated with an increased serum CRP level.
28. A kit comprising (i) a first drug from a drug pair selected from the pairs listed on Table 1A or Table 3; and (ii) instructions for administering said first drug and a second drug from said drug pair to a patient diagnosed with or at risk of developing an immunoinflammatory disorder, an ophthalmic disorder, a musculoskeletal disorder or pain associated therewith, a periodontal disease, or a disease or condition associated with an increased serum CRP level.
29. A kit, comprising: (i) a first drug listed on Table 1A or Table 3; (ii) a second drug selected from an anti-VEGF compound, photodynamic therapy, corticosteroid, NSAID, antiallergic agent, antihistamine, glaucoma-treating agent, antibiotic, antiviral agent, and antimycotic agent; and (iii) instructions for administering said first drug and said second drug to a patient diagnosed with an ophthalmic disorder.
30. A kit comprising (i) a first drug listed on Table 1A or Table 3; and (ii) instructions for administering said first drug and a second drug selected from an anti-VEGF compound, photodynamic therapy, corticosteroid, NSAID, antiallergic agent, antihistamine, glaucoma-treating agent, antibiotic, antiviral agent, and antimycotic agent to a patient diagnosed with an ophthalmic disorder.
31. A method of treating an ophthalmic disorder in a patient, said method comprising administering to said patient a composition comprising a corticosteroid and a non-steroidal immunophilin-dependent immunosuppressant, wherein at least one of said corticosteroid and said non-steroidal immunophilin-dependent immunosuppressant is present at a low concentration.
32. The method of 31, wherein said corticosteroid is selected from the group consisting of the class of selective glucocorticosteroid receptor agonists (SEGRAs), fluocinolone acetonide, fluorometholone, dexamethasone, hydrocortisone, loteprednol, medrysone, methylprednisolone, prednisolone, rimexolone, and triamcinolone.
33. The method of 32, wherein said corticosteroid is prednisolone.
34. The method of 33, wherein said prednisolone is present at a concentration between 0.01% and 0.1%.
35. The method of 31, wherein said corticosteroid is present at a concentration equivalent to a concentration of prednisolone of between 0.01% and 0.1%.
36. The method of 31, wherein said non-steroidal immunophilin-dependent immunosuppressant is selected from the group consisting of cyclosporine A, ABT-281, ISAtx247, tacrolimus, ascomycin, pimecrolimus, rapamycin, and everolimus.
37. The method of 36, wherein said non-steroidal immunophilin-dependent immunosuppressant is cyclosporine A.
38. The method of 37, wherein said cyclosporine A is present at a concentration between 0.001% and 0.049%.
39. The method of 31, wherein said non-steroidal immunophilin-dependent immunosuppressant is present at a concentration equivalent to a concentration of cyclosporine A between 0.001% and 0.049%.
40. The method of 31, wherein said corticosteroid is prednisolone and said non-steroidal immunophilin-dependent immunosuppressant is cyclosporine A.
41. The method of 31, wherein said ophthalmic disorder is selected from the group consisting of age related macular degeneration, alkaline erosive keratoconjunctivitis, allergic conjunctivitis, allergic keratitis, anterior uveitis, Behcet's disease, blepharitis, blood-aqueous barrier disruption, chorioiditis, chronic uveitis, conjunctivitis, contact lens-induced keratoconjunctivitis, corneal abrasion, corneal trauma, corneal ulcer, crystalline retinopathy, cystoid macular edema, dacryocystitis, diabetic keratophathy, diabetic macular edema, diabetic retinopathy, dry eye disease, dry age-related macular degeneration, eosinophilic granuloma, episcleritis, exudative macular edema, Fuchs' Dystrophy, giant cell arteritis, giant papillary conjunctivitis, glaucoma, glaucoma surgery failure, graft rejection, herpes zoster, inflammation after cataract surgery, iridocorneal endothelial syndrome, iritis, keratoconjunctiva sicca, keratoconjunctival inflammatory disease, keratoconus, lattice dystrophy, map-dot-fingerprint dystrophy, necrotic keratitis, neovascular diseases involving the retina, uveal tract or cornea such as neovascular glaucoma, corneal neovascularization, neovascularization resulting following a combined vitrectomy and lensectomy, neovascularization of the optic nerve, and neovascularization due to penetration of the eye or contusive ocular injury, neuroparalytic keratitis, non-infectious uveitisocular herpes, ocular lymphoma, ocular rosacea, ophthalmic infections, ophthalmic pemphigoid, optic neuritis, panuveitis, papillitis, pars planitis, persistent macular edema, phacoanaphylaxis, posterior uveitis, post-operative inflammation, proliferative diabetic retinopathy, proliferative sickle cell retinopathy, proliferative vitreoretinopathy, retinal artery occlusion, retinal detachment, retinal vein occlusion, retinitis pigmentosa, retinopathy of prematurity, rubeosis iritis, scleritis, Stevens-Johnson syndrome, sympathetic ophthalmia, temporal arteritis, thyroid associated ophthalmopathy, uveitis, vernal conjunctivitis, vitamin A insufficiency-induced keratomalacia, vitreitis, and wet age-related macular degeneration.
42. The method of 41, wherein said ophthalmic disorder is dry eye.
43. The method of 41, wherein said ophthalmic disorder is allergic conjunctivitis.
44. A composition suitable for ophthalmic administration comprising a corticosteroid and a non-steroidal immunophilin-dependent immunosuppressant, wherein at least one of said corticosteroid and said non-steroidal immunophilin-dependent immunosuppressant is present at a low concentration.
45. The composition of 44, wherein said composition is a solution, gel, ointment, suspension, emulsion, or solid insert.
46. The composition of 44, wherein said corticosteroid is selected from the group consisting of the class of selective glucocorticosteroid receptor agonists (SEGRAs), fluocinolone acetonide, fluorometholone, dexamethasone, hydrocortisone, loteprednol, medrysone, methylprednisolone, prednisolone, rimexolone, and triamcinolone.
47. The composition of 46, wherein said corticosteroid is prednisolone.
48. The composition of 47, wherein said prednisolone is present at a concentration between 0.01% and 0.1%.
49. The composition of 44, wherein said corticosteroid is present at a concentration equivalent to a concentration of prednisolone of between 0.01% and 0.1%.
50. The composition of 44, wherein said non-steroidal immunophilin-dependent immunosuppressant is selected from the group consisting of cyclosporine A, ABT-281, ISAtx247, tacrolimus, ascomycin, pimecrolimus, rapamycin, and everolimus.
51. The composition of 50, wherein said non-steroidal immunophilin-dependent immunosuppressant is cyclosporine A.
52. The composition of 144, wherein said corticosteroid is prednisolone and said non-steroidal immunophilin-dependent immunosuppressant is cyclosporine A.
53. The composition of 49, wherein said cyclosporine A is present at a concentration between 0.001% and 0.049%.
54. The composition of 44, wherein said non-steroidal immunophilin-dependent immunosuppressant is present at a concentration equivalent to a concentration of cyclosporine A between 0.001% and 0.049%.

## Claims

1. A composition comprising a corticosteroid and a non-steroidal immunophilin-dependent immunosuppressant for treating an ophthalmic disorder in a patient, wherein at least one of said corticosteroid and said non-steroidal immunophilin-dependent immunosuppressant is present at a low concentration, in particular wherein said corticosteroid is prednisolone acetate and said non-steroidal immunophilin-dependent immunosuppressant is cyclosporine A.

2. The composition as claimed in claim 1, wherein said prednisolone acetate is present at a concentration between 0.01 % and 0.12%.

3. The composition as claimed in claim 1 or 2, wherein said cyclosporine A is present at a concentration between 0.001 % and 0.049%.

4. The composition as claimed in claim 1, wherein said corticosteroid is selected from the group consisting of the class of selective glucocorticosteroid receptor agonists (SEGRAs), fluocinolone acetonide, fluorometholone, dexamethasone, hydrocortisone, loteprednol, medrysone, methylprednisolone, prednisolone, rimexolone, and triamcinolone.

5. The composition as claimed in claim 1 or 4, wherein said corticosteroid is present at a concentration equivalent to a concentration of prednisolone acetate of between 0.01% and 0.12%.

6. The composition as claimed in any of the claims 1, 4 and 5, wherein said non-steroidal immunophilin-dependent immunosuppressant is selected from the group consisting of ABT-281, ISAtx247, tacrolimus, ascomycin, pimecrolimus, rapamycin, and everolimus.

7. The composition as claimed in any of the claims 1 and 4-6, wherein said non-steroidal immunophilin-dependent immunosuppressant is present at a concentration equivalent to a concentration of cyclosporine A between 0.001 % and 0.049%.

8. The composition as claimed in any of the claims 1-7, wherein said ophthalmic disorder is selected from the group consisting of age related macular degeneration, alkaline erosive keratoconjunctivitis, allergic conjunctivitis, allergic keratitis, anterior uveitis, Behcet's disease, blepharitis, blood-aqueous barrier disruption, chorioiditis, chronic uveitis, conjunctivitis, contact lens-induced keratoconjunctivitis, corneal abrasion, corneal trauma, corneal ulcer, crystalline retinopathy, cystoid macular edema, dacryocystitis, diabetic keratophathy, diabetic macular edema, diabetic retinopathy, dry eye disease, dry age-related macular degeneration, eosinophilic granuloma, episcleritis, exudative macular edema, Fuchs' Dystrophy, giant cell arteritis, giant papillary conjunctivitis, glaucoma, glaucoma surgery failure, graft rejection, herpes zoster, inflammation after cataract surgery, iridocorneal endothelial syndrome, iritis, keratoconjunctiva sicca, keratoconjunctival inflammatory disease, keratoconus, lattice dystrophy, map-dot-fingerprint dystrophy, necrotic keratitis, neovascular diseases involving the retina, uveal tract or cornea such as neovascular glaucoma, corneal neovascularization, neovascularization resulting following a combined vitrectomy and lensectomy, neovascularization of the optic nerve, and neovascularization due to penetration of the eye or contusive ocular injury, neuroparalytic keratitis, non-infectious uveitisocular herpes, ocular lymphoma, ocular rosacea, ophthalmic infections, ophthalmic pemphigoid, optic neuritis, panuveitis, papillitis, pars planitis, persistent macular edema, phacoanaphylaxis, posterior uveitis, post-operative inflammation, proliferative diabetic retinopathy, proliferative sickle cell retinopathy, proliferative vitreoretinopathy, retinal artery occlusion, retinal detachment, retinal vein occlusion, retinitis pigmentosa, retinopathy of prematurity, rubeosis iritis, scleritis, Stevens-Johnson syndrome, sympathetic ophthalmia, temporal arteritis, thyroid associated ophthalmopathy, uveitis, vernal conjunctivitis, vitamin A insufficiency-induced keratomalacia, vitreitis, and wet age-related macular degeneration, in particular wherein said ophthalmic disorder is dry eye or allergic conjunctivitis.

9. The composition of any of the claims 1-8, wherein said composition is suitable for ophthalmic administration, in particular said composition is a solution, gel, ointment, suspension, emulsion, or solid insert.
